# EUROPEAN PATENT APPLICATION

(11) **EP 4 368 719 A1**
(43) Date of publication of application: **15.05.2024**
(21) Application number: 22838081.2
(22) Date of filing: 11.07.2022
(51) Int. Cl.: C12N 15/63, C12N 5/0783, C12N 15/85

(54) **TRANSPOSON SYSTEM AND USES THEREOF**

(30) Priority: 09.07.2021 KR 20210090133
(71) Applicant: Neogentc Corp., Seoul 05505 (KR)
(72) Inventor: LIM, Chae Lyul, Hanam-si, Gyeonggi-do 12904 (KR); KIM, Young-Ae, Seoul 05532 (KR); CHUNG, Jiwon, Seoul 04746 (KR); PARK, Seeun, Seoul 05388 (KR)
(74) Representative: Nederlandsch Octrooibureau
(86) International application number: PCT/KR2022/010075
(87) International publication number: WO 2023/282730

(57) **Abstract**

The present invention relates to a transposon vector, a transposon system comprising same, a transposon kit, a cell into which the transposon vector is inserted, and uses thereof, wherein the present invention has been completed by confirming that an exogenous gene is effectively transferred into the chromosome of a target cell to produce a high yield of genetically modified cells. In particularly, it was confirmed that the transposon according to the present invention can effectively transfer a TCR or CAR-encoding gene to immune cells, and cells expressing the TCR or CAR show high reactivity to an antigen, and thus, it is expected that various TCR-T cells and CAR-T cells can be produced by using the transposon system according to the present invention. In particular, CAR-T cells with a high yield can be obtained at a low cost by using the transposon of the present invention, such that the prices of therapeutics can be lowered by lowering the production costs of CAR-T cellular therapeutic agents, whereas conventional CAR-T cells require high costs for the production of CAR as well as transfer to target cells. Moreover, it was confirmed that the transposon of the present invention can effectively transfer an antibody gene, such as an oncovirus-targeting neutralizing antibody, to HEK293 cells used for mass production of antibodies, such that various antibodies can easily be mass-produced by means of the transposon of the present invention In particular, since the transposon according to the present invention is not limited in the type of gene that can be transferred as a vector, it is expected that the transposon can be actively utilized according to various purposes in the development of genome-modified cell lines expressing various genes in addition to antibody genes.

## Description

### [Technical Field]

The present invention relates to a transposon vector, a transposon system including the same, a transposon kit, a cell into which the transposon vector is inserted, and uses thereof.

This application claims priority to and the benefit of Korean Patent Application No. 10-2021-0090133, filed on July 9, 2021, and Korean Patent Application No. 10-2022-0085306, filed on July 11, 2022, the disclosure of which is incorporated herein by reference in their entirety.

### [Background Art]

Chimeric antigen receptor (CAR)-T cells are used as a cell therapeutic produced by inserting the sequence of an antibody binding to a tumor antigen (e.g., CD19) into a T cell by combining it with a domain required for T cell signaling, such as CD3/4-1BB/CD28. There are several ways to insert these CAR genes into T cells, but most of the methods utilize a lentivirus delivery system. The characteristic of a lentivirus is that it can continuously express genes because it is integrated into the chromosome of a cell. These lentiviruses have high production costs, which is a major factor in increasing the prices of therapeutics. However, once produced, they can be used on multiple patients.

However, personalized TCR-T is produced by finding a T-cell receptor (TCR) sequence responding to a neoantigen in each patient and transferring the gene into a T cell using a gene transfer system. However, since TCR-T is personalized with a different TCR sequence applied for each patient, it is almost impossible to apply such TCR-T to a lentivirus. Therefore, it is necessary to develop TCR-T cells using a non-viral carrier, such as a transposon, which can be produced more easily and at lower cost compared to a lentivirus, and is capable of continuous gene expression by integration into the chromosome.

### [Disclosure]

### [Technical Problem]

As a result of intensive research to meet the needs described above, the present inventors developed a transposon as a gene delivery system that enables the integration of an exogenous gene into the chromosome (genome) of a target cell, particularly, an immune cell, and confirmed that transposon mutants additionally produced by modifying the 5' inverted terminal repeat (ITR) and 3' ITR of the transposon also have excellent gene delivery efficiency. Based on this, the present invention was completed.

Therefore, the present invention is directed to providing a transposon vector including a 5' ITR and a 3' ITR, which are able to exhibit an excellent gene delivery effect.

The present invention is also directed to providing a transposon system for delivering target DNA, which includes the transposon vector and a transposase (a protein or a nucleic acid molecule encoding the same).

The present invention is also directed to providing a transposon kit for delivering target DNA, including the transposon system, and instructions.

The present invention is also directed to providing a cell into which the transposon vector and a transposase are introduced.

The present invention is also directed to providing a method of inserting a target DNA sequence into the genome of a cell, which includes introducing the transposon vector and a transposase into the cell.

The present invention is also directed to providing a pharmaceutical composition, which includes immune cells into which the transposon vector and a transposase are introduced as an active ingredient.

However, technical problems to be solved in the present invention are not limited to the above-described problems, and other problems which are not described herein will be fully understood by those of ordinary skill in the art from the following description.

### [Technical Solution]

One aspect of the present invention provides a transposon vector, which includes a 5' ITR having a sequence of at least 71 consecutive nucleotides in the nucleotide sequence represented by SEQ ID NO: 1; and a 3' ITR having a sequence of at least 66 consecutive nucleotides in the nucleotide sequence represented by SEQ ID NO: 2.

In one embodiment of the present invention, the 5' ITR may be selected from the following:
a 5' ITR having the nucleotide sequence represented by SEQ ID NO: 1;
a 5' ITR having the nucleotide sequence represented by SEQ ID NO: 5; or
a 5' ITR having the nucleotide sequence represented by SEQ ID NO: 6.

The 3' ITR may be selected from the following:
a 3' ITR having the nucleotide sequence represented by SEQ ID NO: 2;
a 3' ITR having the nucleotide sequence represented by SEQ ID NO: 9;
a 3' ITR having the nucleotide sequence represented by SEQ ID NO: 10; or
a 3' ITR having the nucleotide sequence represented by SEQ ID NO: 11.

In another embodiment of the present invention, the 5' ITR may include one or more of the nucleotide sequences represented by SEQ ID NO: 7, 5'-ACACTTGG-3', and SEQ ID NO: 8, but the present invention is not limited thereto.

In still another embodiment of the present invention, the 3' ITR may include one or more of the nucleotide sequences represented by SEQ ID NO: 13 and SEQ ID NO: 14, but the present invention is not limited thereto.

In yet another embodiment of the present invention, the nucleotide sequence of the 5' ITR may be included upstream of a position where target DNA is inserted into the transposon vector in the 5' to 3' direction, and the nucleotide sequence of the 3' ITR may be included downstream of the position where the target DNA is inserted into the transposon vector in the 5' to 3' direction, but the present invention is not limited thereto.

In yet another embodiment of the present invention, in the transposon vector, antisense DNA having a reverse complement sequence of the nucleotide sequence of the 3' ITR (instead of the 3' ITR) may be included downstream of the position where target DNA is inserted into the transposon vector in the 5' to 3' direction, but the present invention is not limited thereto.

In yet another embodiment of the present invention, the reverse complement sequence of the 3' ITR may include the nucleotide sequence represented by any one of SEQ ID NOs: 15 to 17, but the present invention is not limited thereto.

In yet another embodiment of the present invention, the transposon vector may include one or more target DNA sequences downstream of the 5' ITR and upstream of the 3' ITR, but the present invention is not limited thereto.

In yet another embodiment of the present invention, the target DNA sequence may be any one or more selected from the group consisting of a therapeutic polypeptide coding sequence, an siRNA coding sequence, an miRNA coding sequence, a reporter protein coding sequence, an antigen-specific receptor coding sequence, a recombinant antibody coding sequence or a fragment thereof, a neutralizing antibody coding sequence or a fragment thereof, an immune checkpoint inhibitor coding sequence, a cytokine receptor coding sequence, a CAR coding sequence or a fragment thereof, and a T-cell receptor (TCR) coding sequence or a fragment thereof, but the present invention is not limited thereto.

In yet another embodiment of the present invention, the transposon vector may include a promoter, one or more target DNAs, and a poly(A) signal, wherein the 5' ITR, the promoter, the target DNA, the poly(A) signal, and the 3' ITR may be sequentially operably linked, but the present invention is not limited thereto.

In yet another embodiment of the present invention, the transposon vector may be a circular plasmid, linearized double stranded DNA (dsDNA), hairpin dsDNA, or minicircle dsDNA, but the present invention is not limited thereto.

In yet another embodiment of the present invention, the transposon vector may have a size of 1,000 to 20,000 bp, but the present invention is not limited thereto.

Another aspect of the present invention provides a transposon system for delivering target DNA, which includes a) the transposon vector into which target DNA is inserted; and
b) a nucleic acid molecule including a sequence encoding a transposase protein or transposase.

In one embodiment of the present invention, the transposase protein may include the amino acid sequence represented by SEQ ID NO: 18, but the present invention is not limited thereto.

Still another aspect of the present invention provides a transposon kit for delivering target DNA, which includes a transposon system for delivering target DNA, and instructions.

Yet another aspect of the present invention provides a cell into which a) the transposon vector into which target DNA is inserted; and
b) a nucleic acid molecule including a sequence encoding a transposase protein or transposase are introduced.

In one embodiment of the present invention, in the cell, the target DNA may be cleaved from the transposon vector by the transposase, and the cleaved target DNA may be integrated into the genome of the cell, but the present invention is not limited thereto.

In another embodiment of the present invention, the cells may be selected from the group consisting of T cells, NK cells, B cells, dendritic cells, macrophages, and mast cells, but the present invention is not limited thereto.

In still another embodiment of the present invention, the cells may be co-cultured with feeder cells after introducing the transposon vector, but the present invention is not limited thereto.

In yet another embodiment of the present invention, the feeder cells may be cells irradiated with radiation, but the present invention is not limited thereto.

In yet another embodiment of the present invention, the cells may express the target DNA for 7 days or more after introducing the transposon vector, but the present invention is not limited thereto.

Yet another aspect of the present invention provides a method of inserting a target DNA sequence into the genome of a cell, which includes introducing a) the transposon vector into which target DNA is inserted; and
b) a nucleic acid molecule including a sequence encoding a transposase protein or transposase into a cell. The method may be performed *in vitro,* but the present invention is not limited thereto.

In one embodiment of the present invention, the introduction may be performed by electroporation, but the present invention is not limited thereto.

In another embodiment of the present invention, this method may further include, after the introduction, co-culturing the transposon vector-inserted cells with feeder cells, but the present invention is not limited thereto.

In still another embodiment of the present invention, the co-culturing with the feeder cells may be performed immediately after the introduction, but the present invention is not limited thereto.

In yet another embodiment of the present invention, the transposon vector may be a circular plasmid, linearized double stranded DNA (dsDNA), hairpin dsDNA, or minicircle dsDNA, but the present invention is not limited thereto.

Yet another aspect of the present invention provides a pharmaceutical composition for preventing or treating cancer, which includes immune cells into which a) the transposon vector into which target DNA is inserted; and
b) a nucleic acid molecule including a sequence encoding a transposase protein or transposase are introduced as an active ingredient,
wherein the target DNA is one or more selected from the group consisting of a tumor antigen-specific CAR coding sequence or a fragment thereof, an oncovirus-specific neutralizing antibody coding sequence or a fragment thereof, an immune checkpoint inhibitor coding sequence, and a tumor antigen-specific T-cell receptor (TCR) coding sequence or a fragment thereof.

Yet another aspect of the present invention provides a method of preventing or treating cancer, which includes administering the immune cells to a subject in need thereof.

Yet another aspect of the present invention provides a use of the immune cells for preventing or treating cancer.

Yet another aspect of the present invention provides a use of the immune cells for preparing a drug for treating cancer.

Yet another aspect of the present invention provides a method of preparing a drug for treating cancer using the transposon vector of the present invention into which one or more selected from the group consisting of a tumor antigen-specific CAR coding sequence or a fragment thereof, an oncovirus-specific neutralizing antibody coding sequence or a fragment thereof, an immune checkpoint inhibitor coding sequence, and a tumor antigen-specific T-cell receptor (TCR) coding sequence or a fragment thereof are inserted.

Yet another aspect of the present invention provides a use of the transposon vector of the present invention into which one or more selected from the group consisting of a tumor antigen-specific CAR coding sequence or a fragment thereof, an oncovirus-specific neutralizing antibody coding sequence or a fragment thereof, an immune checkpoint inhibitor coding sequence, and a tumor antigen-specific T-cell receptor (TCR) coding sequence or a fragment thereof are inserted to prepare a drug for treating cancer.

In one embodiment of the present invention, the tumor antigen may be one or more selected from the group consisting of CD19, NY-ESO-1, EGFR, TAG72, interleukin 13 receptor alpha-2 subunit (IL13Rα2), CD52, CD33, CD20, TSLPR, CD22, CD30, GD3, CD171, neural cell adhesion molecule (NCAM), folate binding protein (FBP), Lewis-Y antigen (Le(Y)), prostate stem cell antigen (PSCA), prostate-specific membrane antigen (PSMA), carcinoembryonic antigen (CEA), human epidermal growth factor receptor 2 (HER2), mesothelin, hyaluronate receptor variant 6 (CD44v6), B7-H3, glypican-3, receptor tyrosine kinase like orphan receptor 1 (ROR1), survivin, folate receptor (FOLR1), Wilm's tumor antigen (WT1), vascular endothelial growth factor 2 (VEGFR2), an oncovirus antigen, TP53, KRAS, and a neoantigen, but the present invention is not limited thereto.

Yet another aspect of the present invention provides a kit for preventing or treating cancer, which includes: a transposon system that includes a) the transposon vector of claim 1 into which target DNA is inserted; and
b) a nucleic acid molecule including a sequence encoding a transposase protein or transposase,
wherein the target DNA is one or more selected from the group consisting of a tumor antigen-specific CAR coding sequence or a fragment thereof, an oncovirus-specific neutralizing antibody coding sequence or a fragment thereof, an immune checkpoint inhibitor coding sequence, and a tumor antigen-specific T-cell receptor (TCR) coding sequence or a fragment thereof.

### [Advantageous Effects]

The present invention relates to a transposon vector, a transposon system including the same, a transposon kit, a cell into which the transposon vector is inserted, and uses thereof, the present invention was completed by confirming that a foreign gene was effectively delivered to the chromosome of a target cell to produce genetically modified cells at high yield. Particularly, the transposon according to the present invention can effectively deliver a TCR- or CAR-encoding gene to immune cells, and through this, it was confirmed that the TCR- or CAR-expressing cells show high reactivity to an antigen. Therefore, it is expected that various TCR-T cells and CAR-T cells can be produced using the transposon system according to the present invention. Particularly, conventional CAR-T cells require high costs not only for CAR production but also for delivery to target cells, but by using the transposon of the present invention, high-yield CAR-T cells can be obtained at low cost, the price of a CAR-T cell therapeutic is lowered, which reduces the production cost of CAR-T cell therapeutics. In addition, since it was confirmed that the transposon of the present invention can effectively deliver an antibody gene such as a tumor virus-targeted neutralizing antibody gene to HEK293 cells used in the mass production of antibodies, various antibodies can be easily produced in large quantities using the transposon of the present invention. Particularly, since the transposon according to the present invention is a gene delivery vehicle and is not limited to the type of gene that can be delivered, it is expected to be actively used in the development of genome-modified cell lines expressing various genes depending on the purpose, in addition to an antibody gene.

### [Description of Drawings]

FIG. 1 is a schematic diagram of a transposon vector according to one embodiment of the present invention.
FIGS. 2A to 2D show the results of observing the degree of GFP expression 1, 2, 3, and 6 days after inserting one or both of a transposon vector and a transposase vector into T cells according to one embodiment of the present invention using a fluorescence microscope.
FIGS. 3A to 3E show the results of confirming the degree of GFP expression 1, 2, 3, 6, and 7 days after inserting one or both of a transposon vector and a transposase vector into T cells according to one embodiment of the present invention through FACS.

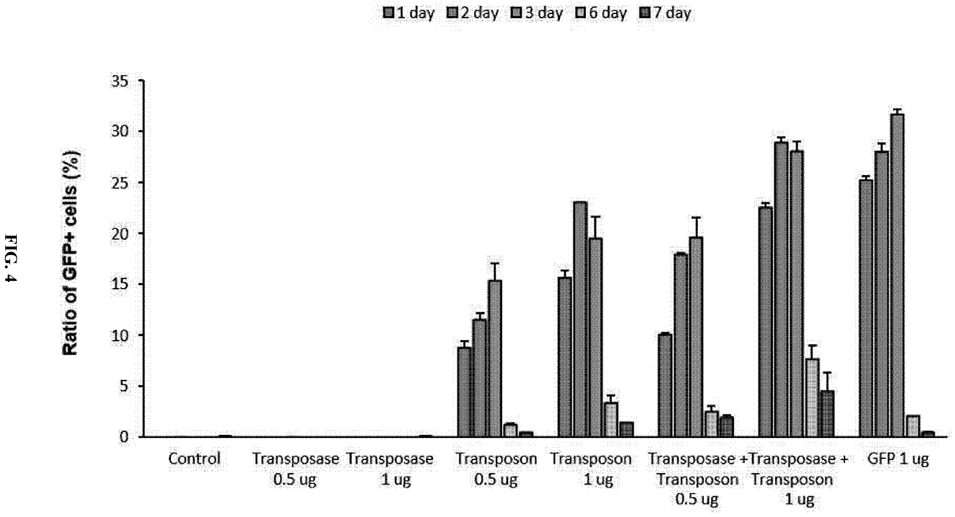

FIG. 4 is a graph showing the degree of GFP expression 1, 2, 3, 6, and 7 days after inserting one or both of a transposon vector and a transposase vector into T cells according to one embodiment of the present invention.
FIG. 5 shows the results of observing the degree of GFP expression in T cells using a fluorescence microscope after single cells expressing GFP are isolated 7 days after inserting a transposon vector and a transposase vector into the T cells according to one embodiment of the present invention, and incubated for 10 days.
FIGS. 6A to 6D show the results of detecting a site of the target DNA integration into the chromosome using a splinkerette PCR method after inserting a transposon vector and a transposase vector into T cells according to one embodiment of the present invention.
FIG. 7A is the map of a transposon original backbone vector for producing a transposon mutant according to the present invention.
FIG. 7B is a schematic diagram of a transposon vector that includes a 5' ITR mutant and a 3' ITR mutant according to the present invention.
FIG. 8A is the result of confirming the degree of GFP expression using a fluorescence microscope 7 days after transfection (electroporation) in an untreated control (Control), a group in which only a pBat transposon is introduced (pBat Transposon only), a group in which only a Piggybac transposase is introduced (pBac), and a GFP plasmid-introduced group (pEGFP).
FIGS. 8B to 8E are the results of confirming the degree of GFP expression using a fluorescence microscope 7 days after transfection in a group in which a B3IS-containing transposon (FIG. 8B), an r3M1 mutant-containing transposon (FIG. 8C), an r3M2 mutant-containing transposon (FIG. 8D), or an r3M3 mutant-containing transposon (FIG. 8E) is introduced.
FIG. 9A is the result of confirming the proportion of GFP-expressing cells through FACS analysis 7 days after transfection in a control group.
FIGS. 9B to 9E are the results of confirming the proportion of GFP-expressing cells through FACS analysis 7 days after transfection in a group in which a B3IS-containing transposon (FIG. 9B), an r3M1 mutant-containing transposon (FIG. 9C), an r3M2 mutant-containing transposon (FIG. 9D), or an r3M3 mutant-containing transposon (FIG. 9E) is introduced.
FIG. 10A is the result of confirming the proportion of GFP-expressing cells 14 days after transfection in a control group through FACS analysis.
FIGS. 10B to 10E are the results of confirming the proportion of GFP-expressing cells through FACS analysis 14 days after transfection in a group in which a B3IS-containing transposon (FIG. 10B), an r3M1 mutant-containing transposon (FIG. 10C), an r3M2 mutant-containing transposon (FIG. 10D), or an r3M3 mutant-containing transposon (FIG. 10E) is introduced.
FIG. 11 is the graph of measuring the proportion of GFP-expressing cells over time after transfection of a transposon vector according to one embodiment of the present invention into cells.
FIG. 12A shows single cell sorting of cells transfected with a transposon vector according to one embodiment of the present invention on day 14 after transfection.
FIG. 12B shows the results of observing GFP expression using a fluorescence microscope on day 31 after transfection after cells transfected with a transposon vector according to one embodiment of the present invention are further incubated through single cell sorting.
FIGS. 13A and 13B show mutant forms selected by alignment of the ITR sequence of each of pBat and piggyBac transposons (FIG. 13A, 5' ITR mutant; FIG. 13B, 3' ITR mutant).
FIG. 14A shows the results of confirming the degree of GFP expression using a fluorescence microscope on day 7 after electroporation in an untreated control (Control), a GFP-expressing plasmid-introduced group (pEGFP), a group in which only a pBat transposon without a transposase is introduced (pBat Transposon only), and a group in which a transposase and an original pBat transposon are introduced (pBat control).
FIGS. 14B to 14F show the results of confirming the degree of GFP expression using a fluorescence microscope on day 7 after electroporation in a group in which a B3IS-containing transposon (FIG. 14B), a 3M1 mutant-containing transposon (FIG. 14C), a 3M2 mutant-containing transposon (FIG. 14D), a 3M3 mutant-containing transposon (FIG. 14E), or a 3M4 mutant-containing transposon (FIG. 14F) is introduced.
FIG. 15A is the result of confirming the proportion of GFP-expressing cells in a control group through FACS analysis 7 days after electroporation.
FIGS. 15B to 15F show the results of confirming the proportion of GFP-expressing cells through FACS analysis 7 days after transfection in a group in which a B3IS-containing transposon (FIG. 15B), a 3M1 mutant-containing transposon (FIG. 15C), a 3M2 mutant-containing transposon (FIG. 15D), a 3M3 mutant-containing transposon (FIG. 15E), or a 3M4 mutant-containing transposon (FIG. 15F) is introduced.
FIG. 16A is the result of confirming the degree of GFP expression using a fluorescence microscope 14 days after transfection in a control group.
FIGS. 16B to 16F are the results of confirming the degree of GFP expression using a fluorescence microscope 14 days after transfection in a group in which a B3IS-containing transposon (FIG. 16B), a 3M1 mutant-containing transposon (FIG. 16C), a 3M2 mutant-containing transposon (FIG. 16D), a 3M3 mutant-containing transposon (FIG. 16E), or a 3M4 mutant-containing transposon (FIG. 16F) is introduced.
FIG. 17A is the result of confirming the proportion of GFP-expressing cells through FACS analysis 14 days after transfection in a control group.
FIGS. 17B to 17F are the results of confirming the degree of GFP-expressing cells through FACS analysis 14 days after transfection in a group in which a B3IS-containing transposon (FIG. 17B), a 3M1 mutant-containing transposon (FIG. 17C), a 3M2 mutant-containing transposon (FIG. 17D), a 3M3 mutant-containing transposon (FIG. 17E), or a 3M4 mutant-containing transposon (FIG. 17F) is introduced.
FIGS. 17G and 17H are the graphs of measuring the proportion of GFP-expressing cells (FIG. 17G) and the proportion of cells expressing high intensity GFP (FIG. 17H) over time after transfection of cells with a transposon vector according to one embodiment of the present invention into GFP-expressing cells.
FIGS. 18A and 18B show the result of confirming the degree of GFP expression using a fluorescence microscope one day (FIG. 18A) or 7 days (FIG. 18B) after inserting pEGFP, a wild-type transposon (Naive-GFP), or a transposon vector according to one embodiment of the present invention into PBMC cells along with a transposase plasmid through electroporation.
FIG. 19A shows the result of confirming the proportions of GFP-expressing CD3⁺ T cells and CD8⁺ T cells in a control group (PBMCs not subjected to electroporation (No EP)), an untreated control (Control), or a pEGFP-introduced group through FACS analysis on day 7 after electroporation.
FIG. 19B show the result of confirming the proportions of GFP-expressing CD3⁺ T and CD8⁺ T cells through FACS analysis on day 7 after introducing Naive-GFP or a transposon vector according to one embodiment of the present invention along with a transposase plasmid into PBMCs by electroporation.
FIG. 20A shows the result of confirming the proportion of GFP-expressing CD3⁺ CD8⁺ T cells through FACS analysis on day 7 after electroporation in a control group or in a group in which a transposon vector and a transposon according to one embodiment of the present invention are introduced into PBMCs by electroporation.
FIG. 20B shows the result of confirming the proportion of GFP-expressing CD3⁺ CD8⁻ T cells through FACS analysis on day 7 after electroporation in a control group or in a group in which a transposon vector and a transposon according to one embodiment of the present invention are introduced into PBMCs by electroporation.
FIG. 21A shows the result of confirming the proportions of 1G4 TCR-expressing CD3⁺ T and CD8⁺ T cells through FACS analysis on day 7 after electroporation in a control group or in a group in which a transposon vector and a transposase plasmid according to one embodiment of the present invention are introduced into PBMCs by electroporation.
FIG. 21B shows the result of confirming the proportions of 1G4 TCR-expressing CD3⁺ T and CD8⁺ T cells through FACS analysis on day 7 after introducing a transposon vector and a transposon according to one embodiment of the present invention into PBMCs by electroporation.
FIG. 22A shows the result of confirming the proportion of 1G4 TCR-expressing CD3⁺ CD8⁺ T cells through FACS analysis on day 7 after electroporation in a control group or a group in which a transposon vector and a transposon according to one embodiment of the present invention are introduced into PBMCs by electroporation.
FIG. 22B shows the result of confirming the proportion of 1G4 TCR-expressing CD3⁺CD8⁻ T cells through FACS analysis on day 7 after electroporation in a control group or a group in which a transposon vector and a transposon according to one embodiment of the present invention are introduced into PBMCs by electroporation.
FIG. 23A shows the result of confirming the proportions of GFP-expressing CD3⁺ T and CD8⁺ T cells through FACS analysis on day 14 after electroporation in a control group.
FIG. 23B shows the result of confirming the proportions of GFP-expressing CD3⁺ T and CD8⁺ T cells through FACS analysis on day 14 after electroporation in a control group or in a group in which a transposon vector and a transposon according to one embodiment of the present invention are introduced into PBMCs by electroporation.
FIG. 24A shows the result of confirming the proportion of GFP-expressing CD3⁺CD8⁺ T cells through FACS analysis on day 14 after electroporation in a control group or in a group in which a transposon vector and a transposon according to one embodiment of the present invention are introduced into PBMCs by electroporation.
FIG. 24B shows the result of confirming the proportion of GFP-expressing CD3⁺CD8⁻ T cells through FACS analysis on day 14 after electroporation in a control group or in a group in which a transposon vector and a transposon according to one embodiment of the present invention are introduced into PBMCs by electroporation.
FIG. 25A shows the result of confirming the proportions of 1G4 TCR-expressing CD3⁺ T and CD8⁺ T cells through FACS analysis on day 14 after electroporation in a control group or in a group in which a transposon vector and a transposase plasmid according to one embodiment of the present invention are introduced into PBMCs by electroporation.
FIG. 25B shows the result of confirming the proportions of 1G4 TCR-expressing CD3⁺ T and CD8⁺ T cells through FACS analysis on day 14 after introducing a transposon vector and a transposon according to one embodiment of the present invention into PBMCs by electroporation.
FIG. 26A shows the result of confirming the proportion of 1G4 TCR-expressing CD3⁺ CD8⁺ T cells through FACS analysis on day 14 after electroporation in a control group or in a group in which a transposon vector and a transposase plasmid according to one embodiment of the present invention are introduced into PBMCs by electroporation.
FIG. 26B is the result of confirming the proportion of 1G4 TCR-expressing CD3⁺ CD8⁻ T cells (CD3⁺ CD4⁺ T cells) through FACS analysis on day 14 after electroporation in a control group or a group in which a transposon vector and a transposase plasmid according to one embodiment of the present invention are introduced into PBMCs by electroporation.
FIG. 27 is the result of confirming the proportion of 1G4 TCR-expressing T cells among CD3⁺ T cells through FACS analysis on day 7 after electroporation after introducing a transposon vector and a transposase plasmid according to one embodiment of the present invention into PBMCs through electroporation and activating T cells with feeder cells (A375) immediately after electroporation (indicated as "immediately after") or one day after electroporation (indicated as "day 1").
FIG. 28 shows the result of confirming the proportion of 1G4 TCR-expressing T cells among CD3⁺ T cells, the proportions of CD4⁺ and CD8⁺ cells, and the proportion of memory-type T cells through FACS analysis on day 10 after electroporation after introducing a transposon vector and a transposase plasmid according to one embodiment of the present invention into PBMCs through electroporation and activating T cells with feeder cells immediately after electroporation or one day after electroporation.
FIG. 29 shows the result of confirming the proportions of total 1G4 TCR-expressing T cells among CD3⁺T cells, CD4⁺ cells, and CD8⁺ cells, and the proportion of memory-type T cells on day 14 after electroporation after introducing a transposon vector and a transposase plasmid according to one embodiment of the present invention into PBMCs through electroporation and activating T cells with feeder cells immediately after electroporation or one day after electroporation.
FIG. 30A shows the result of confirming cell viability over time after introducing a transposon vector and a transposase plasmid according to one embodiment of the present invention into PBMCs through electroporation and activating T cells with feeder cells.
FIG. 30B shows the result of confirming the proportion of TCR-expressing CD3⁺ T cells over time after introducing a transposon vector and a transposase plasmid according to one embodiment of the present invention into PBMCs through electroporation and activating T cells with feeder cells.
FIG. 31 shows the results of confirming the proportions of total T cells expressing CD19 CAR, CD4⁺ cells, and CD8⁺ cells after introducing a transposon vector and a transposase plasmid according to one embodiment of the present invention into PBMCs by electroporation and activating T cells.
FIGS. 32A and 32B show the results of confirming the proportion of total T cells expressing CD19 CAR (FIG. 32A) and the proportions of CD4⁺ cells and CD8⁺ cells (FIG. 32B) after introducing a transposon vector and a transposase plasmid according to one embodiment of the present invention into PBMCs through electroporation and activating T cells.
FIG. 33 is the result of confirming the proportions of total T cells expressing CD19 CAR, CD4⁺ cells, CD8⁺ cells, and memory-type T cells after introducing a transposon vector and a transposase plasmid according to one embodiment of the present invention into PBMCs through electroporation and activating T cells.
FIGS. 34A to 34C are the results of confirming cell viability (FIG. 34A), the proportion of CD19 CAR-expressing CD3⁺ T cells (FIG. 34B), and the proportion of memory-type T cells (FIG. 34C) on day 7 after electroporation after introducing a transposon vector and a transposase plasmid according to one embodiment of the present invention into PBMCs through electroporation and activating T cells.
FIG. 35 is the result of confirming the reactivity of CAR-T cells produced with a transposon system according to one embodiment of the present invention to a B cell line (BJAB) expressing a target antigen (CD19).
FIG. 36A is a set of images that observe GFP-expressing cells using a fluorescence microscope 7 days after electroporation after introducing various forms (plasmid, linear dsRNA, and minicircle dsRNA) of transposon vectors and transposase plasmids into Jukat cells by electroporation.
FIG. 36B shows the results of confirming the proportion of GFP-expressing cells through FACS analysis 7 days after electroporation after introducing various types of transposon vectors, and transposase plasmids into Jukat cells through electroporation.
FIG. 37A are a set of images of GFP-expressing cells observed using a fluorescence microscope 14 days after electroporation after introducing various types of transposon vectors and transposase plasmids into Jukat cells by electroporation.
FIG. 37B shows the results of confirming the proportions of GFP-expressing cells through FACS analysis 14 days after electroporation after various types of transposon vectors and transposase plasmids are introduced into Jukat cells by electroporation.
FIG. 38A shows the result of measuring the proportion of GFP-expressing cells over time after the transfection of a transposon vector according to the present invention.
FIG. 38B shows the result of measuring the proportion of high intensity GFP-expressing cells over time after the transfection of a transposon vector according to the present invention.
FIG. 39A shows the results of observing GFP expression levels of cells over time using a fluorescence microscope after introducing a transposon vector according to one embodiment of the present invention into HEK293 cells.
FIG. 39B shows the results of observing GFP expression levels of cells over time through FACS analysis after introducing a transposon vector according to one embodiment of the present invention into HEK293 cells.
FIG. 40A shows the result of confirming a JWW-2 mRNA expression level over time through qPCR after introducing a transposon vector according to one embodiment of the present invention into HEK293 cells.
FIG. 40B shows the result of confirming a JWW-2 protein expression level over time through ELISA analysis after introducing a transposon vector according to one embodiment of the present invention into HEK293 cells.
FIG. 41A shows the results of confirming GFP expression levels using a fluorescence microscope one day after electroporation in a negative control (EP only), GFP plasmid-treated group (pEGFP), wild-type transposon-treated group (wild type), B3IS-B5IE transposon-treated group, or small-size B3IS-B5IE transposon-treated group to confirm gene transfer efficiency according to the size of a transposon vector.
FIG. 41B shows the results of confirming GFP expression levels through FACS analysis one day after electroporation of various plasmids and transposons into Jurkat cells to confirm gene transfer efficiency according to the size of a transposon vector.
FIG. 42A shows the results of confirming GFP expression levels using a fluorescence microscope 7 days after electroporation of various plasmids or transposons into Jurkat cells.
FIG. 42B shows the results of confirming GFP expression levels through FACS analysis 7 days after electroporation of various plasmids or transposons into Jurkat cells.
FIG. 43A shows the results of confirming GFP expression levels using a fluorescence microscope 14 days after electroporation of various plasmids or transposons into Jurkat cells.
FIG. 43B shows the results of confirming GFP expression levels through FACS analysis 14 days after electroporation of various plasmids or transposons into Jurkat cells.
FIG. 44A shows the result of confirming the proportions of GFP-expressing cells over time by group after electroporation of various plasmids or transposons into Jurkat cells.
FIG. 44B shows the result of confirming the proportions of high intensity GFP-expressing cells over time by group after electroporation of various plasmids or transposons into Jurkat cells.

### [Modes of the Invention]

The present invention relates to a transposon vector, a transposon system including the same, a transposon kit, a cell into which the transposon vector is inserted, and uses thereof, and the present invention was completed by confirming that an exogenous gene is effectively transferred into the chromosome of a target cell, thereby producing genetically modified cells at high yield.

Accordingly, the present invention provides a transposon vector that includes a 5' inverted terminal repeat (5' ITR) having a sequence of at least 71 consecutive nucleotides in the nucleotide sequence represented by SEQ ID NO: 1; and a 3' inverted terminal repeat (3' ITR) having a sequence of at least 66 consecutive nucleotides in the nucleotide sequence represented by SEQ ID NO: 2.

The term "transposon" used herein refers to a polynucleotide that can be translocated by deletion of specific genes from a donor polynucleotide (e.g., vector) and integrated into a target site (e.g., the genome or extrachromosomal DNA of a cell). The transposon is a polynucleotide including cis-acting nucleotide sequences that are located at both sides, wherein at least one cis-acting nucleotide sequence is located at the 5' end of the nucleotide sequence, and the other one is located at the 3' end of the nucleotide sequence. The cis-acting nucleotide sequence includes at least one inverted repeat (IR) at each end of the transposon, called an inverted terminal repeat (ITR), to which a transposase binds. In the present invention, the ITR located at the 5' end of the transposon nucleotide sequence is referred to as 5' ITR, and the ITR located at the 3' end of the transposon nucleotide sequence is referred to as 3' ITR.

The term "vector" used herein refers to a nucleic acid molecule that can transport a different nucleic acid molecule to which it is linked. Specifically, the vector refers to any vehicle for introduction and/or transfer of a base into a host cell *in vitro, ex vivo,* or *in vivo,* and may be a replicon to which another DNA fragment binds, leading to the replication of the bound fragment. The "replicon" refers to any genetic unit that can serve as a self-unit for DNA replication *in vivo,* that is, can be replicated under its own control (e.g., a plasmid, a phage, a cosmid, a chromosome, a virus, etc.). The vector includes a bacterium, a plasmid, a phage, a cosmid, an episome, a virus, and an insertable DNA fragment, that is, a fragment that can be inserted into the host cell genome by homologous recombination, but the present invention is not limited thereto.

The vector according to the present invention may consist of double-stranded DNA, such as plasmid DNA, linear DNA, hair-pin DNA, or mini-circle DNA, or may be a recombinant viral vector, but the present invention is not limited thereto. The vector may be any one that includes a transposon sequence and target DNA and can be delivered into a target cell without limitation, and one with ordinary skill in the art may select and use various vectors known in the art.

The recombinant vector of the present invention preferably includes a promoter, which is a transcription initiation factor to which RNA polymerase binds, any operator sequence for regulating transcription, an enhancer sequence, a sequence encoding a suitable mRNA ribosome-binding site, a sequence regulating the termination of transcription and translation, and a terminator, and more preferably, further includes a polyhistidine-tag (amino acid motif consisting of at least five histidine residues), a signal peptide gene, an endoplasmic reticulum retention signal peptide, and a cloning site, and a selection marker gene such as a gene for tagging or an antibiotic resistance gene for selecting a transformant. In the recombinant vector, the polynucleotide sequence of each gene is operably linked to a promoter. The term "operably linked" used herein refers to a functional linkage between a nucleotide expression regulatory sequence such as a promoter sequence and another nucleotide sequence, whereby the regulatory sequence regulates the transcription and/or translation of the other nucleotide sequence.

The recombinant vector may be constructed using prokaryotic cells or eukaryotic cells as a host. For example, when the vector of the present invention is an expression vector and a prokaryotic cell is used as a host, it generally includes a potent promoter that can advance transcription (e.g., a pLλ promoter, a trp promoter, a lac promoter, a tac promoter, or a T7 promoter), a ribosome-binding site for initiating translation, and a sequence terminating transcription/translation. When a eukaryotic cell is used as a host, the origin of replication where a vector operates in the eukaryotic cell may include, but is not limited to, a f1 replication origin, an SV40 replication origin, a pMB1 replication origin, an adeno replication origin, an AAV replication origin, or a BBV replication origin. In addition, a promoter derived from the genome of mammalian cells (e.g., metallothionein promoter) or a promoter derived from mammalian viruses (e.g., adenovirus late promoter, vaccinia virus 7.5K promoter, SV40 promoter, cytomegalovirus promoter, or HSV tk promoter) may be used. The recombination vector may generally include a polyadenylation sequence as a transcription termination sequence. In addition, the recombination vector may include a poly A signal as a signal sequence, but the present invention is not limited thereto.

The gene for tagging may be representatively an Avi tag, a calmodulin tag, a polyglutamate tag, an E tag, a FLAG tag, an HA tag, a His tag (polyhistidine-tag), a Myc tag, an S tag, an SBP tag, an IgG-Fc tag, a CTB tag, a Softag 1 tag, a Softag 3 tag, a Strep tag, a TC tag, a V5 tag, a VSV tag, and an Xpress tag. Preferably, the vector according to the present invention includes a Myc tag.

In the present invention, the vector may be delivered into a cell by various techniques typically used to introduce an exogenous nucleic acid into prokaryotic or eukaryotic host cells. For example, the vector according to the present invention may be inserted into cells by calcium phosphate coprecipitation; electroporation; microfluidics gene editing; nucleofection; cell squeezing; sonoporation; optical transfection; impalefection; a gene gun; magnetofection; viral transduction; DEAE-dextran transfection; lipofection; or transfection with a dendrimer, a liposome, or a cationic polymer, but the present invention is not limited thereto.

The term "nucleic acid" or "nucleic acid molecule" used herein has a meaning comprehensively including DNA (gDNA and cDNA) and RNA molecules. Nucleotides, the basic structural units of nucleic acids, include not only natural nucleotides, but also analogues with modified sugar or base sites. The sequence of a nucleic acid according to the present invention may be modified. The modification includes the addition, deletion, or non-conservative or conservative substitution of nucleotides. The nucleic acid according to the present invention includes a nucleotide sequence that has substantial identity to the above nucleotide sequence. The substantial identity indicates a nucleotide sequence having at least 80%, preferably 90%, and most preferably 95% homology when the nucleotide sequence of the present invention and any other sequence are aligned to match as much as possible, and the aligned sequence is analyzed using an algorithm conventionally used in the art.

That is, in the present invention, a polynucleotide consisting of a base sequence represented by a particular sequence number is not limited to only a corresponding base sequence, and variant of the base sequence are included in the scope of the present invention. A nucleic acid molecule consisting of a base sequence represented by a specific sequence number is a concept including variants in which a functional equivalent of a nucleic acid molecule constituting it, for example, a part of the base sequence of the nucleic acid molecule has been modified by deletion, substitution or insertion, but are capable of performing the same functional action. Specifically, the polynucleotide disclosed herein includes preferably a base sequence having 70% or more, more preferably, 80% or more, and even more preferably, 90% or more, and most preferably, 95% or more sequence homology to a base sequence represented by a specific sequence number. For example, the polynucleotide disclosed herein includes a polynucleotide having 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100% sequence homology. "% sequence homology" for a polynucleotide is determined by comparing two optimally aligned sequences with a comparison region, and a part of the polynucleotide sequence in the comparison region may include an addition or deletion (that is, a gap) compared with a reference sequence (not including an addition or deletion) for the optimal alignment of two sequences.

The term "transfection efficiency" refers to the number of cells containing an introduced polynucleotide within a population of host cells. Generally, the transfection efficiency may be determined by transfecting a polynucleotide encoding a reporter gene, such as GFP, into a population of target cells. Accordingly, the transfection efficiency may be determined by analyzing a gene product encoded by the introduced polynucleotide. For example, the transfection efficiency is determined by measuring the number of cells with GFP activity.

The transposon vector according to the present invention may include one or more 5' ITRs and one or more 3' ITRs.

A transposon vector according to the present invention including one or more 5' ITRs and one or more 3' ITRs will be described below.

The nucleotide sequence of the 5' ITR may include the 5' to 3' sequence thereof in the 5' end of the transposon vector (or transposon encoding a polynucleotide molecule) in the 5' to 3' direction, and/or the nucleotide sequence of the 3' ITR may include the 5' to 3' sequence thereof in the 3' end of the transposon vector in the 5' to 3' direction. In other words, the nucleotide sequence of the 5' ITR may be included upstream of a position where target DNA is inserted into the transposon vector in the 5' to 3' direction and/or the nucleotide sequence of the 3' ITR may be included downstream of a position where the target DNA is inserted into the transposon vector in the 5' to 3' direction. Being included in the 5' end or 3' end of the vector in the 5' to 3' direction means being included in the 5' to 3' direction of a sense strand of a polynucleotide molecule.

The 5' ITR according to the present invention may have a sequence of at least 71 consecutive nucleotides among the 157-nucleotide sequence represented by SEQ ID NO: 1. Preferably, the sequence of at least 71 consecutive nucleotides refers to a sequence of at least 71 consecutive nucleotides in the 5' to 3' direction among the nucleotide sequence represented by SEQ ID NO: 1. Here, the sequence of at least 71 consecutive nucleotides may be selected from the entire nucleotide sequence represented by SEQ ID NO: 1. For example, the first nucleotide ('T' in SEQ ID NO: 1) to at least the 71 nucleotide may be selected in the 5' to 3' direction of the nucleotide sequence represented by SEQ ID NO: 1, but the present invention is not limited thereto. For example, the 5' ITR may have a sequence of at least 70, 80, 90, 100, 110, 120, 130, 140, or 150 consecutive nucleotides among the 157-nucleotide sequence represented by SEQ ID NO: 1. In addition, the 5' ITR has fewer than 157, 140, 130, 120, or 115 nucleotides. Here, to select at least 71 nucleotides, selection may start sequentially from the first nucleotide, or may be performed by deleting, adding, or modifying some nucleotides.

The 5' ITR according to the present invention may have a sequence of not more than 71 nucleotides among the 157-nucleotide sequence represented by SEQ ID NO: 1, but must have a sequence exceeding 33 nucleotides. For example, when the 5' ITR according to the present invention is included in a transposon vector and exhibits an effective effect as a desired transposon vector of the present invention, the 5' ITR may have a sequence of at least 34, 35, 38, 40, 50, or 60 nucleotides among the 157-nucleotide sequence represented by SEQ ID NO: 1, but the sequence is not limited to at least 71 nucleotides. Here, to select more than 33 nucleotides, selection may start sequentially from the first nucleotide in the 5' to 3' direction of the nucleotide sequence represented by SEQ ID NO: 1, or may be performed by deleting, adding, or modifying some nucleotides.

For example, the 5' ITR according to the present invention includes the nucleotide sequence represented by SEQ ID NO: 7 (5'-TTAACACTTGGATTGCGGGAAACGAG-3'). Here, SEQ ID NO: 7 corresponds to the first to 26^{th} nucleotides from the 5'end of the nucleotide sequence represented by SEQ ID NO: 1.

For example, the 5' ITR according to the present invention includes a nucleotide sequence (8mer) represented by 5'-ACACTTGG-3'. The sequence corresponds to the 4^{th} to 11^{th} nucleotides from the 5' end of the nucleotide sequence represented by SEQ ID NO: 1.

For example, the 5' ITR according to the present invention includes the nucleotide sequence (15mer) represented by SEQ ID NO: 8 (5'-TGCGGGAAACGAGTT-3'). Here, the SEQ ID NO: 8 corresponds to the 14^{th} to 28^{th} nucleotides from the 5' end of the nucleotide sequence represented by SEQ ID NO: 1.

For example, the 5' ITR according to the present invention includes one or more of the nucleotide sequences represented by the above-described SEQ ID NO: 7, 5'-ACACTTGG-3', and SEQ ID NO: 8.

In one embodiment of the present invention, the 5' ITR may be any one selected from the group consisting of the following 5' ITRs:
a) a 5' ITR having the nucleotide sequence represented by SEQ ID NO: 1;
b) a 5' ITR having the nucleotide sequence represented by SEQ ID NO: 5; and
c) a 5' ITR having the nucleotide sequence represented by SEQ ID NO: 6.

The 3' ITR according to the present invention may have a sequence of at least 66 nucleotides among the 212-nucleotide sequence represented by SEQ ID NO: 2. Here, the sequence of at least 66 nucleotides may be selected from the entire nucleotide sequence represented by SEQ ID NO: 2, and the first nucleotide to at least the 66 nucleotide in a 3' to 5' direction of the sense strand nucleotide sequence represented by SEQ ID NO: 2 ('A' in SEQ ID NO: 2), but the present invention is not limited thereto. For example, the 3' ITR may have a sequence of at least 60, 70, 80, 90, 100, 110, 120, 130, 140, 150, 160, 170, 180, 190, 200, or 210 consecutive nucleotides among the 212-nucleotide sequence represented by SEQ ID NO: 2. In addition, the 3' ITR includes fewer than 212, 200, 190, 180, 170, or 160 nucleotides. Here, to select at least 66 nucleotides, selection may start sequentially from the first nucleotide, or may be performed by deleting, adding, or modifying some nucleotides.

The 3' ITR according to the present invention may have a sequence of at least 66 nucleotides among the 212-nucleotide sequence represented by SEQ ID NO: 2, but must have a sequence exceeding 37 nucleotides. For example, when the 3' ITR according to the present invention is included in the transposon vector and exhibits an effective effect as a desired transposon vector of the present invention, the 3' ITR may have at least 40, 50, or 60 nucleotides among the 212-nucleotide sequence represented by SEQ ID NO: 2, but the sequence is not limited to at least 66 nucleotides. Here, to select more than 37 nucleotides, selection may start sequentially from the first nucleotide in a 3' to 5' direction of the sense strand nucleotide sequence represented by SEQ ID NO: 2, or may be performed by deleting, adding, or modifying some nucleotides.

For example, the 3' ITR according to the present invention includes the nucleotide sequence (30mer) represented by SEQ ID NO: 13 (5'-ttggcgggaaattcacccgacaccgtagtg-3'). Here, the SEQ ID NO: 13 corresponds to the 5^{th} to 34^{th} nucleotides from the 3' end of the nucleotide sequence represented by SEQ ID NO: 2.

For example, the 3' ITR according to the present invention includes the nucleotide sequence (18mer) represented by SEQ ID NO: 14 (5'-aactctgattttgcgcgg-3'). Here, the SEQ ID NO: 14 corresponds to the 69^{th} to 86^{th} nucleotides from the 3' end of the nucleotide sequence represented by SEQ ID NO: 2.

For example, the 3' ITR according to the present invention includes one or more of the nucleotide sequences represented by SEQ ID NO: 13, and SEQ ID NO: 14.

In one embodiment of the present invention, the 3' ITR may be any one selected from the group consisting of the following 3' ITRs:
a) a 3' ITR having the nucleotide sequence represented by SEQ ID NO: 2;
b) a 3' ITR having the nucleotide sequence represented by SEQ ID NO: 9;
c) a 3' ITR having the nucleotide sequence represented by SEQ ID NO: 10; and
d) a 3' ITR having the nucleotide sequence represented by SEQ ID NO: 11.

The transposon vector according to the present invention includes a combination of one or more 5' ITRs and one or more 3' ITRs described above, wherein
the nucleotide sequence of the 5' ITR may be included in the 5' to 3' direction in the 5' end of the transposon vector, or the 5' to 3' sequence of antisense DNA having the reverse complement sequence of the nucleotide sequence of the 5' ITR represented by the above-mentioned sequence number may be included in the 5' to 3' direction in the 5' end of the transposon vector. In other words, the nucleotide sequence of the 5' ITR may be included upstream of a position where target DNA is inserted into the transposon vector in the 5' to 3' direction, or antisense DNA having the reverse complement sequence of the nucleotide sequence of the 5' ITR may be included upstream of a position where target DNA is inserted into the transposon vector in the 5' to 3' direction.

In addition, the nucleotide sequence of the 3' ITR may be included in the 3' end of the transposon vector in the 5' to 3' direction, or the 5' to 3' nucleotide sequence of antisense DNA having the reverse complement sequence of the 3' ITR represented by the above-described sequence number may be included in the 3' end of the transposon vector in the 5' to 3' direction of the sense strand. In other words, the nucleotide sequence of the 3' ITR may be included downstream of a position where target DNA is inserted into the transposon vector in the 5' to 3' direction, or antisense DNA having the reverse complement sequence of the nucleotide sequence of the 3' ITR may be included downstream of a position where target DNA is inserted into the transposon vector in the 5' to 3' direction.

For example, the nucleotide sequence (3M3) of the 3' ITR represented by SEQ ID NO: 9 is 5'- aacctaaataattgcccgcgccatcttatattttggcgggaaattcacccgacaccgtagtgttaa-3', and when the 3' ITR is sequentially included in the 3' end of the sense strand of the transposon vector in the 5' to 3' direction, it is included in the 3' end of the transposon vector in the sequence order of 5'-aacctaaataattgcccgcgccatcttatattttggcgggaaattcacccgacaccgtagtgttaa-3'. On the other hand, when the 5' to 3' nucleotide sequence of the antisense strand of the 3' ITR represented by SEQ ID NO: 9 is included in the 3' end of the sense strand of the transposon vector in the 5' to 3' direction, it is included in the 3' end of the transposon vector in the sequence order of 5'-ttaacactacggtgtcgggtgaatttcccgccaaaatataagatggcgcgggcaattatttaggtt-3'. In one embodiment, the reverse complement sequence of the nucleotide sequence of 3' ITR represented by SEQ ID NO: 9 is represented by SEQ ID NO: 15 (r3M3).

In one embodiment of the present invention, the reverse complement sequence of the 3' ITR may include a nucleotide sequence represented by any one of SEQ ID NOs: 15 to 17.

The transposon vector according to the present invention may include a combination of one or more 5' ITRs and one or more 3' ITRs described above.

For example, the combination may include a combination of the 5' ITR having the nucleotide sequence represented by SEQ ID NO: 1 and the 3' ITR having the nucleotide sequence represented by SEQ ID NO: 11, and using the above-described three types of 5' ITRs (B51E, 5M3, and 5M4) and the above-described seven types of 3' ITRs (B3IS, 3M1, 3M2, 3M3, r3M1, r3M2, and r3M3), transposons that include a total of 21 combinations of 5' ITRs and 3' ITRs may be obtained, but the present invention is not limited to these 21 combinations.

In one embodiment of the present invention, the transposon vector may be a transposon vector that includes one or more target DNA sequences downstream of the 5' ITR and upstream of the 3' ITR, but the present invention is not limited thereto.

In the present invention, "target DNA" refers to an exogenous DNA molecule that is intended to be delivered into a cell using the transposon of the present invention. The target DNA is any one that can be expressed after being inserted into the transposon vector and then introduced into a target cell. That is, it is obvious that the target DNA is not limited to a specific type of DNA, and one of ordinary skill in the art may select a desired target DNA depending on the purpose without limitation.

In one embodiment of the present invention, the target DNA sequence may encode an antibiotic resistance protein, a therapeutic polypeptide, siRNA, miRNA, a reporter protein, a cytokine, a kinase, an antigen, an antigen-specific receptor, a cytokine receptor, a suicide polypeptide, a recombinant antibody, a neutralizing antibody against one of various viruses and other antigens, or a part thereof. The target DNA sequence may encode, for example, a chimeric antigen receptor (CAR), a T cell receptor (TCR), or a part thereof, but the present invention is not limited thereto.

In the present invention, the "therapeutic polypeptide" refers to a polypeptide or peptide that is effective in preventing, improving, and/or treating any disease, and one of ordinary skill in the art may suitably select a polypeptide that is effective in treating a specific disease depending on the purpose. The disease is not limited to a specific type, but in one embodiment, the disease may be cancer.

In one embodiment of the present invention, a promoter may be operably linked to the transposon vector downstream of the 5' ITR and upstream of the 3' ITR, but the present invention is not limited thereto.

For example, the transposon vector may further include a promoter downstream of the 5' ITR and upstream of a target DNA cloning site.

The "operably linked" refers to a functional linkage between a nucleic acid expression regulatory sequence (e.g., a promoter, a signal sequence, or an array at a transcription regulatory factor-binding site) and another nucleotide sequence, whereby the regulatory sequence regulates the transcription and/or translation of the other nucleotide sequence.

The promoter may include, for example, a cytomegalovirus (CMV) promoter, a Rous sarcoma virus (RSV) promoter, a lac promoter, a T7 promoter, a simian virus 40 (SV40) promoter, a mouse mammary oncovirus (MMTV) promoter, a phosphoglycerate kinase promoter, a chicken β actin (CAG) promoter, an elongation factor 1-α (EF1α) promoter, a human H1 promoter, and a U6 promoter, but the present invention is not limited thereto.

In one embodiment of the present invention, the transposon vector may also be operably linked to one or more of an enhancer, a silencer, an insulator, a terminator, and a poly-A signal, as well as the promoter downstream of the 5' ITR and upstream of the 3' ITR, but the present invention is not limited thereto.

The enhancer may include, for example, a CMV enhancer, but the present invention is not limited thereto.

For example, the transposon vector may include a promoter, one or more target DNAs, and a poly-A signal, and the 5' ITR, the promoter, the target DNA, the poly-A signal, and the 3' ITR may be sequentially operably linked.

Alternatively, the transposon vector may further include an enhancer, and the 5' ITR, the enhancer, the promoter, the target DNA, the poly-A signal, and the 3' ITR may be sequentially operably linked, but the present invention is not limited thereto.

The transposon vector of the present invention may be a DNA molecule (ds DNA), but its specific form is not limited. The transposon vector of the present invention is preferably a circular plasmid, linear ds DNA, or minicircle DNA, but the present invention is not limited thereto. The linear dsDNA may be obtained by synthesis, or cleaving a circular plasmid with a restriction enzyme. The "minicircle DNA" refers to a nucleic acid molecule typically lacking any plasmid/vector backbone sequence required for replication, such as a prokaryotic antibiotic resistance gene and a prokaryotic origin of replication, and a smaller circular DNA molecule than a common plasmid. The minicircle DNA may be produced *in vivo* from a bacterial plasmid by site-specific intramolecular recombination between recombinase recognition sites on the plasmid, producing a bacterial plasmid backbone DNA-free minicircle DNA vector is produced, but the present invention is not limited thereto. For example, the minicircle DNA may be produced by an enzymatic digestion/ligation method, and may be prepared using a commercially available kit, that is, a minicircle DNA production kit (System Biosciences, CA, USA). In addition, the transposon according to the present invention may be hairpin dsDNA. The hairpin structure is a structure in which bonds between base pairs in single-stranded DNA are formed, and is formed when two regions in one strand are reverse-complementary. Since the hairpin dsDNA-type transposon has a loop structure with no truncated ends, it is more stable than linearized dsDNA. The hairpin dsDNA-type transposon may be produced by linearization through cleaving a circular plasmid with a restriction enzyme, and forming both ends of the linearized dsDNA molecule in a hairpin shape (e.g., a linear covalently closed (LCC) DNA mini vector, a minimalistic immunogenic defined gene expression vector (MIDGE), or a micro-linear vector (MiLV)). A method of forming both ends of a plasmid or linearized plasmid in a hairpin shape is known in the art, and specifically, reference can be made to [Mol Ther Methods Clin Dev. 2020 Jan 16; 17:359-368].

In addition, the transposon vector according to the present invention may have a size of 1,000 to 20,000 bp, but the present invention is not limited thereto. The present inventors confirmed through specific examples that the transposon vectors according to the present invention have an excellent gene transfer function, and particularly confirmed that the smaller the transposon vector, the higher the gene transfer efficiency. Accordingly, the size of the transposon vector may be 1,000 to 20,000 bp, 1,000 to 15,000 bp, 1,000 to 13,000 bp, 1,000 to 10,000 bp, 1,000 to 9,000 bp, 1,000 to 8,000 bp, 1,000 to 7,000 bp, 1,000 to 6,000 bp, 1,000 to 5,000 bp, 1,000 to 4,000 bp, or 1,000 to 3,000 bp, but the present invention is not limited thereto.

In addition, the present invention provides a transposon system for delivering target DNA, which includes a) the transposon vector according to the present invention into which target DNA is inserted; and
b) a nucleic acid molecule including a sequence encoding a transposase protein or transposase.

A "transposase" used herein refers to an enzyme that recognizes and binds to both ends (particularly, inverted terminal repeats) of a transposon, cleaves the corresponding part, and then transports and inserts a gene fragment (i.e., target DNA-containing site) between both ends into a different position in the chromosome. In the present invention, the transposase is sufficient for inserting (or integrating) target DNA present between the 5' ITR and 3' ITR into the chromosome of a target cell by binding to the 5' ITR and 3' ITR of the transposon according to the present invention and cleaving them, and its specific type is not limited. For example, in the present invention, the transposase includes a recombinant transposase, which is artificially produced, as well as a natural transposase, without limitation. In one embodiment of the present invention, the transposase may be pBat transposase.

In the present invention, the transposase protein itself may be introduced into cells, or may be expressed in cells after being introduced into the cells in the form of a nucleic acid molecule (DNA or RNA molecule) that includes a sequence encoding the transposase protein.

In one embodiment of the present invention, the nucleic acid molecule that includes a sequence encoding the transposase may be selected from the following materials:
i) a transposase protein including the amino acid sequence represented by SEQ ID NO: 18;
ii) a vector (transposase vector or transposase plasmid) including the nucleotide sequence represented by SEQ ID NO: 19, and
iii) an mRNA molecule including the nucleotide sequence represented by SEQ ID NO: 20.

That is, the transposase vector may have the nucleotide sequence represented by SEQ ID NO: 19, but the present invention is not limited thereto, and may include, for example, the sequence of ThyPLGMH, mycPBase, TPLGMH, or HAhyPBase.

The transposase vector may be produced by a conventional method known in the art, for example, the method suggested by Yaa-Jyuhn James Meir et al. (A versatile, highly efficient, and potentially safer piggyBac transposon system for mammalian genome manipulations, FASEB, 2013: 27, 4429-4443), but the present invention is not limited thereto.

The transposase vector may include a promoter that is operably linked to the nucleotide sequence encoding a transposase.

The promoter may be selected from, for example, a cytomegalovirus (CMV) promoter, a Rous sarcoma virus (RSV) promoter, a lac promoter, a T7 promoter, a simian virus 40 (SV40) promoter, a mouse mammary oncovirus (MMTV) promoter, a phosphoglycerate kinase promoter, a chicken β actin (CAG) promoter, an elongation factor 1-α (EF1α) promoter, a human H1 promoter, and a U6 promoter, but the present invention is not limited thereto.

Meanwhile, a polypeptide including an amino acid sequence represented by a specific sequence number is not limited only to the corresponding amino acid sequence, and a variant of the amino acid sequence is included in the scope of the present invention. A polypeptide molecule consisting of an amino acid sequence represented by a specific sequence number is a concept that includes variants in which a functional equivalent of a polypeptide molecule constituting it, for example, a part of the amino acid sequence of the polypeptide molecule has been modified by deletion, substitution or insertion, but are capable of performing the same functional action as a corresponding polypeptide. Specifically, the polypeptide disclosed in the present invention may include an amino acid sequence having preferably 70% or more, more preferably 80% or more, more preferably 90% or more, and most preferably 95% or more sequence homology with an amino acid sequence represented by a specific sequence number. For example, the polypeptide disclosed in the present invention may include polypeptides having 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, and 100% sequence homology. "% sequence homology" for a polypeptide is determined by comparing two optimally aligned sequences with a comparison region, and a part of the polypeptide sequence in the comparison region may include an addition or deletion (that is, a gap) compared to a reference sequence for the optimal alignment of two sequences (not including an addition or deletion).

In addition, the present invention provides a transposon kit for target DNA delivery that includes the transposon system for target DNA delivery and instructions.

The instructions include pamphlets, recordings, diagrams, or other presentation media (e.g., a CD, VCD, DD, and USB) that can be used to convey or teach how to use the transposon system of the present invention. The instructions may be attached to a container, or independently packaged from a container including the transposon system of the present invention.

The kit may further include a container for containing the transposon system of the present invention.

In addition, the kit may further include a buffer solution for stabilizing the transposon system and/or performing cell transfection. The buffer solution may be, for example, phosphate-buffered saline, tris-based saline, a Tris-EDTA buffer, a 4-(2-hydroxyethyl)-1- piperazine ethanesulfonic acid buffer, or a (N,N-bis(2-hydroxyethyl)-2-amino ethanesulfonic acid (BES) buffer, but the present invention is not limited thereto.

In addition, the present invention provides a cell into which a) a target DNA-inserted transposon vector; and
b) a nucleic acid molecule including a sequence encoding a transposase protein or transposase are introduced.

In the present invention, in the cell, the target DNA in the transposon vector may be cleaved by the transposase, and the cleaved target DNA may be integrated into the genome of the cell. That is, the target DNA may be inserted into the genome of a target cell by the transposon and transposase according to the present invention and stably expressed. The target DNA inserted into the cell genome may be expressed for 5, 7, 10, 15, 20, or 30 days or more in the cell after introducing the transposon vector and the transposase into the cell, but the present invention is not limited thereto.

That is, the present invention provides a cell that is genetically manipulated by inserting target DNA into the genome by the transposon. The "manipulation" in the present invention refers to any manipulation of a cell causing a detectable change in the cell. Here, manipulation includes the insertion of a heterologous/homologous polynucleotide and/or polypeptide into cells, and the mutation of a polynucleotide and/or polypeptide native to cells, but the present invention is not limited thereto.

In one embodiment of the present invention, the cells may be one or more immune cells selected from the group consisting of bone marrow cells such as T cells, B cells, natural killer cells, monocytes, macrophages, eosinophils, mast cells, basophils, and granulocytes, and dendritic cells; or stem cells derived from bone marrow, adipose tissue, peripheral blood, cord blood, or dental pulp, but the present invention is not limited thereto. In addition, the cells may be insect-derived cells, plant-derived cells, fish-derived cells, or mammalian cells, particularly, human-derived cells, but the present invention is not limited thereto.

The term "immune cells" used herein refers to cells participating in an immune response.

In addition, the cells may be co-cultured with feeder cells after introducing the transposon vector and transposase (a protein or a nucleic acid molecule). The feeder cells refer to auxiliary cells that provide extracellular secretions, including growth factors, so that the cells into which target DNA is introduced can proliferate without proliferating themselves. The feeder cells are not limited to specific types, and any cells known to act as feeder cells in the art may be applied without limitation. Non-limiting examples may include fibroblasts, human bone marrow-derived mesenchymal cells, human amniotic epithelial cells, adipose-derived mesenchymal stem cells, and melanoma cells (A375 cells). Preferably, the feeder cells may be irradiated in advance before being co-cultured with the cells into which the target DNA has been introduced.

The co-culture with the feeder cells may contribute to enhancing gene delivery efficiency, especially by introducing a CAR or TCR into immune cells using the transposon system according to the present invention, and enhancing the proliferation of the gene-introduced cells and the expression rate of the gene. A method of activating the gene-introduced cells is not limited to the co-culture with feeder cells, and an appropriate cell activation method may be used without limitation according to the type of target cells. For example, when the cells are T cells, they may be activated using Transact or Dynabeads.

The co-culture with the feeder cells is preferably performed immediately after introducing a transposon vector and a transposase into cells through electroporation, but the present invention is not limited thereto. The co-culture may be performed within 1 to 10 days, 1 to 5 days, 1 to 3 days, 1 to 2 days, 1 day, 20 hours, 10 hours, 5 hours, 3 hours, 1 hour, 30 minutes, or 10 minutes after introduction.

In addition, the present invention provides a method of inserting a target DNA sequence into the genome of a cell, which includes introducing a) the transposon vector into which target DNA is inserted; and
b) a nucleic acid molecule including a sequence encoding a transposase protein or transposase into cells.

In addition, the method may further include co-culturing the transposon vector-inserted cells with feeder cells after the introduction.

The term "introduction" used herein refers to the introduction (delivery) of a polynucleotide (e.g., a transposon vector or a transposase vector) into cells or an organism. Polynucleotides may be in the form of naked DNA or RNA, or may bind to various proteins or integrated into a vector. The term "introduction" used herein means delivery in the broadest possible sense, for example, introduction by a transfection method (a method of introducing a polynucleotide into eukaryotic cells through physical and/or chemical treatment), a transformation method (a method of introducing a polynucleotide into prokaryotic cells through physical and/or chemical treatment), a virus method/viral transduction method (a method of introducing a polynucleotide into eukaryotic and/or prokaryotic cells via a virus or viral vector), a conjugation method (a method of introducing a polynucleotide from one cell to another by direct cell-to-cell contact or by a cytoplasmic bridge between cells), and a fusion method (a method of fusing two cells including homotypic cell fusion and heterotypic cell fusion). Preferably, the introduction is performed by electroporation.

In addition, the present invention provides a composition for various purposes, which includes cells into which target DNA is inserted into the genome via the transposon vector according to the present invention. Here, the cells may be autologous or allogeneic cells.

In one embodiment of the present invention, a pharmaceutical composition for preventing or treating an immune-related disease, which includes the cells of the present invention as an active ingredient, is provided.

The term "immune-related disease" used herein refers to a disease and/or condition in which the immune system is involved in the pathogenesis of a disease, or the appropriate stimulation or inhibition of the immune system can lead to treatment and/or prevention from a disease. An exemplary immune-related disease that can be treated by the present invention may include a tumor, an inflammatory disease, an allergy, an autoimmune disease, a graft-versus-host disease, or an inflammatory disease, but the present invention is not limited thereto.

Through specific examples, the present inventors confirmed that CAR T cells produced using the transposon of the present invention differentiate into cytotoxic T cells and memory T cells in response to antigens. Accordingly, those of ordinary skill in the art may use the transposon of the present invention to deliver an appropriate antigen-specific CAR or TCR gene into immune cells, thereby producing genetically manipulated cells, which have a more activated immune function, and may use these cells to prevent or treat an immune-related disease.

For example, those of ordinary skill in the art may enhance the immune function of cells to a target antigen by inserting a gene encoding the corresponding antigen into the transposon according to the present invention and delivering the gene to the immune cells. Enhancing immune function may refer to activating the function of, for example, antigen-presenting cells, natural killer cells, or T cells (particularly, cytotoxic T cells) against the corresponding antigen, but regulating the activity of regulatory T cells, myeloid-derived suppressor cells (MDSCs), or M2 macrophages. However, the present invention is not limited thereto.

Particularly, the present invention provides a pharmaceutical composition for preventing or treating cancer, which includes immune cells into which a) a target DNA-inserted transposon vector; and
b) a nucleic acid molecule including a sequence encoding a transposase protein or transposase are introduced as an active ingredient,
wherein the target DNA is one or more selected from the group consisting of a tumor antigen-specific CAR coding sequence or a fragment thereof, an oncovirus-specific neutralizing antibody coding sequence or a fragment thereof, an immune checkpoint inhibitor coding sequence, and a tumor antigen-specific TCR coding sequence or a fragment thereof.

In the present invention, the immune cells may express a tumor antigen-specific CAR or TCR on the cell surface by inserting the tumor antigen-specific CAR, tumor antigen-specific TCR, or a functional fragment thereof into the chromosome, and thus respond to a tumor antigen.

The specific description of the tumor antigen is as described above.

The oncovirus refers to a virus that causes cancer, and an oncovirus antigen refers to a protein, enveloped virus, or toxin, which specifically produces a corresponding oncovirus. The oncovirus antigen may be, for example, a cytomegalovirus (CMV) antigen, an Epstein-Barr virus (EBV) antigen, a human papilloma virus (HPV) antigen, a hepatitis B virus (HBV) antigen, a hepatitis C virus (HCV) antigen, a human immunodeficiency virus (HIV) antigen, a human herpes virus-8 (HHV-8) antigen, or a human T-lymphotrophic virus (HTLV-1) antigen, but the present invention is not limited thereto. The oncovirus antigen may be any virus-specific antigen that causes cancer without limitation.

The neoantigen refers to an antigenic peptide that is specifically generated only in cancer cells. A neoantigen is expressed only in cancer cells, not in normal cells, and in the case that the neoantigen is presented on the surface of antigen-presenting cells that absorbs it, it may bind to a T cell receptor, inducing an immune response. Neoantigens include both shared neoantigens and personalized neoantigens. Shared neoantigens refer to neoantigens with high sharing frequency, which are commonly shown in two or more patients. Personalized neoantigens are neoantigens that specifically shown only in specific patients, enabling patient-customized treatment by targeting them.

The immune checkpoint inhibitor may include any one that can inhibit an immune checkpoint expressed in immune cells or cancer cells without limitation. That is, the immune checkpoint may be an antibody that targets an immune checkpoint, and specific examples of the immune checkpoint inhibitors may include an anti-PD-L1 antibody, an anti-PD-1 antibody, an anti-CTLA-4 antibody, an anti-LAG3 antibody, an anti-TIM3 antibody, an anti-BTLA antibody, an anti-4-1BB antibody, an anti-OX40 antibody, or a functional fragment thereof, but the present invention is not limited thereto.

The immune cells may be selected from T cells, NK cells, B cells, dendritic cells, and macrophages, and preferably, T cells.

The "cancer" used herein includes both solid cancer and blood cancer. In one embodiment of the present invention, the cancer may be one or more selected from the group consisting of breast cancer, colorectal cancer, lung cancer, head and neck cancer, small cell lung cancer, stomach cancer, liver cancer, blood cancer, bone cancer, pancreatic cancer, skin cancer, head cancer, neck cancer, skin melanoma, intraocular melanoma, uterine cancer, ovarian cancer, rectal cancer, anal cancer, colon cancer, fallopian tube carcinoma, endometrial carcinoma, cervical cancer, vaginal cancer, vulvar carcinoma, Hodgkin's disease, esophageal cancer, small intestine cancer, endocrine gland cancer, thyroid cancer, parathyroid cancer, adrenal cancer, soft tissue sarcoma, urethral cancer, penile cancer, prostate cancer, chronic or acute leukemia, lymphocytic lymphoma, bladder cancer, kidney cancer, urinary tract cancer, renal cell carcinoma, renal pelvic carcinoma, CNS central nervous system (CNS) tumors, primary CNS lymphoma, spinal cord tumors, brainstem gliomas, and pituitary adenomas, but the present invention is not limited thereto.

In the present invention, the tumor antigen-specific CAR or TCR may be a CAR or TCR against a common tumor antigen that is particularly overexpressed in cancer to be treated or expressed specifically only in the corresponding cancer, or a neoantigen expressed by somatic a mutation occurring only in the corresponding cancer. That is, the tumor antigen is any one that is expressed specifically in cancer cells or highly expressed particularly in cancer cells without limitation, and specific types of the tumor antigen may be, but are not limited to, selected from CD19, NY-ESO-1, EGFR, TAG72, interleukin 13 receptor alpha-2 subunit (IL13Rα2), CD52, CD33, CD20, TSLPR, CD22, CD30, GD3, CD171, a neural cell adhesion molecule (NCAM), a folate binding protein (FBP), a Lewis-Y antigen (Le(Y)), a prostate stem cell antigen (PSCA), a prostate-specific membrane antigen (PSMA), a carcinoembryonic antigen (CEA), human epidermal growth factor receptor 2 (HER2), mesothelin, hyaluronate receptor variant 6 (CD44v6), B7-H3, glypican-3, receptor tyrosine kinase like orphan receptor 1 (ROR1), survivin, folate receptor (FOLR1), Wilm's tumor antigen (WT1), vascular endothelial growth factor 2 (VEGFR2), an oncovirus antigen, TP53, KRAS, and a neoantigen. However, those of ordinary skill in the art may select an appropriate tumor antigen known in the art according to the type of cancer to be treated and apply it to the present invention.

The content of the cells in the composition of the present invention can be appropriately adjusted according to the symptoms of a disease, the degree of the progression of symptoms, and a patient's condition, and may be, for example, 0.0001 to 99.9 wt%, or 0.001 to 50 wt% based on the total weight of the composition, but the present invention is not limited thereto. The content ratio is a value based on a dry content from which a solvent is removed.

The pharmaceutical composition according to the present invention may further include appropriate carrier, excipient and diluent, which are generally used in the preparation of the pharmaceutical composition. The excipient may be, for example, one or more selected from the group consisting of a diluent, a binder, a disintegrant, a lubricant, an adsorbent, a humectant, a film-coating material, and a controlled-release additive.

The pharmaceutical composition according to the present invention may be formulated in the form of a powder, a granule, a sustained-release granule, an enteric granule, a liquid, an ophthalmic solution, an elixir, an emulsion, the supernatant, a spirit, a troche, aromatic water, a lemonade, a tablet, a sustained-release tablet, an enteric tablet, a sublingual tablet, a hard capsule, a soft capsule, a sustained-release capsule, an enteric capsule, a pill, a tincture, a soft extract, a dry extract, a fluid extract, an injection, a capsule, a perfusate, a plaster, a lotion, a paste, a spray, an inhalant, a patch, a sterile injection, or an external agent such as an aerosol according to a conventional method, and the external agent may be formulated in a cream, a gel, a patch, a spray, an ointment, a plaster, a lotion, a liniment, a paste, or a cataplasma.

The carrier, excipient and diluent which may be included in the pharmaceutical composition according to the present invention may include lactose, dextrose, sucrose, an oligosaccharide, sorbitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, water, methylhydroxybenzoate, propylhydroxybenzoate, talc, magnesium stearate and mineral oil.

The pharmaceutical composition according to the present invention may be prepared with a diluent or an excipient such as a filler, a thickening agent, a binder, a wetting agent, a disintegrant, and a surfactant, which are commonly used, during formulation.

Additives for a tablet, powder, granule, capsule, pill and troche may include excipients such as corn starch, potato starch, wheat starch, lactose, sucrose, glucose, fructose, di-mannitol, precipitated calcium carbonate, synthetic aluminum silicate, calcium monohydrogen phosphate, calcium sulfate, sodium chloride, sodium bicarbonate, purified lanolin, microcrystalline cellulose, dextrin, sodium alginate, methylcellulose, carboxymethylcellulose, sodium carboxymethylcellulose, kaolin, urea, colloidal silica gel, hydroxypropyl starch, hydroxypropyl methyl cellulose (HPMC) 1928, HPMC 2208, HPMC 2906, HPMC 2910, propylene glycol, casein, calcium lactate and Primoj el; binders such as gelatin, gum arabic, ethanol, agar powder, cellulose acetate phthalate, carboxymethyl cellulose, carboxymethyl cellulose calcium, glucose, purified water, sodium caseinate, glycerin, stearic acid, sodium carboxymethylcellulose, sodium methylcellulose, methylcellulose, microcrystalline cellulose, dextrin, hydroxycellulose, hydroxypropyl starch, hydroxymethylcellulose, purified shellac, starch powder, hydroxypropylcellulose, hydroxypropylmethylcellulose, polyvinyl alcohol and polyvinylpyrrolidone; disintegrants such as hydroxypropylmethylcellulose, corn starch, agar powder, methylcellulose, bentonite, hydroxypropyl starch, sodium carboxymethylcellulose, calcium citrate, sodium lauryl sulfate, silicic anhydride, 1-hydroxypropyl cellulose, dextran, an ion exchange resin, polyvinyl acetate, formaldehyde-treated casein and gelatin, alginic acid, amylose, guar gum, sodium bicarbonate, polyvinylpyrrolidone, calcium phosphate, gelled starch, gum arabic, amylopectin, pectin, sodium polyphosphate, ethyl cellulose, sucrose, magnesium aluminum silicate, a di-sorbitol solution and light anhydrous silicic acid; and lubricants such as calcium stearate, magnesium stearate, stearic acid, hydrogenated vegetable oil, talc, lycopodium powder, kaolin, petrolatum, sodium stearate, cacao butter, sodium salicylate, magnesium salicylate, polyethylene glycol (PEG) 4000, PEG 6000, liquid paraffin, hydrogenated soybean oil (Lubri wax), aluminum stearate, zinc stearate, sodium lauryl sulfate, magnesium oxide, Macrogol, synthetic aluminum silicate, silicic anhydride, a higher fatty acid, a higher alcohol, silicone oil, paraffin oil, polyethylene glycol fatty acid ether, starch, sodium chloride, sodium acetate, sodium oleate, dileucine and light anhydrous silicic acid.

Additives for a liquid according to the present invention may include water, diluted hydrochloric acid, diluted sulfuric acid, sodium citrate, monostearate sucrose, polyoxyethylene sorbitol fatty acid esters (Tween esters), polyoxyethylene monoalkylethers, lanolin ethers, lanolin esters, acetic acid, hydrochloric acid, acetic acid, hydrochloric acid, aqueous ammonia, ammonium carbonate, potassium hydroxide, sodium hydroxide, prolamine, polyvinylpyrrolidone, ethyl cellulose, and sodium carboxymethylcellulose.

A solution of white sugar, other sugars, or a sweetener may be used in a syrup according to the present invention, and if needed, a fragrance, a colorant, a preservative, a stabilizer, a suspending agent, an emulsifier, or a thickener may be used.

Purified water may be used in an emulsion according to the present invention and if needed, an emulsifier, a preservative, a stabilizer, or a fragrance may be used.

Acacia, tragacanth, methyl cellulose, carboxymethylcellulose, sodium carboxymethylcellulose, microcrystalline cellulose, sodium alginate, hydroxypropyl methylcellulose (HPMC), HPMC 1828, HPMC 2906, or HPMC 2910 may be used in a suspension according to the present invention, and if needed, a surfactant, a preservative, a stabilizer, a coloring agent, or a fragrance may be used.

For an injection according to the present invention, a solvent such as injectable sterile water, 0.9% sodium chloride for injection, Ringer's solution, dextrose for injection, dextrose+sodium chloride for injection, PEG, lactated Ringer's solution, ethanol, propylene glycol, non-volatile oil-sesame oil, cottonseed oil, peanut oil, soybean oil, corn oil, ethyl oleate, isopropyl myristic acid or benzene benzoate; a solubilizing agent such as sodium benzoate, sodium salicylate, sodium acetate, urea, urethane, monoethylacetamine, butazolidine, propylene glycol, Tween, nicotinamide, hexamine or dimethylacetamide; a buffer such as a weak acid and a salt thereof (acetic acid and sodium acetate), a weak base and a salt thereof (ammonia and ammonium acetate), an organic compound, a protein, albumin, peptone, or gums; an isotonic agent such as sodium chloride; a stabilizer such as sodium bisulfite (NaHSO₃), carbon dioxide gas, sodium metabisulfite (Na₂S₂O₃), sodium sulfite (Na₂SO₃), nitrogen gas (N₂) or ethylenediaminetetracetic acid; an antioxidant such as sodium bisulfide 0.1%, sodium formaldehyde sulfoxylate, thiourea, disodium ethylenediaminetetraacetate or acetone sodium bisulfite; a pain-relief agent such as benzyl alcohol, chlorobutanol, procaine hydrochloride, glucose or calcium gluconate; or a suspending agent such as sodium CMC, sodium alginate, Tween 80 or aluminum monostearate, may be used.

For a suppository according to the present invention, a base such as cacao butter, lanolin, Witepsol, polyethylene glycol, glycerogelatin, methyl cellulose, carboxymethylcellulose, a mixture of stearate and oleate, Subanal, cottonseed oil, peanut oil, palm oil, cacao butter + cholesterol, lecithin, Lanette wax, glycerol monostearate, Tween or Span, Imhausen, monolene (propylene glycol monostearate), glycerin, Adeps solidus, Buytyrum Tego-G, Cebes Pharma 16, hexalide base 95, Cotomar, Hydrokote SP, S-70-XXA, S-70-XX75 (S-70-XX95), Hydrokote 25, Hydrokote 711, Idropostal, Massa estrarium, A, AS, B, C, D, E, I, T), Mass-MF, Masupol, Masupol-15, neosuppostal-N, paramount-B, supposiro (OSI, OSIX, A, B, C, D, H, L), suppository base IV types (AB, B, A, BC, BBG, E, BGF, C, D, 299), Suppostal (N, Es), Wecoby (W, R, S, M ,Fs), or a Tegester triglyeride base (TG-95, MA, 57) may be used.

A solid formulation for oral administration may be a tablet, a pill, a powder, a granule or a capsule, and such a solid formulation may be produced by mixing at least one of excipients, such as starch, calcium carbonate, sucrose, lactose, or gelatin, with the active ingredient. Also, in addition to the simple excipient, lubricants such as magnesium stearate and talc may also be used.

As a liquid formulation for oral administration, a suspension, a liquid for internal use, an emulsion, or a syrup may be used, and a generally-used simple diluent such as water or liquid paraffin, as well as various types of excipients, for example, a wetting agent, a sweetener, a fragrance and a preservative may be included. A formulation for parenteral administration may be a sterilized aqueous solution, a non-aqueous solvent, the supernatant, an emulsion, a lyophilizing agent or a suppository. As the non-aqueous solvent or suspension, propylene glycol, polyethylene glycol, a vegetable oil such as olive oil, or an injectable ester such as ethyl oleate may be used.

The pharmaceutical composition according to the present invention may be administered at a pharmaceutically effective amount. The term "pharmaceutically effective amount" used herein refers to an amount sufficient for treating a disease at a reasonable benefit/risk ratio applicable for medical treatment, and an effective dosage may be determined by parameters including the type of a patient's disease, severity, drug activity, sensitivity to a drug, administration time, an administration route and an excretion rate, the duration of treatment and concurrently used drugs, and other parameters well known in the medical field.

The pharmaceutical composition according to the present invention may be administered separately or in combination with other therapeutic agents, and may be sequentially or simultaneously administered with a conventional therapeutic agent, or administered in a single or multiple dose(s). In consideration of all of the above-mentioned parameters, it is important to achieve the maximum effect with the minimum dose without a side effect, and such a dose may be easily determined by one of ordinary skill in the art.

The pharmaceutical composition of the present invention may be administered to a subject via various routes. All administration routes may be expected, and the pharmaceutical composition of the present invention may be administered by, for example, oral administration, subcutaneous injection, intraperitoneal administration, intravenous, intramuscular or intrathecal injection, sublingual administration, buccal administration, rectal insertion, vaginal insertion, ocular administration, ear administration, nasal administration, inhalation, spraying through the mouth or nose, skin administration, or transdermal administration.

The pharmaceutical composition of the present invention is determined by the type of drug, which is an active ingredient, along with various related factors such as a disease to be treated, an administration route, a patient's age, sex, and body weight, and the severity of a disease. Specifically, the effective amount of the composition according to the present invention may vary according to a patient's age, sex or body weight, and generally, 0.001 to 150 mg, and preferably 0.01 to 100 mg per kg of body weight may be administered daily or every other day, or one to three times a day. However, the effective amount may be increased or decreased depending on the route of administration, the severity of obesity, gender, a body weight or age, and thus it does not limit the scope of the present invention in any way.

In the present invention, "individual" refers to a subject in need of treatment of a disease, and more particularly, a mammal such as a human or a non-human primate, a mouse, a rat, a dog, a cat, a horse, or a cow.

In the present invention, "administration" refers to providing a predetermined composition of the present invention to an individual by any appropriate method.

In the present invention, "prevention" refers to all actions that inhibit or delay the occurrence of a target disease, "treatment" refers to all actions involved in alleviating or beneficially changing symptoms of a target disease and related metabolic disorders by the administration of the pharmaceutical composition according to the present invention, and "improvement" refers to all actions of reducing parameters related to a target disease, for example, the severity of symptoms, by the administration of the composition according to the present invention.

In addition, the present invention provides a kit for preventing or treating cancer, which includes a transposon system including a) a transposon vector into which a target DNA is inserted; and
b) a nucleic acid molecule including a sequence encoding a transposase protein or transposase,
wherein the target DNA is one or more selected from the group consisting of a tumor antigen-specific chimeric antigen receptor (CAR)-coding sequence or a fragment thereof, an oncovirus-specific neutralizing antibody-coding sequence or a fragment thereof, an immune checkpoint inhibitor-coding sequence, and a tumor antigen-specific T-cell receptor (TCR)-coding sequence or a fragment thereof.

The kit may further include immune cells for expressing the target DNA.

In addition, the kit may further include instructions containing a detailed description of the transposon vector of the present invention or the vector-introduced cells (characteristics, preparation method, storage method, administration method, etc.).

Hereinafter, preferred examples are presented to better understand the present invention. However, the following examples are provided to more easily understand the present invention, and the content of the present invention is not limited by the following examples.

### [Experimental methods]

### Example A. ITR detection and identification of its function

### 1. Bioinformatics

Since a transposable element (TE or transposon) has large interspecies variation, a repeat family is explored and modeled de novo using RepeatModeler software version 2.0.1 in *Myotis lucifugus 7x* assembly (myoLuc2), thereby producing a bat species-specific repeat library. After combining the repeat library obtained above and the mammalian library data in RepeatMasker, transposable elements were masked and the sequences of the masked DNA transposons were obtained using RepeatMasker software version 4.1.1 with the rmblast 2.10.0+ search engine.

The sequences of the obtained 5' ITR and 3' ITR are as follows.
a. 5' ITR_157 (SEQ ID NO: 1) 
5'-
ttaacacttggattgcgggaaacgagttaagtcggctcgcgtgaattgcgcgtactccgcgggagccgtcttaactcggttcata
tagatttgcggtggagtgcgggaaacgtgtaaactcgggccgattgtaactgcgtattaccaaatatttgtt-3'
b. 3' ITR 212 (SEQ ID NO: 2)

5'-
aattatttatgtactgaatagataaaaaaatgtctgtgattgaataaattttcattttttacacaagaaaccgaaaatttcatttcaatcg
aacccatacttcaaaagatataggcattttaaactaactctgattttgcgcgggaaacctaaataattgcccgcgccatcttatatttt
ggcgggaaattcacccgacaccgtagtgttaa-3'

### 2. pBat transposon vector

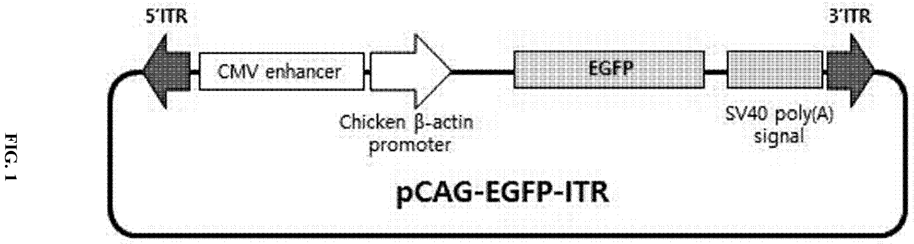

Each of the 5' ITR and 3' ITR of the obtained DNA transposon was cloned in a pCAG-EGFP vector through Bioinformatics, wherein the 5' ITR was designed to be located in front of the chicken beta-actin promoter and the 3' ITR was designed to be located after the SV40 poly(A) signal in the vector (FIG. 1).

### 3. Gene delivery (transfection)

After washing a T cell line (Jurkat cells) with PBS, the cells were resuspended in 90 µL of resuspension buffer per 1×10⁵ cells. 3 mL of an electrolytic buffer was added to a Neon tube, and then put into a Neon pipette station. One µg per 1 × 10⁵ cells each of pCAG-EGFP-ITR plasmid DNA and transposase plasmid DNA (here, a transposase has the nucleotide sequence of SEQ ID NO: 19) was added to the cells, and a total volume was adjusted to 100 µL per 1×10⁵ cells. As a control, pEGFP plasmid DNA not containing an ITR was used. 1×10⁵ of the cells were taken with a Neon pipette for electroporation under conditions of 1,600 V, 10 ms, and 3 pulses, and the cells were added to each well containing 400 µL of medium (RPMI, 10% FBS, No P/S) in a 24-well plate. The cells were incubated at 37 °C in a CO₂ incubator for 1 day, and 500 µL of an antibiotic-containing medium was added to each well. One day, 7 days, and 14 days after transfection, the GFP expression level in the cells was confirmed using a fluorescence microscope and FACS.

Transposase nucleic acid sequence (SEQ ID NO: 19)

atgtcgcaacactcagattactccgacgatgaattttgtgctgacaaactgtccaattattcatgcgatagcgacctcgaaaacg
cttccacgtctgatgaagatagcagcgatgatgaagtaatggtgaggcctcgcaccctccgccgtcgccgcatcagctcttcgagctctg
attctgaatccgatattgagggtggccgcgaggagtggtcccacgtagacaatccgccggtgctggaggacttcctaggccaccaagg
tctgaacactgacgcagtaatcaacaatatcgaagatgcagttaaactgtttatcggtgacgatttcttcgagtttctggtggaggaatctaa
ccggtactataaccagaatcgtaataacttcaagctctctaaaaagtctctgaagtggaaggacatcacccctcaggagatgaaaaagttc
ctcggtctgatcgttctgatgggccaagttcgcaaggatcgtcgtgacgactattggactaccgaaccgtggacggaaactccatactttg
gcaagaccatgactcgtgaccgtttccgtcagatctggaaggcctggcacttcaataacaacgctgacattgtcaacgagtctgatcgtct
gtgtaaggttcgccctgtgctggattacttcgttccaaaattcattaacatttacaaaccacatcagcagctgtccctggatgagggcatcgt
gccgtggcgcggccgcctgttcttccgtgtctataatgctggcaagattgtgaagtacggtatcctggttcgcctgctgtgcgaaagcgac
actggctacatctgtaacatggagatctactgcggcgagggcaaacgtctcctcgaaactatccagaccgtcgtgtctccatacacggat
tcctggtatcatatttacatggataactattataacagcgtggctaactgtgaagctctgatgaaaaataagttccgtatttgcggtactatcc
gtaagaatcgtggaattccgaaagatttccagaccatctccctgaaaaagggtgaaactaagttcattcgcaaaaacgacatcctcctgca
agtctggcagtctaaaaagcctgtatatctgatctcatctattcacagcgctgaaatggaagaatctcagaacattgatcgcacctccaaga
aaaagatcgtcaaaccgaatgcattgattgattacaacaagcacatgaagggcgttgatcgtgctgaccagtacctgtcttattactctatc
ctgcgccgtactgtgaagtggactaaacgtctcgctatgtacatgattaattgtgcgctgttcaattcttacgctgtgtataaaagcgtgcgtc
agcgcaaaatgggctttaaaatgttcctgaagcagacggctattcactggctgaccgacgatattccggaagatatggacattgtcccgg
atctccagccggtaccgagcaccagcggtatgcgtgctaaacctccgactagtgatccgccttgccgtctgtctatggatatgcgtaagc
ataccctgcaggcaattgtgggctctggcaaaaagaaaaatatcctgcgtcgttgccgcgtatgctctgtacacaaactgcgttctgagac
tcgttatatgtgtaaattttgcaatattccactccacaagggtgcgtgcttcgagaagtaccatacgctgaagaactat

Transposase mRNA sequence (SEQ ID NO: 20)

augucgcaacacucagauuacuccgacgaugaauuuugugcugacaaacuguccaauuauucaugcgau
agcgaccucgaaaacgcuuccacgucugaugaagauagcagcgaugaugaaguaauggugaggccucgcacccucc
gccgucgccgcaucagcucuucgagcucugauucugaauccgauauugaggguggccgcgaggaguggucccacg
uagacaauccgccggugcuggaggacuuccuaggccaccaaggucugaacacugacgcaguaaucaacaauaucga
agaugcaguuaaacuguuuaucggugacgauuucuucgaguuucugguggaggaaucuaaccgguacuauaacca
gaaucguaauaacuucaagcucucuaaaaagucucugaaguggaaggacaucaccccucaggagaugaaaaaguuc
cucggucugaucguucugaugggccaaguucgcaaggaucgucgugacgacuauuggacuaccgaaccguggacg
gaaacuccauacuuuggcaagaccaugacucgugaccguuuccgucagaucuggaaggccuggcacuucaauaaca
acgcugacauugucaacgagucugaucgucuguguaagguucgcccugugcuggauuacuucguuccaaaauuca
uuaacauuuacaaaccacaucagcagcugucccuggaugagggcaucgugccguggcgcggccgccuguucuucc
gugucuauaaugcuggcaagauugugaaguacgguauccugguucgccugcugugcgaaagcgacacuggcuaca
ucuguaacauggagaucuacugcggcgagggcaaacgucuccucgaaacuauccagaccgucguguccccauacac
ggauuccugguaucauauuuacauggauaacuauuauaacagcguggcuaacugugaagcucugaugaaaaauaa
guuccguauuugcgguacuauccguaagaaucguggaauuccgaaagauuuccagaccaucucccugaaaaaggg
ugaaacuaaguucauucgcaaaaacgacauccuccugcaagucuggcagucuaaaaagccuguauaucugaucuca
ucuauucacagcgcugaaauggaagaaucucagaacauugaucgcaccuccaagaaaaagaucgucaaaccgaaug
cauugauugauuacaacaagcacaugaagggcguugaucgugcugaccaguaccugucuuauuacucuauccugc
gccguacugugaaguggacuaaacgucucgcuauguacaugauuaauugugcgcuguucaauucuuacgcugug
uauaaaagcgugcgucagcgcaaaaugggcuuuaaaauguuccugaagcagacggcuauucacuggcugaccgac
gauauuccggaagauauggacauugucccggaucuccagccgguaccgagcaccagcgguaugcgugcuaaaccu
ccgacuagugauccgccuugccgucugucuauggauaugcguaagcauacccugcaggcaauugugggcucuggc
aaaaagaaaaauauccugcgucguugccgcguaugcucuguacacaaacugcguucugagacucguuauaugugu
aaauuuugcaauauuccacuccacaagggugcgugcuucgagaaguaccauacgcugaagaacuau

### 4. FACS analysis

The transfected cells of each well were transferred to a 1.5 mL tube. After centrifugation at 1,500 rpm for 5 minutes, a supernatant was removed and washed with 500 µL PBS (2% FBS). After repeating washing twice, the cells were suspended in PBS (2% FBS, 1x DAPI) and transferred to a FACS tube for FACS analysis. The proportion (%) of GFP-expressing cells among live cells (DAPI negative) was compared by group.

### 5. Fluorescence microscopy

The GFP-expressing cells were observed at 100x magnification using a Carl Zeiss fluorescence microscope.

### 6. Single cell sorting

Seven days after transfection, the GFP-expressing cells were sorted into single cells using FACSAria (Aria) equipment (BD), a 10% FBS-containing RPMI medium was added to a 96-well plate, and the cells were incubated in a 37 °C, 5% CO₂ incubator. In addition, 10 days later, whether GFP was expressed in the cells was observed using a fluorescence microscope, and to detect an integration site, splinkerette PCR was performed.

### 7. Confirmation of DNA integration site in chromosome using splinkerette PCR

The cells that had been sorted into single cells and incubated were collected, and washed with PBS. gDNA was extracted from a cell pellet and quantified using a genomic DNA (gDNA) kit. 100 ng of gDNA was digested at 37 °C for 1 hour using a Sau3AI restriction enzyme. A Sau3AI adaptor was produced by annealing (left at 95 °C for 3 minutes and then reducing the temperature to room temperature) two single-stranded DNAs (GATCCCACTAGTGTCGACACCAGTCTCTAATTTTTTTTTTCAAAAAAA (SEQ ID NO: 21), and CGAAGAGTAACCGTTGCTAGGAGAGACCGTGGCTGAATGAGACTGGTGTCG ACACTAGTGG (SEQ ID NO: 22). 20 ng of gDNA digested with Sau3AI was allowed to react with the annealed Sau3AI adaptor at 16 °C for 8 hours, and then 1^{st} PCR (98 °C for 20 sec, 55 °C for 15 sec, 72 °C for 2 min, 29 cycles) and 2^{nd} PCR (98 °C for 20 sec, 55 °C for 15 sec, 72 °C for 2 min, 29 cycles) were performed. The PCR results were confirmed by electrophoresis, and then sequencing was performed.
1^{st} PCR primer: 
5' 1st F - CGAAGAGTAACCGTTGCTAGGAGAGACC (SEQ ID NO: 23), 5' 1st
R - CCCTATAGTGAGTCGTATTACCAA (SEQ ID NO: 24), 3' 1st F -
CATTACCCTGTTATCCCTAGCTAGCA (SEQ ID NO: 25), 3' 1st R -
CGAAGAGTAACCGTTGCTAGGAGAGACC (SEQ ID NO: 26).
2^{nd} PCR primer:

5' 2nd F - GTGGCTGAATGAGACTGGTGTCGAC (SEQ ID NO: 27), 5' 2nd R
- GGTCATAGCTGTTTCCTGCT (SEQ ID NO: 28), 3' 2nd F -
CATTTCAATCGAACCCATACTTCAAAA (SEQ ID NO: 29), 3' 2nd R -
GTGGCTGAATGAGACTGGTGTCGAC (SEQ ID NO: 30).

### Example B. Production of ITR mutant and identification of its function

### 1. Cells and vector

As to be described below, a pBat transposon mutant-type plasmid was produced and used in an experiment, and as cells for confirming gene delivery efficiency, Jukat cells (ATCC, Cat no. TIB-152, Lot no. 70017560) were used.

### 2. Production of ITR mutant form

To produce an ITR mutant, a backbone transposon plasmid vector (plasmid vector) was produced by inserting a BamHI site in front of the 5' ITR of the original transposon plasmid vector and inserting a SalI site after the 3' ITR.

A 5' ITR mutant plasmid vector was produced by cleaving the backbone transposon plasmid vector with BamHI and EcoRV restriction enzymes and inserting a 5' ITR mutant.

A 3' ITR mutant plasmid vector was produced by cleaving the backbone transposon plasmid vector with BmtI and Sall restriction enzymes and inserting a 3' ITR mutant.

### 3. Cloning

To produce 5' ITR and 3' ITR mutants, a BamHI site was added in front of a 5' ITR in a pCAG-GFP-ITR vector, and a SalI site was added after a 3' ITR. In the case of the 5' ITR, BamHI and EcoRV were added to a transposon vector and a pUC57-5' ITR mutant vector, and allowed to react at 37 °C for 2 hours and at 50 °C for 2 hours for digestion, respectively. In the case of the 3' ITR, BmtI and SalI were added to a transposon vector and a pUC57-3' ITR mutant vector, and allowed to react at 37 °C for 2 hours for digestion. After digestion and then gel extraction, ligation was performed using T4 DNA ligase, the ligated result was transformed into DH5α, and spread on a LB plate (containing ampicillin). Colonies formed thereby were grown in LB broth (containing ampicillin), thereby obtaining a plasmid using mini-prep, and sequencing was performed. The sequenced mutant vectors were subjected to mini-prep, thereby producing DNA to be transfected.

### 4. Neon Transfection (Electroporation)

After washing a T cell line (Jurkat cells) with PBS, the cells were resuspended in 90 µL of resuspension buffer per 1×10⁵ cells. 3 mL electrolytic buffer was added to a Neon tube and then put into a Neon pipette station. One µg per 1×10⁵ cells each of mutant DNA and transposase DNA was added, and a total volume was adjusted to 100 µL per 1×10⁵ cells. 1×10⁵ of the cells were taken with a Neon pipette for electroporation under conditions of 1,600 V, 10 ms, and 3 pulses, and the cells were added to each well containing 400 µL of medium (RPMI, 10% FBS, No P/S) in a 24-well plate. The cells were incubated at 37 °C in a COz incubator for 1 day, and 500 µL of an antibiotic-containing medium was added to each well. One day, 7 days, and 14 days after transfection, the GFP expression level in the cells was confirmed using a fluorescence microscope and FACS.

### 5. FACS

The transfected cells of each well were transferred to a 1.5 mL tube. After centrifugation at 1,500 rpm for 5 minutes, a supernatant was removed and washed with 500 µL of PBS (2% FBS). After repeating washing twice, the cells were suspended in PBS (2% FBS, 1x DAPI) and transferred to a FACS tube for FACS analysis. The proportion (%) of GFP-expressing cells among live cells (DAPI negative) was compared by group.

### 6. Fluorescence microscopy

GFP-expressing cells were observed at 100x magnification using a Carl Zeiss fluorescence microscope.

### 7. Single cell sorting and culture

Fourteen days after transfection, the GFP-expressing Jurkat cells were sorted into single cells. The cells of each well were transferred to a conical tube and centrifuged at 1,500 rpm for 5 minutes to remove a supernatant, and then the cell pellet was suspended in washing buffer (PBS + 2% FBS) for washing, and then washed again with washing buffer twice. Subsequently, the cells were suspended by adding 200 µL of 1X DAPI-containing washing buffer to each well, and then transferred to a FACS filter tube. In addition, 1mL of 1X DAPI-containing washing buffer was added. 100 µL of complete medium was added to each well of a 96-well plate, and single cell sorting was performed for each cell per well. The plate subjected to single cell sorting was incubated at 37 °C in a CO₂ incubator. After 3 days, 100 µL of complete medium was added to each well, and the medium was replaced with 100 µL of complete medium per well every 2 to 3 days during cell culture. When the cells were grown in the 96 well plate, the cells were transferred to a 24-well plate, and a volume for each well was adjusted with a complete medium to 500 µL. After 2 to 3 days, 500 µL of complete medium was added to each well of the 24-well plate, and after 2 to 3 days, 1 mL of complete medium was added to each well to make a total volume of 2 mL. The cells were cultured while the medium is replaced with 1 mL of complete medium every 2 to 3 days.

### Example C. Production of ITR mutant (reverse) and identification of its function

### 1. Cells and vector

As to be described below, a pBat transposon mutant-type plasmid was produced and used in an experiment, and as cells for confirming gene delivery efficiency, Jukat cells (ATCC, Cat no. TIB-152, Lot no. 70017560) were used.

### 2. Production of ITR mutant forms

TTR mutants were produced in the same manner as in Example B.

### 3. Transfection, FACS, fluorescence microscopy, and single cell sorting

Mutant DNA and transposase DNA were transfected into cells in the same manner as in Example B (electroporation). Seven days and 14 days after transfection, the degree of GFP expression of the cells was observed using a fluorescence microscope, and the proportion of GFP-expressing cells among live cells (DAPI negative) was confirmed by FACS analysis. In addition, single cell sorting and culture were performed in the same manner as in Example B.

Transfection groups were prepared as in Table 1 below.

**[Table 1]**

| No. | Name | Plasmid (1µg each) |
|---|---|---|
| 1 | Control | X |
| 2 | pBat transposon only | pBat B3IS-B5IE |
| 3 | pBat control | pBat original + transposase |
| 4 | pEGFP | pEGFP |
| 5 | B3IS-B5IE | pBat B3IS-B5IE + transposase |
| 6 | B3IS-5M2 | pBat B3IS-5M2 + transposase |
| 7 | B3IS-5M3 | pBat B3IS-5M3 + transposase |
| 8 | B3IS-5M4 | pBat B3IS-5M4 + transposase |
| 9 | 3M1-B5IE | pBat 3M1-B5IE + transposase |
| 10 | 3M1-5M2 | pBat 3M1-5M2 + transposase |
| 11 | 3M1-5M3 | pBat 3M1-5M3 + transposase |
| 12 | 3M1-5M4 | pBat 3M1-5M4 + transposase |
| 13 | 3M2-B5IE | pBat 3M2-B5IE + transposase |
| 14 | 3M2-5M2 | pBat 3M2-5M2 + transposase |
| 15 | 3M2-5M3 | pBat 3M2-5M3 + transposase |
| 16 | 3M2-5M4 | pBat 3M2-5M4 + transposase |
| 17 | 3M3-B5IE | pBat 3M3-B5IE + transposase |
| 18 | 3M3-5M2 | pBat 3M3-5M2 + transposase |
| 19 | 3M3-5M3 | pBat 3M3-5M3 + transposase |
| 20 | 3M3-5M4 | pBat 3M3-5M4 + transposase |
| 21 | 3M4-B5IE | pBat 3M4-B5IE + transposase |
| 22 | 3M4-5M2 | pBat 3M4-5M2 + transposase |
| 23 | 3M4-5M3 | pBat 3M4-5M3 + transposase |
| 24 | 3M4-5M4 | pBat 3M4-5M4 + transposase |

### Example D. Confirmation of gene delivery efficiency of pBat transposon to PBMCs

In the previous examples, to confirm the gene delivery efficiency by the pBat transposon system in the Jurkat cell line, electroporation was performed using Neon equipment. However, electroporation using the Neon equipment has the disadvantages of very low efficiency in primary T cells and having to be conducted only with a small scale of cells (maximum 10⁶). Accordingly, in this example, the gene delivery efficiency by the pBat transposon system in PBMCs was confirmed using Maxcyte equipment. To this end, the gene delivery efficiencies of mutant-type transposons were compared using GFP gene-containing transposons (Naive-GFP, 3M3-5M3-GFP) and 1G4 TCR gene-containing transposons (B3IS-B5IE-1G4, 3M3-5M3-1G4). A transposase was expressed from plasmid DNA.

### 1. Test materials

### 1-1. DNA and RNA molecules

The following four types of pBat transposon plasmid vectors were used, and as a control, pEGFP (pBat B3IS-B5IE) was used. For transposase expression, a pBat transposase plasmid was also introduced (SEQ ID NO: 19).

**[Table 2]**

| **No.** | **pBat-GFP** | **No.** | **pBat-1G4 TCR** |
|---|---|---|---|
| 1 | Naive-GFP | 3 | B3IS-B5IE-1G4 |
| 2 | 3M3-5M3-GFP | 4 | 3M3-5M3-1G4 |
| * Naive represents a wild-type transposon gene present in nature. | | | |
| * B3IS-B5IE is a construct in which enzyme sites are added to the gene outside the 5' ITR and 3' ITR of the wild-type transposon. | | | |

### 1-2. Cells

As target cells for confirming the gene delivery efficiency of a transposon, fresh PBMCs and A375 cells (ATCC, Cat no. CRL-1619, Lot no. 70032966) obtained by collecting blood from humans were used.

### 2. Isolation of PMBCs from blood

PBMCs were obtained from blood acquired by blood collection from humans according to the following methods: 15 mL of Ficoll-Paque was dispensed into each of four 50 mL conical tubes, 30 mL of 80 mL whole blood obtained from a healthy person was slowly added over the Ficoll-Paque layer of each tube without being mixed. Subsequently, centrifugation was performed under conditions of 1000xg and Break 0 for 15 minutes. From the tube that had been centrifuged, only the PBMC layer was carefully taken out using a pipette and then transferred to a new 50 mL conical tube. After filling the tube with washing buffer (DPBS + 2% FBS) to have a total volume of 50 mL and then inverting the tube, centrifugation was performed at 450xg for 10 minutes, and the supernatant was removed. Subsequently, the pellet was resuspended by adding 50 mL of washing buffer, and centrifuged at 450xg for 10 minutes to remove a supernatant. The pellet was resuspended by adding 50 mL of washing buffer, and then the number and viability of PBMCs were confirmed. Subsequently, centrifugation was performed again at 450xg for 10 minutes to remove a supernatant, and the obtained cell pellet (PBMCs) was resuspended in 20 mL of RPMI media (a total of 8×10⁷ cells) and transferred to a T75 flask. The cells were stored at room temperature until further experiments.

### 3. Electroporation

The gene delivery to cells was accomplished by electroporation. First, after collecting PBMCs isolated from human blood in a conical tube, the cells were centrifuged at 1,500 rpm for 5 minutes, a supernatant was removed, and then the cells were suspended in 50 mL of PBS. Cell counting was performed on the suspended cells, and the cells were centrifuged at 1,500 rpm for 5 minutes to remove a supernatant. By adding Opti-MEM buffer to the obtained cell pellet, the cells were suspended to a concentration of 4×10⁶ cells/50 µL. Subsequently, a 1.5 mL tube was prepared for each electroporation condition (Table 3). 5 µg of the transposon vector per 4×10⁶ PBMCs was added to each tube, and 4×10⁶ PBMCs were added to each tube containing the transposon vector. Subsequently, the cell suspension (4×10⁶ cells/50 µL) was carefully added to an OC100X2 assembly without generating bubbles. For electroporation, the Resting T cell 14-3 protocol was performed in Maxcyte GTx. The OC100x2 assembly was put in GTx to perform the protocol, followed by electroporation. After electroporation, a cell suspension was transferred from the OC100x2 assembly to a 12-well plate (4×10⁶ cells/50 µL/well). An OC100X2 well was washed with 50 µL of Opti-MEM medium, and then added to each well of the plate, followed by a recovery time of 20 minutes in a CO₂ incubator at 37 °C. After the recovery time, 800 µL of complete medium (AIM-V + 3% HS + 200 IU/mL IL-2) was carefully added to each well of a 6-well plate, and then the plate was put back into the CO₂ incubator at 37 °C to incubate for one day.

**[Table 3]**

| **No.** | **Group** | **Plasmid (5 µg each)** |
|---|---|---|
| 1 | Control | X |
| 2 | pEGFP | EGFP |
| 3 | Naive-GFP + DNA | pBat naive-GFP + transposase plasmid |
| 4 | 3ME-SM3-GFP + DNA | pBat 3M3-5M3-GFP + transposase plasmid |
| 5 | B3IS-B5IE-1G4 + DNA | pBat B3IS-B5IE-1G4 + transposase plasmid |
| 6 | 3M3-5M3-1G4 + DNA | pBat 3M3-5M3-1G4 + transposase plasmid |

### 4. Addition of feeder cells

One day after electroporation, as 50Gy-irradiated feeder cells, A375 was added to the electroporated PBMCs. Specifically, after removing the medium from the A375 (p8) cells incubated in eight 150π dishes, the dishes were washed with 5 mL of PBS, and then 2 mL of 0.05% trypsin-EDTA was added. After incubation at 37 °C in a CO₂ incubator for 3 minutes, 10 mL of fresh medium (DMEM + 10% FBS, + 1× P/S) was added and then the cells detached from the dish bottom were collected. Subsequently, the cell suspension was centrifuged at 1,500 rpm for 5 minutes, a supernatant was removed, and the cell pellet was suspended in 30 mL of fresh medium (DMEM + 10% FBS + 1x P/S). The obtained A375 cells were added to one T75 flask at a concentration of 9×10⁷ cells/20 mL, and irradiated with 50Gy. The irradiated A375 cells were collected in a fresh 50 mL conical tube, and centrifuged at 1,500 rpm for 5 minutes to remove a supernatant. The cell pellet was suspended in 50 mL of PBS and further centrifuged at 1,500 rpm for 5 minutes, and then a supernatant was removed and then the cell pellet was resuspended in 50 mL of PBS, followed by cell counting. The cell sample that completed cell counting was centrifuged at 1,500 rpm for 5 minutes, a supernatant was removed, and then the cell pellet was suspended in a medium (AIM-V + 3% HS + 1x P/S + 200 IU/mL IL-2) to have 2×10⁶ cells/100 µL. One day after electroporation, 100 µL (2×10⁶ cells) of the produced feeder cell suspension was added to the cultured PBMCs, and the cells were incubated at 37 °C in a CO₂ incubator.

### 5. Cell culture

Three days after electroporation, 1 mL of medium (AIM-V + 3% HS + 1x P/S + 200 IU/mL IL-2) was added, and the cells were incubated at 37 °C in a CO₂ incubator (primary medium addition). Six days after electroporation, 2 mL of medium (AIM-V + 3% HS + 1× P/S + 200 IU/mL IL-2) was added, and then incubated at 37 °C in a CO₂ incubator (secondary medium addition). Seven days after electroporation, the cultured cells were suspended, 2.5 mL of the cell suspension was transferred to a new tube for FACS analysis, 1.5 mL of fresh medium (AIM-V + 3% HS + 1× P/S + 200 IU/mL IL-2) was added to the remaining1.5 mL of the cell suspension and then incubated at 37 °C in a CO₂ incubator. Ten days after electroporation, the cultured cells were suspended, 1.5 mL of the cell suspension in each well was transferred to a new well (1:1 split), and 1.5 mL of medium (AIM-V + 3% HS + 1× P/S + 200 IU/mL IL-2) was added to each well, followed by incubating at 37 °C in a CO₂ incubator. Fourteen days after electroporation, the cells of each well were transferred to a new tube for FACS analysis.

### 6. Observation of GFP expression using fluorescence microscope

In a GFP gene-containing pBat transposon vector group, 1 and 7 days after electroporation, GFP expression was observed using a fluorescence microscope.

### 7. FACS analysis

FACS analysis was performed 7 and 14 days after electroporation. Seven days after electroporation, GFP expression was observed using a fluorescence microscope, and then FACS analysis was performed. Specifically, cells of each well were suspended, and then transferred to sixteen 1.5 mL tubes. Each tube was centrifuged at 1,500 rpm for 5 minutes, a supernatant was removed, and then the cells were suspended in 1 mL of washing buffer (PBS + 2% FBS). Centrifugation and supernatant removal were further performed twice to wash the cells. After adding 1 µL of human TruStain FcX + 30 µL of washing buffer to each tube, a reaction was conducted at room temperature for 5 minutes. As shown in Table 4, 1 µL of antibodies were added to each tube and allowed to react at 4 °C for 30 minutes. After the reaction, the cells were washed with washing buffer, suspended in 200 µL of 1× DAPI-containing washing buffer, and then transferred to a FACS tube, followed by FACS analysis.

**[Table 4]**

| **No.** | **Sample** | **mTCRβ PE** | **CD8 Percp-Cy5.5** | **CD3 APC-Cy7** | **DAPI Pacific blue** |
|---|---|---|---|---|---|
| **1** | No stain | - | - | - | - |
| **2** | Isotype | IgG | IgG | IgG | IgG |
| **3** | Singe DAPI | - | - | - | O |
| **4** | Single CD3 | - | - | O | - |
| **5** | Single CD8 | - | O | - | - |
| **6** | Single GFP | - | - | - | - |
| **7** | Control | O | O | O | O |
| **8** | pEGFP | O | O | O | O |
| **9** | Naive-GFP + DNA | O | O | O | O |
| **10** | 3ME-SM3-GFP + DNA | O | O | O | O |
| **11** | B3IS-BSIE-1G4 + DNA | O | O | O | O |
| **12** | 3M3-5M3-1G4 + DNA | O | O | O | O |

### Example E. Confirmation of gene delivery efficiency according to activation period of T cells in TCR-T production using pBat transposon system

### 1. Test materials and cells

As pBat transposon plasmids, GFP gene-containing pBat transposon 3M3-5M3-GFP and 1G4 TCR gene-containing pBat transposon 3M3-5M3-1G4 TCR were used, and the pBat transposase plasmids were also introduced for transposase expression.

As target cells for confirming the gene delivery efficiency of a transposon, fresh PBMCs and A375 cells (ATCC, Cat no. CRL-1619, Lot no. 70032966) obtained by blood collection from humans were used. The A375 cells used herein had been irradiated with radiation (50Gy) and then cryopreserved. Fresh PBMCs were isolated from human whole blood in the same manner as in Example D.

### 2. Electroporation

To introduce the transposon and the transposase plasmid into the PBMCs, electroporation was performed. The overall process was carried out in the same manner as in Example D, and specific electroporation conditions for each sample are shown in Table 5 below.

**[Table 5]**

| **No.** | Conditions | **Transposon plasmid** | **Transposase** |
|---|---|---|---|
| 1 | Electroporation (EP) only (after one day) | - | - |
| 2 | 3M3-5M3-GFP + DNA (after one day) | 3M3-5M3-GFP (5 µg) | plasmid (5 µg) |
| 3 | 3M3-5M3-1G4 + DNA (after one day) | 3M3-5M3-1G4 TCR (5 µg) | plasmid (5 µg) |
| 4 | 3M3-5M3-1G4 + DNA (immediately after) | 3M3-5M3-1G4 TCR (5 µg) | plasmid (5 µg) |
| 5 | No EP (after one day) | - | - |

According to the Resting T cell 14-3 protocol of Maxcyte STx, electroporation was carried out, and then a cell suspension (5× 10⁶ cells/100 nL/well) was transferred from an OC100x2 assembly to a T25 flask, and the OC100X2 well was washed with 100 µL of AlyS medium and added to each T25 flask. After recovering the electroporated cells for 20 minutes at 37 °C in a CO₂ incubator, except for a 3M3-5M3-1G4 + DNA (immediately after) group, 800 µL of a medium (ALyS + 3% HS + 200 lU/mL IL-2) was carefully added to each T25 flask, and then the cells were incubated at 37 °C in a CO₂ incubator.

### 3. T cell activation after electroporation

As a T cell activation method, a method of specifically activating 1G4 TCR-T cells by adding A375 cells (irradiated with 50Gy radiation) expressing an NY-ESO-1 antigen, which is the target antigen of the 1G4 TCR, as feeder cells was carried out. Here, T cell activation was carried out in two periods, immediately after electroporation or one day after electroporation, and the results according to the activation period were compared. As shown in Table 6, in all groups excluding the 3M3-5M3-1G4 + DNA (immediately after) group, the immediately after electroporation group, and the 3M3-5M3-1G4 + DNA (immediately after) group, one day after electroporation, 50Gy-irradiated A375 cells as feeder cells were added to the electroporated PBMCs.

**[Table 6]**

| **No.** | **Conditions** | **Method of activating T cells** | **Activation time** |
|---|---|---|---|
| 1 | Electroporation (EP) only (after one day) | irradiated A375 cells | one day after EP |
| 2 | 3M3-5M3-GFP + DNA (after one day) | irradiated A375 cells | one day after EP |
| 3 | 3M3-5M3-1G4 + DNA (immediately after) | irradiated A375 cells | Immediately after EP |
| 4 | 3M3-5M3-1G4 + DNA (after one day) | irradiated A375 cells | one day after EP |
| 5 | No EP (after one day) | irradiated A375 cells | one day after EP |

### 3-1. T cell activation using "irradiated A375 cells immediately after electroporation" and culture

Immediately after electroporation, for the 3M3-5M3-1G4 + DNA (immediately after) group in which T cells were activated by adding the feeder cells, T cells were activated and cultured by the following method:
One vial of A375 irradiated with 50Gy radiation, which was cryopreserved, was thawed in a 37 °C water bath. 9 mL of ALyS medium was added to a 50 mL tube, and 1 mL of the thawed A375 cell suspension was slowly added. The tube was centrifuged at room temperature and 1,500 rpm for 5 minutes, a supernatant was removed, and the cell pellet was suspended in 10 mL of ALyS medium, followed by cell counting. 5 × 10⁶ of the A375 cells were transferred to a 15 mL tube and centrifuged at 1,500 rpm for 5 minutes, followed by removing a supernatant. The cell pellet was suspended in 800 µL of medium (ALyS + 3% HS + 200 lU/mL IL-2), and added, as shown in Table 6, to the 3M3-5M3-1G4+DNA (immediately after) group that was recovered after electroporation, followed by incubation at 37 °C in a CO₂ incubator. After one day, 1.5 mL of medium (ALyS + 3% HS + 200 lU/mL IL-2) was added, and the cells were incubated at 37 °C in a CO₂ incubator. After 2 days, 5 mL of medium (AIM-V + 3% HS + 1× P/S + 200 IU/mL IL-2) was added, and incubation was carried out at 37 °C in a CO₂ incubator. After three days, 10 mL of medium (AIM-V + 3% HS + 1× P/S + 200 IU/mL IL-2) was added, and then incubation was carried out at 37 °C in a CO₂ incubator. After six days, the cultured cells were suspended in the T25 flask and then transferred to a T75 flask, and 30 mL of medium (AIM-V + 3% HS + 1× P/S + 200 IU/mL IL-2) was added and then the flask was incubated at 37 °C in a CO₂ incubator. After 8 days, 20 mL of medium (AIM-V + 3% HS + 1× P/S + 200 IU/mL IL-2) was added to the T75 flask, and the cells were incubated at 37 °C in a CO₂ incubator. After 10 days, 40 mL of the medium was removed from the T75 flask using a pipette, and 30 mL of medium (AIM-V + 3% HS + 1× P/S + 200 IU/mL IL-2) was added, followed by incubation at 37 °C in a CO₂ incubator.

### 3-2. T cell activation using "irradiated A375 cells one day after electroporation" and culture

In Groups 1, 2, 4, and 5 in Table 6, T cells were activated and cultured by the following method:
One day after electroporation, one vial of A375 irradiated with 50Gy radiation, which was cryopreserved, was thawed in a 37 °C water bath. 9 mL of ALyS medium was added to a 50 mL tube, and 1 mL of the thawed A375 cell suspension was slowly added. 9 mL of ALyS medium was added to a 50 mL tube, and 1 mL of the thawed A375 cell suspension was slowly added. The tube was centrifuged at room temperature and 1,500 rpm for 5 minutes, a supernatant was removed, and the cell pellet was suspended in 10 mL of ALyS medium, followed by cell counting. 5×10⁶ of the A375 cells were transferred to a 15 mL tube and centrifuged at 1,500 rpm for 5 minutes. After removing a supernatant, the cell pellet was suspended in a medium (ALyS + 3% HS + 200 IU/mL IL-2) to be 2×10⁶ cells/500 µL, and then added to the flasks of the groups except the "3M3-5M3-1G4 + DNA (immediately after)" group under the condition of "irradiated A375 cells" in the column of "T cell activation method" in Table 6. Subsequently, 1 mL of medium (ALyS + 3% HS + 200 lU/mL IL-2) was added, and then the cells were incubated at 37 °C in a CO₂ incubator. After 3 days, 5 mL of medium (AIM-V + 3% HS + 1× P/S + 200 IU/mL IL-2) was added to each T25 flask, and the cells were incubated at 37 °C in a CO₂ incubator. After 6 days, 5 mL of medium (AIM-V + 3% HS + 1× P/S + 200 IU/mL IL-2) was added to each T25 flask, and the cells were incubated at 37 °C in a CO₂ incubator. After 8 days, for the "EP only (after one day) and irradiated A375 cell groups" and "No EP group," the cultured cells in the T25 flask were suspended and then transferred to a T75 flask, 10 mL of medium (AIM-V + 3% HS + 1× P/S + 200 lU/mL IL-2) was added, and then 5 mL of the medium was added to the flasks of the other groups, followed by incubation at 37 °C in a CO₂ incubator. After 10 days, 10 mL of the medium was removed from each flask using a pipette, and 10 mL of fresh medium (AIM-V + 3% HS + 1× P/S + 200 IU/mL IL-2) was added, followed by incubating at 37 °C in a CO₂ incubator.

### 4. FACS analysis

To analyze the gene delivery efficiency by a transposon system, FACS was carried out, and the overall process was carried out in the same manner as in the FACS method described in Example D.

### Example F. Production of CAR-T using pBat transposon system

### 1. Test materials and cells

As a pBat transposon plasmid, CD19 CAR gene-containing pBat transposon 3M3-5M3-CD19 CAR was used, and for transposase expression, a pBat transposase plasmid was also introduced.

As target cells for confirming the gene delivery efficiency of a transposon, LK053 PBMCs that had been isolated from a healthy person and cryopreserved were used.

### 2. T cell activation after electroporation

### 2-1. Electroporation and culture of PBMCs

After thawing two vials of LK053 PBMC cells stored in LN₂ in a 37 °C water bath, 18 mL of medium (AIM-V + 3% HS) was added to a 50 mL tube, and 2 mL of the thawed cell suspension was slowly added. The tube was centrifuged at room temperature and 1,500 rpm for 5 minutes, a supernatant was removed, and the cell pellet was suspended in 20 mL of medium (AIM-V + 3% HS), followed by cell counting. 3.5×10⁷ PBMCs were centrifuged at room temperature and 1,500 rpm for 5 minutes, a supernatant was removed, the cell pellet was suspended in 10 mL of PBS. Subsequently, the cell suspension was centrifuged at room temperature and 1,500 rpm for 5 minutes, a supernatant was completely removed, and the cell pellet was suspended in 350 µL of warm Opti-MEM to be 5×10⁶ cells/50 µL.

5 µg of each plasmid was added to a 1.5 mL tube for each condition shown in Table 7, and 50 µL (5×10⁶ cells) of the prepared PBMC suspension was added. The cell suspension in 6) was carefully added to an OC100X2 assembly without generating bubbles. Electroporation was carried out according to the Resting T cell 14-3 protocol of Maxcyte STx in the same manner as in Example D. After electroporation, the cell suspension from the OC100x2 assembly was transferred to each of two T25 flasks, an OC100X2 well was washed with 50 µL of AIM-V medium and then added to each T25 flask. The T25 flask was incubated at 37 °C in a CO₂ incubator for 20 minutes while being tilted on its short edge to allow time for cell recovery. After the recovery time, 900 µL of complete medium (AIM-V + 3% HS + 200 IU/mL IL-2) was carefully added to each T25 flask. The T25 flask was tilted on its short bottom edge and incubated for one day at 37°C in a CO₂ incubator.

**[Table 7]**

| No. | Group | Plasmid (5 µg each) |
|---|---|---|
| 1 | Electroporation (EP) only | X |
| 2 | 3M3-5M3-CD19 CAR | 3M3-5M3-CD19 CAR + transposase |

### 2-2. T cell activation and culture

One day after electroporation, 1.5 mL of complete medium (AIM-V + 3% HS + 200 IU/mL IL-2) was added to a T25 flask, 50 µL of Transact was added, and then incubated at 37 °C in a CO₂ incubator for 2 days to activate T cells. Three days after electroporation, 2.5 mL of complete medium (AIM-V + 3% HS + 200 lU/mL IL-2) was added to a T25 flask, and the cells were continuously incubated at 37 °C in a CO₂ incubator while the T25 flask was upright. Six days after electroporation, 3 mL of complete medium (AIM-V + 3% HS + 200 IU/mL IL-2) was added to a T25 flask, and the cells were continuously incubated at 37 °C in a CO₂ incubator while the T25 flask was upright. Seven days after electroporation, the cells were suspended and then 1 mL of the cell suspension was transferred to a FACS tube, followed by FACS analysis. The remaining cells were continuously incubated at 37 °C in a CO₂ incubator while the T25 flask was upright.

### 3. FACS analysis

To analyze the gene delivery efficiency by a transposon system, FACS was carried out 7 days after electroporation, and the overall process was carried out in the same manner as in the FACS method described in Example D.

### Example G. Additional verification of CAR-T production using pBat transposon system

### 1. Test materials and cells

As a pBat transposon plasmid, CD19 CAR gene-containing pBat transposon 3M3-5M3-CD19 CAR was used, and for transposase expression, a pBat transposase plasmid was also introduced.

As target cells for confirming the gene delivery efficiency of a transposon, LK053 PBMCs that had been isolated from a healthy person and cryopreserved were used.

### 2. T cell activation after electroporation

The introduction of the 3M3-5M3-CD19 CAR transposon and the transposase plasmid into PBMCs using electroporation and the activation of T cells after electroporation were performed in the same manner as in Example F.

### 3. FACS analysis

To analyze the gene delivery efficiency by a transposon system, FACS was carried out 7 days after electroporation, and the overall process was carried out in the same manner as in the FACS method described in Example D.

### Example H. Confirmation of in vitro efficacy of CAR-T cells produced using pBat transposon system

### 1. Cells

CAR-T cells (LK053 CAR-T) were used by introducing a CD19 CAR gene using the pBat transposon system in the same manner as in Example F or G and incubating them for 2 weeks. As a control, as in the production of CD19 CAR-T cells, cells (LK053 control) that underwent the same electroporation and 2-wek culture were used.

### 2. T Cell thawing and resting

Two vials each of LK053 control T cells stored in LN₂ and CD19 CAR-T produced with the transposon system were thawed in a 37 °C water bath. 18 mL of medium (ALyS + 3% HS) was added to each of two 50 mL tubes, and 2 mL of the thawed cell suspension was slowly added to each tube. After centrifugation at room temperature and 1,500 rpm for 5 minutes, a supernatant was removed, and the cell pellet was suspended in 10 mL of medium (ALyS + 3% HS), followed by cell counting. Subsequently, the cells were transferred to a T75 flask, and a total of 50 mL of medium (ALyS + 3% serum) was added to be 1×10⁷ cells/10 mL. The cells were incubated at 37 °C in a CO₂ incubator for one day and then rested.

### 3. Co-culture of T cells and B cell line

A CD19-expressing B cell line (BJAB cells) was incubated and then collected in a 15 mL tube. After centrifugation at room temperature and 1,500 rpm for 5 minutes, a supernatant was removed, the cell pellet was suspended in 2 mL of ALyS medium, followed by cell counting. The cell suspension was centrifuged again at room temperature and 1,500 rpm for 5 minutes, a supernatant was completely removed, and then the cell pellet was suspended in a medium (ALyS + 3% HS) to be 1 ×10⁵ cells/100 µL. 1×10⁵ of the suspended BJAB cells were added to each well of a 96-well plate according to the conditions of Table 8. The control T cells and CD19 CAR-T cells rested in the previous example were collected in respective 50 mL tubes, centrifuged at room temperature and 1,500 rpm for 5 minutes to remove a supernatant, and the cell pellet was suspended in 10 mL of medium (ALyS + 3% HS), followed by cell counting. 2×10⁶ of the control T cells and 6×10⁶ of the CD19 CAR-T cells were divided into two 15 mL tubes, respectively. Subsequently, each cell suspension was centrifuged at room temperature and 1,500 rpm for 5 minutes, and a supernatant was completely removed. A pellet of the 2×10⁶ cells was suspended in 2 mL of medium (ALyS + 3% HS) to be 1×10⁵ cells/100 µL, and a pellet of the 6×10⁶ cells was suspended in 1.5 mL of medium (ALyS + 3% HS) to be 4×10⁵ cells/100 µL.

The prepared cells were added at 100 µL/well to a 96-well plate in which BJAB cells had been previous dispensed according to the conditions of Table 8. As a positive control, a PMA/Iono group was treated with 100 nM of PMA and 1 µg/mL of ionomycin, and incubated at 37 °C in a CO₂ incubated for 24 hours. After 24 hours, the plate was centrifuged at room temperature and 1,500 rpm for 5 minutes. To measure the amount of IFN-γ secreted in the reaction of the CD19 CAR-T cells with target cells, 130 µL of the cell culture was recovered from each well and then transferred to a new 96 well plate, and the plate was sealed with foil and then stored at -80 °C.

**[Table 8]**

| **No.** | **Group (B cell :T cell ratio)** | **Well number** | **Number of B cells (BDCM or BJAB)** | **Number of T cells** |
|---|---|---|---|---|
| 1 | T only control (1:1) | 3 | - | 1 × 10⁵ |
| 2 | B + T (1:1) | 3 | 1 × 10⁵ | 1 × 10⁵ |
| 3 | T only control (1:4) | 3 | - | 4 × 10⁵ |
| 4 | B + T (1:4) | 3 | 1 × 10⁵ | 4 × 10⁵ |
| 5 | PMA/Iono | 3 | - | 1 × 10⁵ |
| 6 | B only | 3 | 1 × 10⁵ | - |

### 4. IFN-γ ELISA assay

ELISA was carried out according to the protocol of a human IFN-gamma ELISA assay kit. The cell culture stored at -80 °C in the previous example was slowly thawed on ice, standard IFN-γ was dissolved in 100 µL nuclease-free water and prepared at a concentration of 20,000 pg/mL. 20,000 pg/mL of IFN-γ was diluted with an assay diluent to prepare standard IFN-γ with different concentrations of 1,500, 750, 375, 187.5, 93.75, 46.875, and 23.438 pg/mL. 20x wash buffer was diluted 1/20 with sterile water to prepare 1x wash buffer, 200 µL of the 1× wash buffer was added to an IFN-γ ELISA plate, and then the plate was inverted to remove the solution (first washing). Subsequently, 300 µL of the 1× wash buffer was added and then the plate was inverted to remove the solution (second washing). These two types of washing processes were repeated twice to perform a total of four washings. For final solution removal, a paper towel was used to completely remove the solution. 100 µL of the thawed cell culture was added to each IFN-γ ELISA plate that had been washed. Subsequently, 100 µL of the standard IFN-γ of each concentration was added to two wells. To two other wells, 100 µL of ALyS medium was added to prepare a blank. With a plate cover attached thereto, the plate was allowed to react at room temperature for 2 hours. After completing the reaction, the plate was inverted to remove the medium, and then after adding 200 µL of 1× wash buffer, the solution was removed by inverting the plate (first washing). Subsequently, 300 µL of the 1× wash buffer was added, and then the solution was removed by inverting the plate (second washing). These two types of washing processes were repeated twice to perform a total of four washings. For final solution removal, a paper towel was used to completely remove the solution. During the washing process, detection antibodies were dissolved in 300 µL nuclease-free water and diluted 1/20 with an assay diluent. 100 µL of the diluted detection antibodies were dispensed into each well of the washed plate and allowed to react at room temperature for 2 hours after a plate cover was attached to the plate. The plate was inverted to remove the medium, and 200 µL of 1× wash buffer was added and then the solution was removed by inverting the plate (first washing). Subsequently, 300 µL of the 1× wash buffer was added and then the plate was inverted to remove the solution (second washing). These two types of washing processes were repeated twice to perform a total of four washings. For final solution removal, a paper towel was used to completely remove the solution. During the washing process, streptavidin-HRP was diluted 1/20 with an assay diluent. 100 µL of the diluted streptavidin-HRP was added to each well of the washed plate. With a plate cover attached thereto, the plate was allowed to react at room temperature for 30 minutes. After completing the reaction, the plate was inverted to remove the medium, and then after adding 200 µL of 1× wash buffer, the solution was removed by inverting the plate (first washing). Subsequently, 300 µL of the 1× wash buffer was added, and then the solution was removed by inverting the plate (second washing). These two types of washing processes were repeated twice to perform a total of four washings. For final solution removal, a paper towel was used to completely remove the solution. 100 µL of TMB was added to each well of the washed plate and allowed to react at room temperature for 5 minutes. 100 µL of a stop solution was added to each well to stop the reaction. Absorbance at 450 nm was measured using Multiscan sky equipment and a concentration was calculated.

### Example I. Confirmation of gene transfer efficiency according to type of pBat transposon delivery

### 1. Cells and DNA molecules

As target cells for confirming the gene delivery efficiency of a transposon, Jurkat cells (ATCC, Cat No. TIB-152, Lot no. 70017560) was used.

For a transposon system, as a control, a pEGFP-C1 plasmid was used, and to confirm the gene delivery efficiency according to the type of transposon delivery, the following three types of transposons were used: i) Transposon 3M3-5M3-GFP plasmid (plasmid type), ii) Transposon 3M3-5M3-GFP linear dsDNA (linear dsDNA type), and iii) Transposon 3M3-SM3-GFP minicircle dsDNA (minicircle dsDNA type).

In addition, for transposase expression, a pBat transposase plasmid was also introduced.

### 2. Neon electroporation

Cultured Jurkat cells were collected in a 50 mL tube, and centrifuged at room temperature and 1,500 rpm for 5 minutes. After removing a supernatant, the cell pellet was suspended in 10 mL of medium (RPMI + 10% FBS), and then cell counting was carried out. 4×10⁶ of the cells were transferred to a 15 mL tube, medium (RPMI + 10% FBS) was added to the final volume of 2 mL, and then centrifugation was performed at room temperature and 1,500 rpm for 5 minutes. A supernatant was removed from the centrifuged sample, and the cell pellet was suspended in 5 mL of Opti-MEM and then centrifuged at room temperature and 1,500 rpm for 5 minutes. Subsequently, 3 mL of electrolytic buffer was added to a Neon tube, and 400 µL of medium (RPMI + 10% FBS) was added to each well of a 24-well plate. A supernatant was removed from the centrifuged Jurkat cell sample, and the cell pellet was suspended in Opti-MEM to be 1×10⁵ cells/100 µL. 4.2×10⁵ cells of the resuspended cells were transferred to a 1.5 mL tube. 1 µg of DNA or mRNA (per 1×10⁵ cells) was added to each 1.5 mL tube for each condition as shown in Table 9. Here, the total volume of the added DNA or mRNA was adjusted not to be more than 10% (10 µL) of the electroporation volume (100 µL).

Subsequently, a Neon tube containing an electrolyte buffer was put in the Neon device, 100 µL of the prepared cell suspension was slowly suctioned using a Neon pipette and a Neon tip, and then the tip was inserted into the Neon device. Electroporation was performed under conditions of 1,600 V, 10 ms, and 3 pulses, and the electroporated cells were seeded in a 24-well plate into which a medium (RPMI + 10% FBS) had been previously dispensed and incubated at 37 °C in a CO₂ incubator.

One day after electroporation, under each condition, the cells were collected from the two wells (the plate observed after one day) to perform FACS analysis, and 1.5 mL of culture medium (RPMI + 10% FBS + 1× P/S) was added to each of the remaining two wells (the plate observed after seven days) and followed by incubation at 37 °C in a CO₂ incubator.

Seven days after electroporation, for FACS analysis, the medium in each well was pipetted to suspend the cells, and 1 mL of the cells out of the total of 2 mL was recovered. In addition, 1 mL of culture medium (RPMI + 10% FBS + 1× P/S) was added to 1 mL of the cell suspension remaining in each well, and then the cells were incubated at 37 °C in a CO₂ incubator.

Eleven days after electroporation, the medium in each well was pipetted to suspend the cells, and 1 mL of the cells out of the total of 2 mL were transferred to a new well. 1 mL of culture medium (RPMI + 10% FBS + 1× P/S) was added to each well, and then the cells were incubated at 37 °C in a CO₂ incubator.

Fourteen days after electroporation, the medium in each well was pipetted to suspend the cells, and 1 mL of the cells out of the total of 2 mL were recovered to perform FACS analysis.

**[Table 9]**

| **Condition** | **Plasmid** | **Well number** |
|---|---|---|
| 1 | No electroporation (EP) | 4 |
| 2 | EP only | 4 |
| 3 | 3M3-5M3-GFP plasmid + transposase plasmid | 4 |
| 4 | 3M3-SM3-GFP linear dsDNA + transposase plasmid | 4 |
| 5 | 3M3-5M3-GFP minicircle dsDNA + transposase plasmid | 4 |
| 6 | pEGFP | 4 |
| 7 | EGFP mRNA | 4 |

### 3. Observation of GFP expression using fluorescence microscope

GFP fluorescence expressed in the Jurkat cells was observed using a fluorescence microscope 1, 7, and 14 days after electroporation.

### 4. FACS analysis

On day 1, 7, and 14 days after electroporation, GFP expression was observed using a fluorescence microscope, followed by FACS analysis in the same manner as in the previous example.

### Example J. Confirmation of antibody gene delivery efficiency by pBat transposon system

### 1. Cells and DNA molecules

As target cells for confirming the gene delivery efficiency of a transposon, a HEK293 cell line (Korean Cell Line Bank, Cat no. KCLB21573 T, Lot no. 46269) was used.

To confirm the antibody gene delivery efficiency of a transposon system, as a representative example, JWW-2 antibodies were used (JWW-2 human chimeric monoclonal antibodies; Addgene, Cat no. 66749).

In addition, for a transposon system, as a control, a pEGFP-C1 plasmid was used, and to confirm the gene delivery efficiency of each transposon system, transposon B3IS-B5IE-JWW-2, transposon 3M3-5M3-JWW-2, transposon 3M3-5M4-JWW-2, transposon B3IS-BSIE-GFP, and transposon 3M3-5M3-GFP, in which a JWW antibody gene was integrated, were used.

In addition, for transposase expression, a pBat transposase plasmid was also introduced.

### 2. Neon electroporation

After removing the medium from a 100π plate where the HEK293 cells were cultured for 3 days, 4 mL of PBS was added to wash the plate and then removed. Subsequently, 1 mL of trypsin-EDTA was added and allowed to react at 37 °C in a CO₂ incubator for 2 minutes. The incubated cells were collected in a 15 mL tube using a culture medium (DMEM + 10% FBS, 1× P/S), centrifuged at room temperature and 1,500 rpm for 5 minutes to remove a supernatant, and the pellet was suspended in 5 mL of PBS, followed by cell counting. Additionally, the cells were centrifuged at room temperature and 1,500 rpm for 5 minutes, a supernatant was removed, and then the cell pellet was suspended in a resuspension buffer to be 4×10⁵ cells/90 µL. 26×10⁵ of the cells were transferred to one 1.5 mL tube, and 18×10⁵ of the cells were transferred to each of six 1.5 mL tubes. 900 µL of medium (DMEM + 10% FBS) was added to each well of a 6-well plate, and 3 mL of electrolytic buffer was dispensed in a Neon tube. Subsequently, 1 µg of a plasmid (per 4×10⁵ cells) was added to each tube prepared above for each condition.

### [Transfection groups]

| **No.** | **Name** | **Plasmid (1 µg each)** | **Number of Wells** |
|---|---|---|---|
| 1 | Electroporation (EP) only | X | 6 |
| 2 | pEGFP | pEGFP | 4 |
| 3 | B3IS-B5IE-GFP | pBat B3IS-BSIE-GFP + transposase | 4 |
| 4 | 3M3-5M3-GFP | pBat 3M3-SM3-GFP + transposase | 4 |
| 5 | B31S-BSIE-JWW-2 | pBat 63IS-65IE-JWW-2 + transposase | 4 |
| 6 | 3M3-5M3-JWW-2 | pBat 3M3-5M3-JWW-2 + transposase | 4 |
| 7 | 3M3-5M4-JWW-2 | pBat 3M3-5M4-JWW-2 + transposase | 4 |

The total volume was adjusted with 100 µL per 4×10⁵ cells with a resuspension buffer, and a Neon tube containing an electrolytic buffer was put into a Neon device. 100 µL of the prepared cell suspension was slowly suctioned using a Neon pipette and a Neon tip, and the tip was then inserted into the Neon device. Electroporation was performed under conditions of 1,600 V, 10 ms, and 3 pulses, and the electroporated HEK293 cells were dispensed in a 6-well plate containing a transfection buffer and incubated at 37 °C in a CO₂ incubator.

One day after electroporation, the cells were recovered from two wells of each group, and FACS analysis was performed on a GFP-containing group to measure GFP, and total RNA was extracted from a JWW-2-containing group to confirm antibody expression. 1 mL of culture medium (DMEM + 10% FBS, 1× P/S) was added to all wells from which some cells were recovered, and the cells were incubated at 37 °C in a CO₂ incubator. Three days after electroporation, 1 mL of the culture medium was taken from each well and stored in a deep freezer to measure expressed antibodies, and the cells were subcultured from one well to two wells and incubated at 37 °C in a CO₂ incubator (subcultured from 2 to 4 wells for each group). Seven days after electroporation, the cells were recovered from two wells of each group, for a GFP-containing group, FACS was performed to analyze GFP expression, and total RNA was extracted from a JWW-2-containing group. The cells in the remaining wells were subcultured from one well to two wells, and incubated at 37 °C in a CO₂ incubator (subcultured from 2 to 4 wells for each group). Ten days after electroporation, the culture medium was taken from each well of 15), and stored in a deep freezer.

### 3. Observation of GFP expression using fluorescence microscope and FACS analysis

One day, 7 days, and 10 days after electroporation, the GFP fluorescence expressed in the Jurkat cells was observed using a fluorescent microscope. Protein observation using a fluorescence microscope and FACS analysis were performed in the same manner as in the previous example.

### 4. Real time PCR analysis

Total RNA was extracted from HEK293 cells recovered 1 day and 7 days after electroporation according to the manual using a RNeasy kit. 1.0 µg of total RNA was added to each premix tube, and a cDNA was synthesized according to the manual using a CDNA synthesis kit. A qPCR mixture was prepared as follows.

### [qPCR mixture (preparation of three tubes per group)]

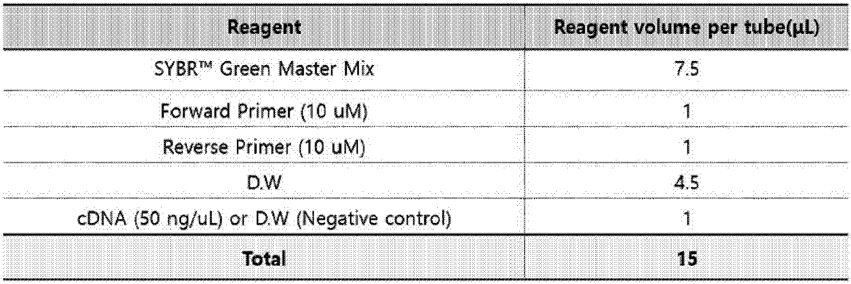

The mixture of 3) was added to each well of a 96-well plate for real-time PCR. After putting the plate in qPCR equipment, qPCR was carried out under the following conditions, and after completing the qPCR, melting analysis was performed.

### [qPCR cycle conditions]

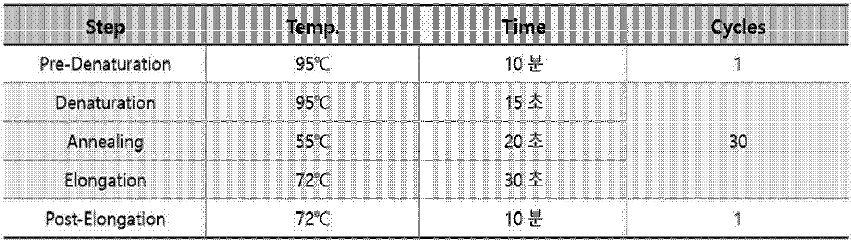

### 5. ELISA assay

The media that had been recovered 3 days and 10 days after electroporation, stored in a cryopreserved state, were slowly melted on ice, and centrifuged at 4 °C and 1,600 rpm for 5 minutes. The supernatant was carefully taken and then transferred to a new 1.5 mL tube. 600 µL of 1X assay diluent was added to an IgG1 standard vial to prepare a 300 ng/mL standard, and the standard was diluted with the 1X assay diluent and then prepared with different concentrations as below.

### [Standard dilution]

| **Standard** | **Dilution ratio** | **Dilute (uL)** | **1X assay diluent (uL)** | **Total Volume (uL)** | **Final concentration (ng/mL)** |
|---|---|---|---|---|---|
| 1 | 1/7.5 | 80 (standard) | 520 | 600 | 40 |
| 2 | 1/3 | 200 (standard #1) | 400 | 600 | 13.3 |
| 3 | 1/3 | 200 (standard #2) | 400 | 600 | 4.44 |
| 4 | 1/3 | 200 (standard #3) | 400 | 600 | 1.48 |
| 5 | 1/3 | 200 (standard #4) | 400 | 600 | 0.49 |
| 6 | 1/3 | 200 (standard #5) | 400 | 600 | 0.16 |
| 7 | 1/3 | 200 (standard #6) | 400 | 600 | 0.05 |
| 8 | 1/3 | 0 | 400 | 400 | 0 |

100 µL of the standard of each concentration and a sample were added to an ELISA plate in duplicate. The plate was gently shaken while being allowed to react at room temperature for 2.5 hours. After removing the solution, 300 µL of 1X wash solution was added to each well to wash the cells and then removed. This process was performed three additional times for a total of 4 washings. Subsequently, 100 µL of 1X biotinylated IgG1 detection antibody was added to each well. The antibody was allowed to react at room temperature for 1 hour while being gently shaken, and then the washing process described above was performed a total of four times. 100 µL of 1X HRP-Streptavidin solution was added to each well of the washed sample, and allowed to react at room temperature for 45 minutes while being gently shaken. After four washing processes, 100 µL of TMB one-step substrate reagent was added to each well. After being allowed to react with gentle shaking at room temperature for 30 minutes, 50 µL of a stop solution was added to each well. Absorbance was measured at 450 nm to analyze the JWW-2 antibody concentration.

### Example K. Confirmation of gene delivery efficiency according to size of pBat transposon vector

### 1. Cells and DNA molecules

As target cells for confirming the gene delivery efficiency of a transposon, Jurkat cells (ATCC, Cat No. TIB-152, Lot no. 70017560) were used.

To confirm the gene delivery efficiency according to the size of a transposon vector, transposon wild-type (wild-type), transposon B3IS-B5IE (7,562 bp), and transposon EF1α-B3IS-B5IE-EGFP-KanR (4,149 bp) were used, and as a control, a pEGFP-C1 plasmid was used.

In addition, for transposase expression, a pBat transposase plasmid was also introduced.

### 2. Neon electroporation

To introduce the transposon vector into the Jurkat cells, Neon electroporation was performed in the same manner as in Example B. Experimental conditions are as shown in the following table.

### [Electroporation conditions]

| **No.** | **Plasmid** | **Number of wells** |
|---|---|---|
| 1 | Electroporation (EP) only | 4 |
| 2 | pEGFP | 4 |
| 3 | Wild-type + transposase | 4 |
| 4 | B3IS-B5IE + transposase | 4 |
| 5 | EF1α-B3IS-B5IE-EGFP-KanR (B3IS-B5IE small) + transposase | 4 |

### 3. Fluorescence microscopy and FACS analysis

To confirm the gene delivery efficiency for each transposon, fluorescence microscopy and FACS analysis were performed in the same manner as in the previous example.

### [Experimental Results]

### Experimental Example A. ITR detection and identification of its function

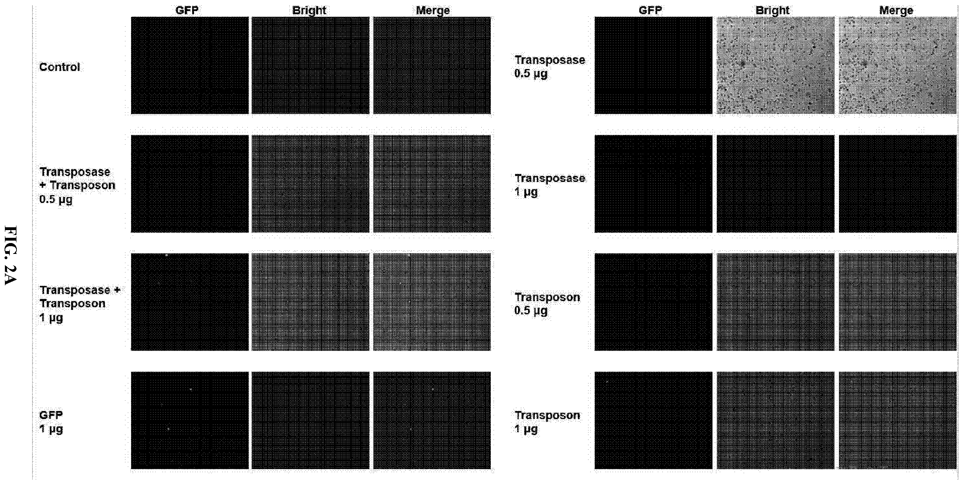

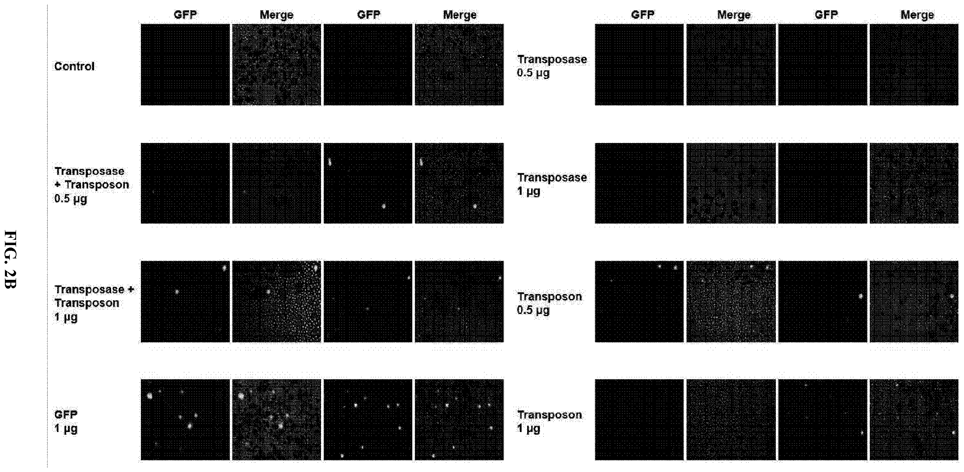

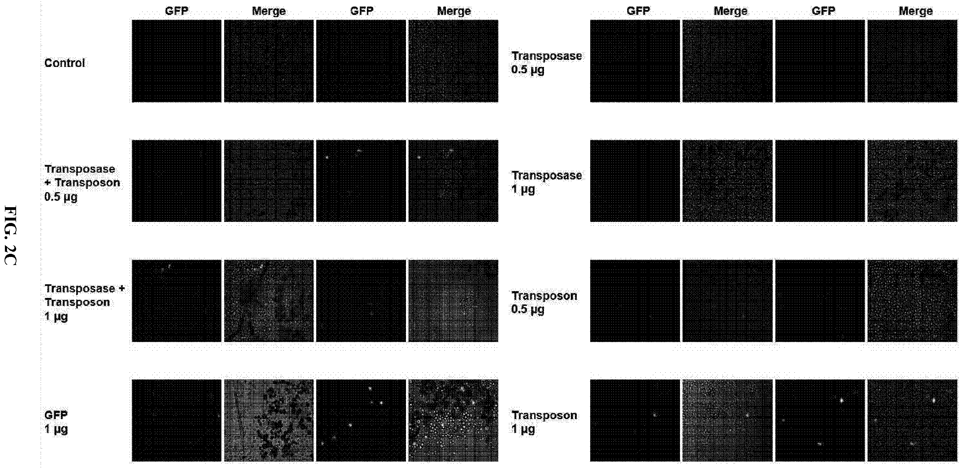

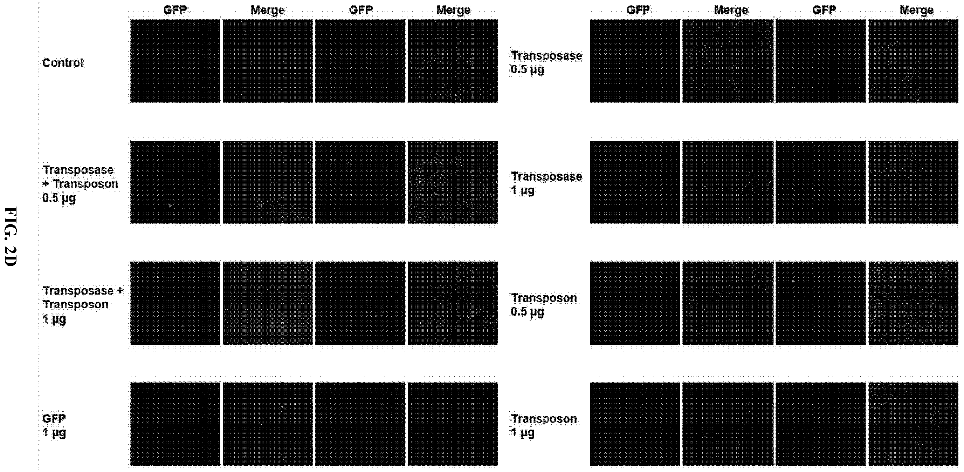

### 1. Fluorescence microscopy (FIGS. 2A to 2D)

On day 1, 2, 3, and 6 after transfection (electroporation) of intact transposons having SEQ ID NOs: 1 and 2 and a transposase alone or in combination into T cells, GFP expression was confirmed with a fluorescence microscope. As a result, until day 3, it was judged that the transposase was transiently expressed because it was even expressed in the GFP control without an ITR. However, on day 6, it was identified that GFP-expressing cells were not observed in the GFP control, whereas GFP was weakly expressed in the cells in which a transposon and a transposase were co-transfected, confirming that integration into the cell's chromosome was achieved through an ITR.

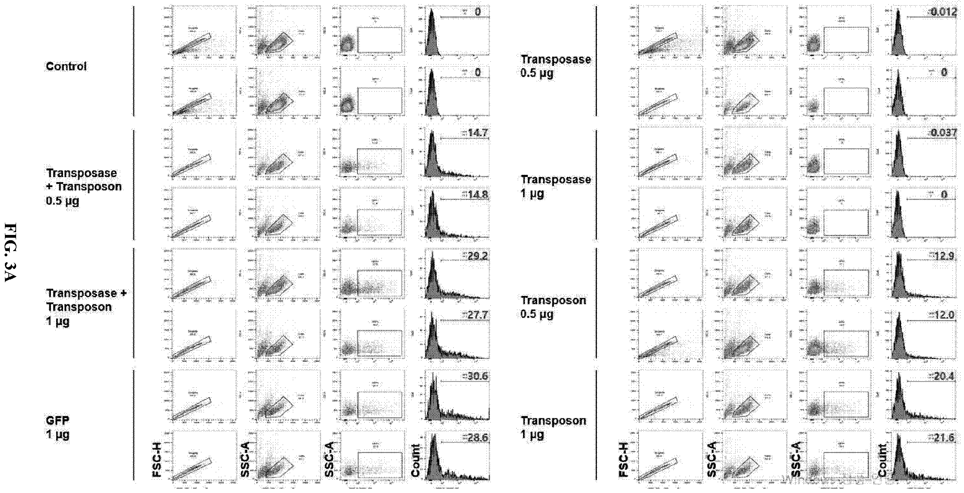

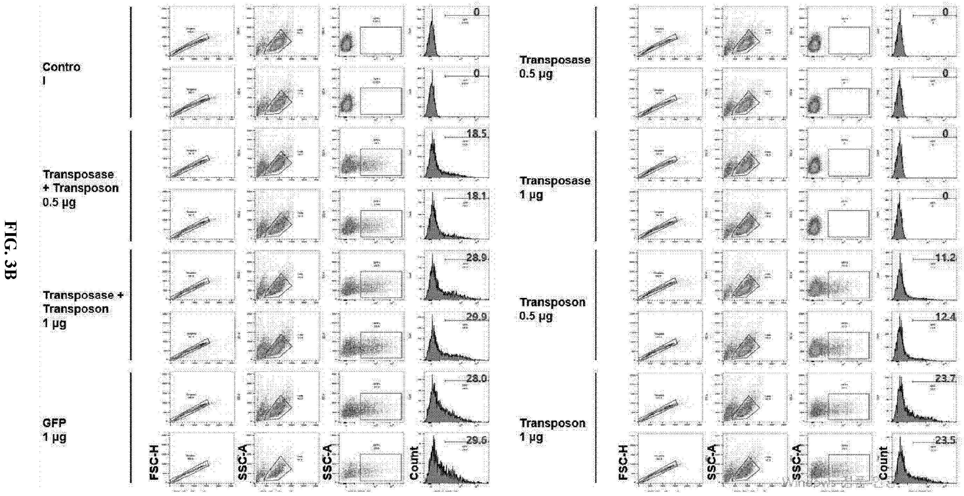

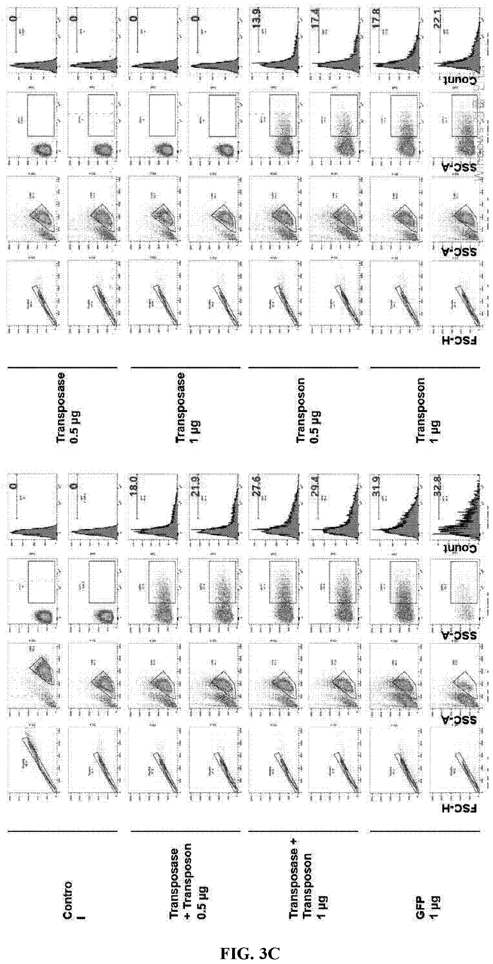

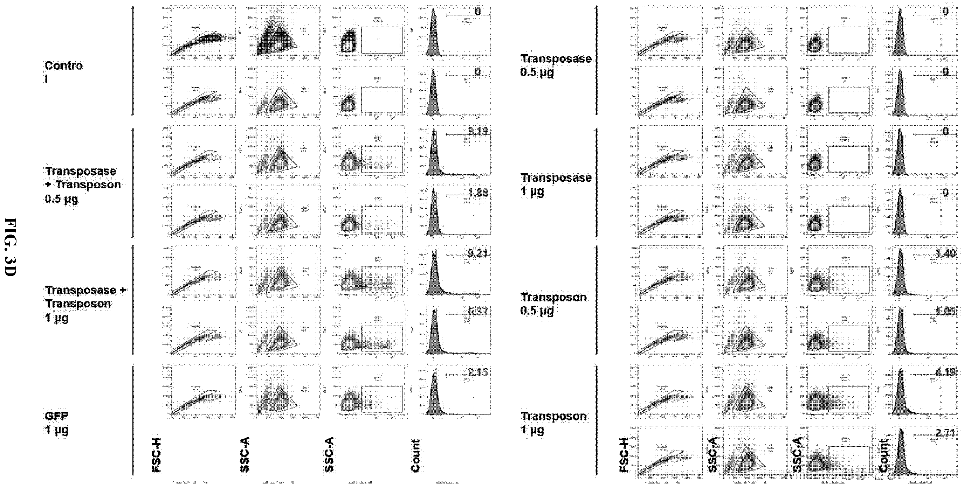

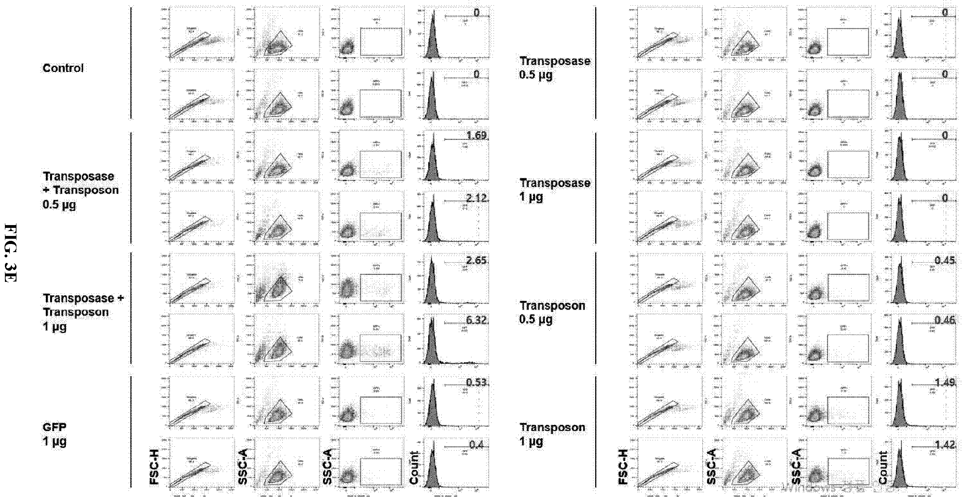

### 2. FACS analysis (FIGS. 3A to 3E)

The GFP expression ratio of the cells observed under a fluorescence microscope was confirmed by FACS. As a result, similar to the fluorescence microscopy result, it was confirmed that, on day 7, GFP was hardly expressed in the GFP control without an ITR, whereas GFP was observed in the cells in which a transposon and a transposase were co-transfected.

Although there was a difference in degree until 3 days after transfection, since GFP was expressed even in the GFP control that include neither the pCAG-EGFP-ITR transposon nor an ITR, it was confirmed that GFP was transiently expressed. However, 6 days after transfection, GFP was hardly expressed in the GFP control that does not include an ITR, and GFP was observed in the cells in which the pCAG-EGFP-ITR transposon and a transposase were co-transfected, confirming that integration into the chromosome was achieved.

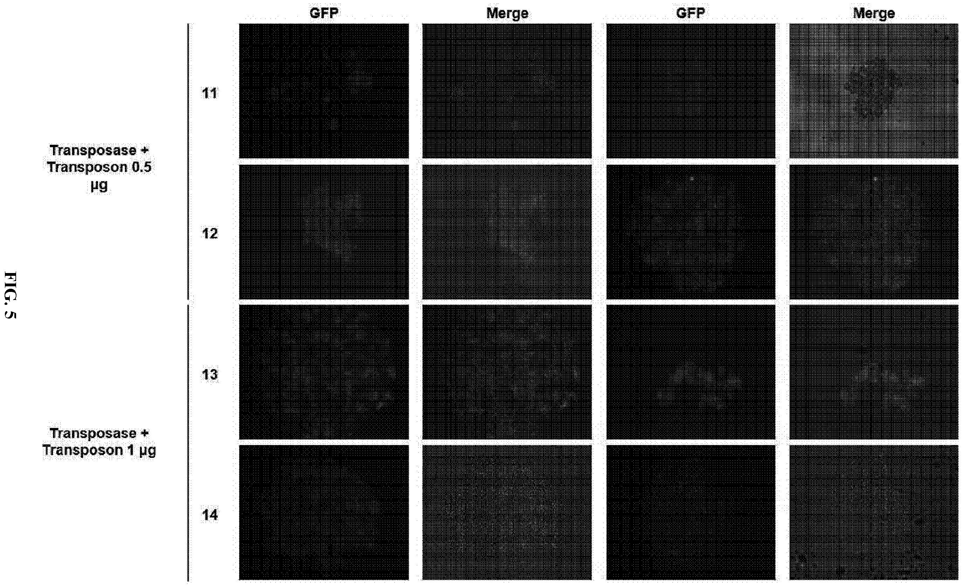

### 3. GFP positive cell sorting and confirmation of GFP expression (FIG. 5)

Seven days after co-transfection of the pCAG-EGFP-ITR transposon and a transposase, GFP-expressing cells were sorted, and the sorted cells were further cultured for 10 days, followed by confirming GFP expression using a fluorescence microscope. As a result, it was confirmed that GFP was continuously expressed in the cells in which the pCAG-EGFP-ITR transposon and a transposase were co-transfected, and GFP was stably expressed by integration into the chromosome.

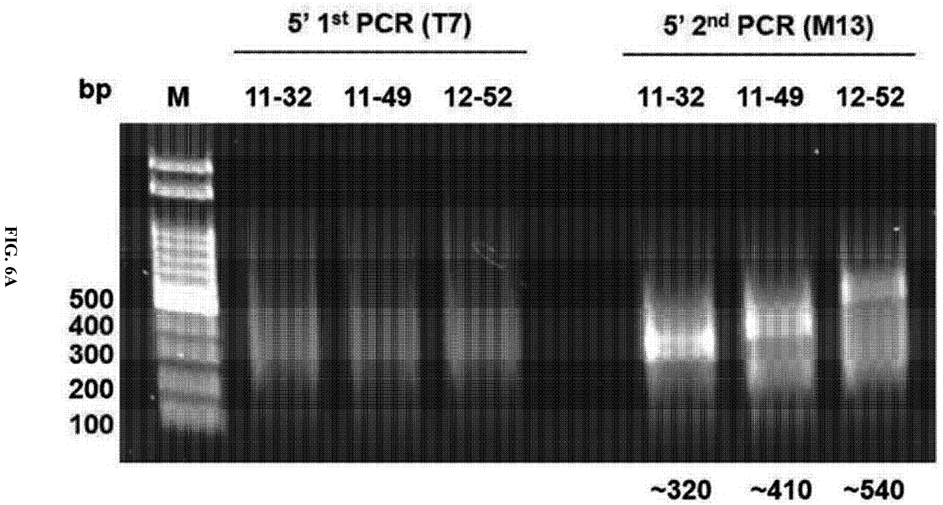

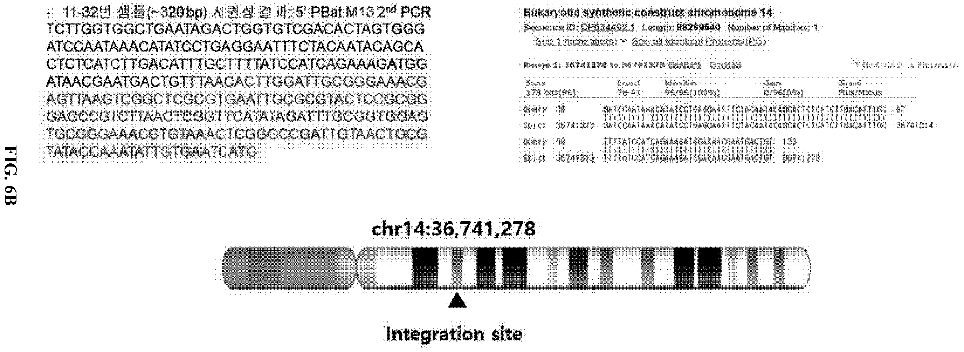

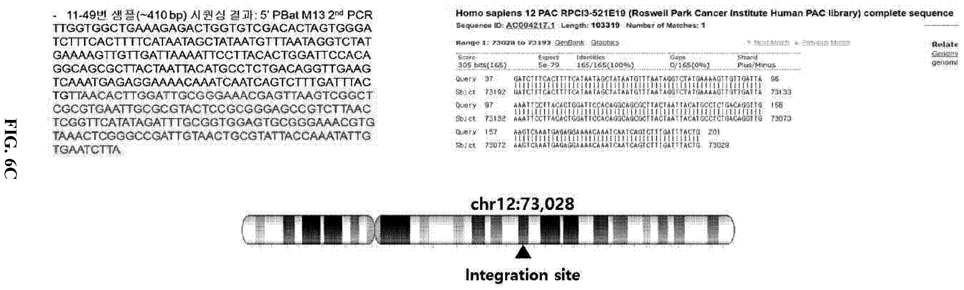

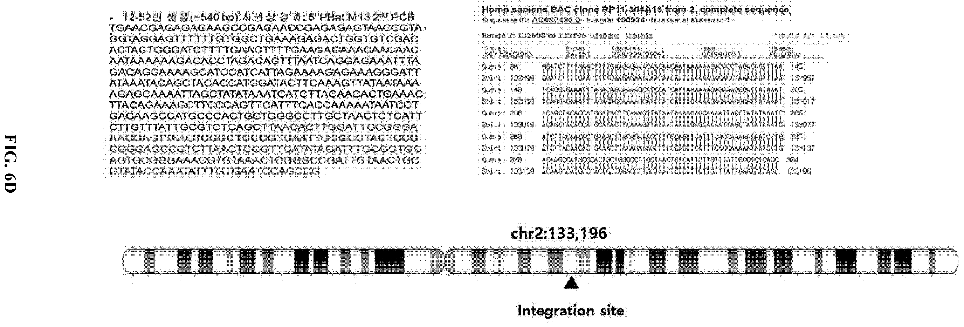

### 4. Confirmation of DNA integration site in chromosome using splinkerette PCR method (FIGS. 6A to 6D)

After sorting, to confirm whether DNA was integrated into the chromosome by an ITR in GFP-expressing cells, three clones were analyzed by splinkerette PCR. As a result of PCR, only a single band was confirmed, and as a result of sequencing and analyzing the PCR product of the single band, it was confirmed that it was integrated into chromosomes 2, 12, and 14, respectively.

### Experimental Example B. Production of ITR mutant and identification of its function

From the previous experimental example, it was confirmed that the transposon discovered by the present inventors can deliver and integrate a gene into the chromosome of a target cell. Accordingly, in this experimental example, deletion mutants for the 3' ITR and 5' ITR regions of the transposon were produced, and the gene delivery efficiency of each mutant form was compared to produce a mutant transposon whose function is further improved.

### 1. Production of mutant form for ITR

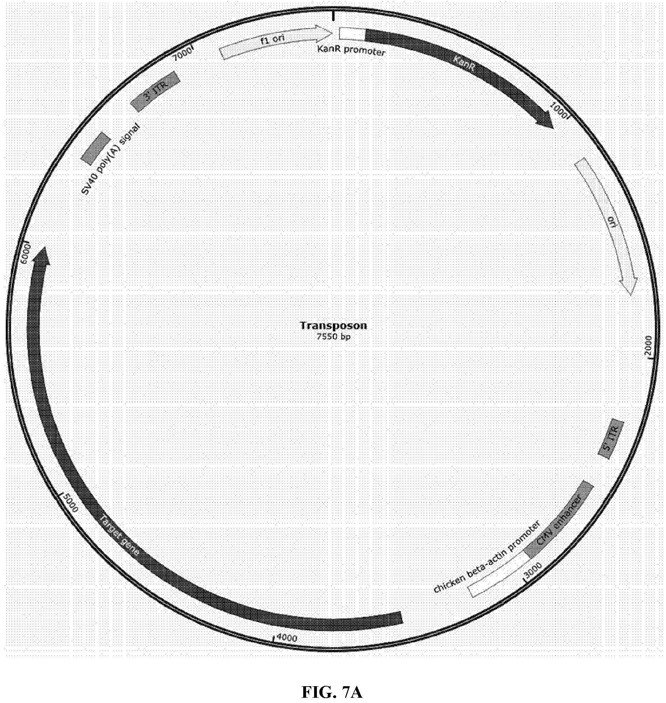

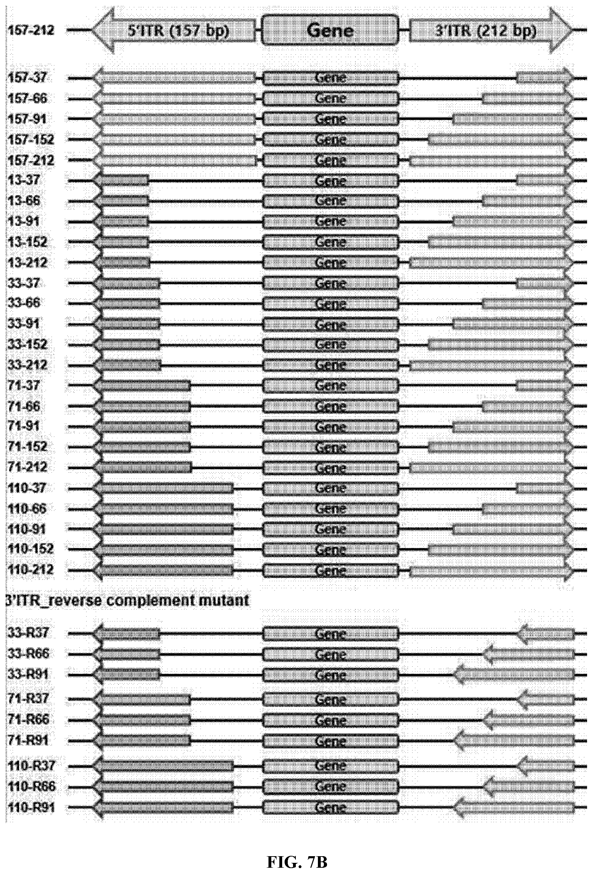

The original transposon backbone vector map for producing a transposon mutant according to the present invention is shown in FIG. 7A. In addition, the types of transposon mutant vectors that can be obtained by modifying the 5' ITR and 3' ITR of a pBat transposon are overall shown in FIG. 7B (also including 3' ITR mutant (reverse)).

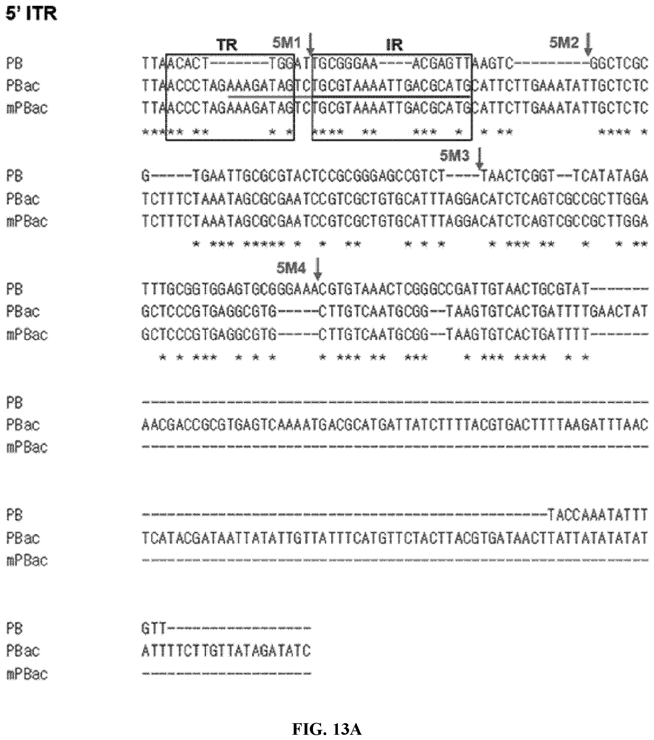

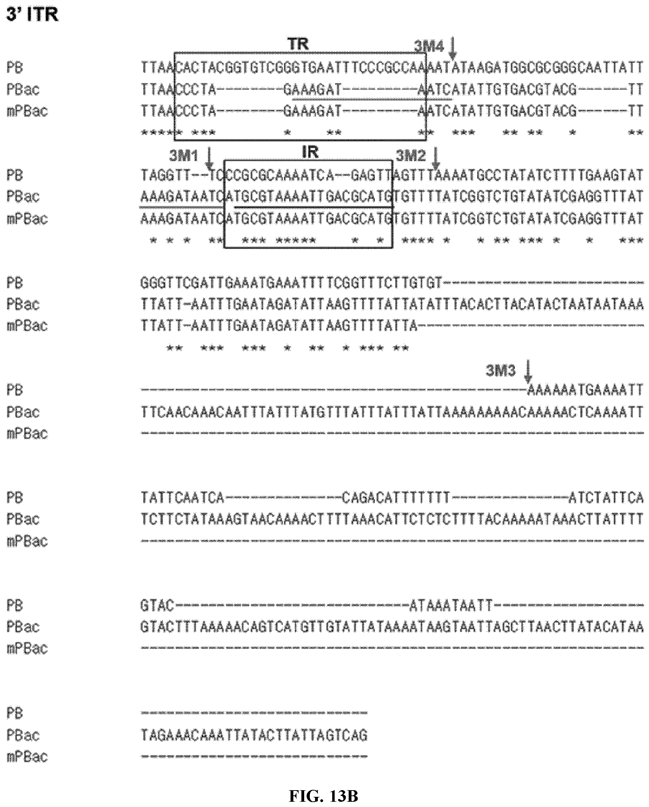

In this experimental example, mutant forms were selected by alignment of the ITR sequence of pBat and the ITR sequence of piggyBac, and here, the location of IR/TR was predicted based on the known piggyBac ITR sequence. The mutant form was designed by selecting a location with or without IT and TR sequences and not aligned with piggyBac (FIGS. 13A and 13B).

Four mutants with a 13-bp, 33-bp, 71-bp, or 110-bp sequence from the 5' end of the 5' ITR (157 bp) were designed, and four mutants having a 37-bp, 66-bp, 91-bp, or 151-bp sequence of the 3' ITR (212 bp) were designed.

The sequence of each mutant is as follows. As to be described below, in the case of the 3' ITR mutant, since mutants in which an antisense strand with the reverse complement sequence of a sense strand was accidentally introduced in plasmid production were produced, the sequences of these antisense strands are also listed (sequences indicated with "reverse" in Table 11 below).

**[Table 10]**

| **5' ITR mutant (the sequence is in 5' →3' direction based on sense strand)** | | | |
|---|---|---|---|
| Classification | Name | Sequence | SEQ ID NO: |
| Intact | Intact: 5' ITR_157 ("B5IE") | 5'- ttaacacttggattgcgggaaacgagttaagtcggctcgcgtgaattgcgcg tactccgcgggagccgtcttaactcggttcatatagatttgcggtggagtgc gggaaacgtgtaaactcgggccgattgtaactgcgtattaccaaatatttgtt- 3' | 1 |
| Mutant #1 (13 mer) | 5' ITR_13 ("5M1") | 5'-ttaacacttggat-3' | 3 |
| Mutant #2 (33 mer) | 5' ITR_33 ("5M2") | 5'-ttaacacttggattgcgggaaacgagttaagtc-3' | 4 |
| Mutant #3 (71 mer) | 5' ITR_71 ("5M3") | 5'- ttaacacttggattgcgggaaacgagttaagtcggctcgcgtgaattgcgcg tactccgcgggagccgtct-3' | 5 |
| Mutant #4 (110 mer) | 5' ITR_110 ("5M4") | 5'- ttaacacttggattgcgggaaacgagttaagtcggctcgcgtgaattgcgcg tactccgcgggagccgtcttaactcggttcatatagatttgcggtggagtgc gggaaa-3' | 6 |

**[Table 11]**

| **3' ITR mutant (the sequence is in 5' →3' direction based on sense strand)** | | | |
|---|---|---|---|
| Classification | Name | Sequence | SEQ ID NO: |
| Intact | Intact: 3' ITR 212 ("B3IS") | 5'- aattatttatgtactgaatagataaaaaaatgtctgtgattgaataaattttcattttttacacaa gaaaccgaaaatttcatttcaatcgaacccatacttcaaaagatataggcattttaaactaac tctgattttgcgcgggaaacctaaataattgcccgcgccatcttatattttggcgggaaattc acccgacaccgtagtgttaa-3' | 2 |
| Mutant # 1 (66 mer) | 3' ITR_66 ("3M1") | 5'- aacctaaataattgcccgcgccatcttatattttggcgggaaattcacccgacaccgtagt gttaa-3' | 9 |
| Mutant #1 reverse (66 mer) | 3' ITR_66 ("r3M1") | 5'- ttaacactacggtgtcgggtgaatttcccgccaaaatataagatggcgcgggcaattattt aggtt-3' | 15 |
| Mutant #2 (91 mer) | 3'ITR_91 ("3M2") | 5'- aaactaactctgattttgcgcgggaaacctaaataattgcccgcgccatcttatattttggcg ggaaattcacccgacaccgtagtgttaa-3' | 10 |
| Mutant #2 reverse (91 mer) | 3'ITR_91 (□r3M2□) | 5'- ttaacactacggtgtcgggtgaatttcccgccaaaatataagatggcgcgggcaattattt aggtttcccgcgcaaaatcagagttagttt-3' | 16 |
| Mutant #3 (151 mer) | 3'ITR_151 ("3M3") | 5'- cacaagaaaccgaaaatttcatttcaatcgaacccatacttcaaaagatataggcattttaa actaactctgattttgcgcgggaaacctaaataattgcccgcgccatcttatattttggcgg gaaattcacccgacaccgtagtgttaa-3' | 11 |
| Mutant #3 reverse (151 mer) | 3'ITR_151 ("r3M3") | 5'- ttaacactacggtgtcgggtgaatttcccgccaaaatataagatggcgcgggcaattattt aggtttcccgcgcaaaatcagagttagtttaaaatgcctatatcttttgaagtatgggttcga ttgaaatgaaattttcggtttcttgtgt-3' | 17 |
| Mutant #4 (37 mer) | 3'ITR_37 ("3M4") | 5'-attttggcgggaaattcacccgacaccgtagtgttaa-3' | 12 |

Plasmids for a total of 16 types of pBat transposon mutant forms were produced as shown in Table 12 by combining the above-described 5' ITR and 3' ITR. Each mutant gene was produced by requesting Genscript to synthesize the same. Here, for cloning of a mutant gene into a transposon original plasmid, in a transposon vector, a BamHI enzyme site was inserted in front of the 5' ITR, and a SalI enzyme site was inserted behind the 3' ITR. In addition, a 5' ITR mutant was manufactured by inserting BamHI and BspQI enzyme sites at both sides, and a 3' ITR mutant was manufactured by inserting BmtI and SalI enzyme sites.

However, while the 3' ITR mutants had to be cloned with sense sequences of SEQ ID NOs: 3M1, 3M2, and 3M3, the 5' to 3' direction of an antisense strand sequence (that is, a reverse complement sequence) of the 3' ITR was cloned in the 5' to 3' direction of a sense strand of the transposon, thereby producing a plasmid starting with the 5'-ttaa-3' sequence at the 5' end of the 3' ITR. In addition, in the case of a 5M1 mutant form, cloning was not possible due to the small DNA size. Accordingly, plasmids containing a 5m1 mutant could not be cloned, and in the case of the 3' ITR mutant, cloning was performed with plasmids containing a reverse sequence, and indicated with 'r' in the name.

**[Table 12]**

| **No.** | **Name of Mutant form** | **3' ITR** | **5' ITR** |
|---|---|---|---|
| 1 | B3IS-BSIE | Full (SalI insertion) | Full (BamHI insertion) |
| 2 | 83IS-5M2 | Full (SalI insertion) | 5' ITR mutant #2 |
| 3 | 83IS-5M3 | Full (SalI insertion) | 5' ITR mutant #3 |
| 4 | B3IS-5M4 | Full (SalI insertion) | 5' ITR mutant #4 |
| 5 | r3M1-B5IE | 3' ITR mutant #1 (reverse) | Full (BamHI insertion) |
| 6 | r3M1-5M2 | 3' ITR mutant #1 (reverse) | 5' ITR mutant #2 |
| 7 | r3M1-SM3 | 3' ITR mutant #1 (reverse) | 5' ITR mutant #3 |
| 8 | r3M1-SM4 | 3' ITR mutant #1 (reverse) | 5' ITR mutant #4 |
| 9 | r3M2-B5IE | 3' ITR mutant #2 (reverse) | Full (BamHI insertion) |
| 10 | r3M2-5M2 | 3' ITR mutant #2 (reverse) | 5' ITR mutant #2 |
| 11 | r3M2-5M3 | 3' ITR mutant #2 (reverse) | 5' ITR mutant #3 |
| 12 | r3M2-5M4 | 3' ITR mutant #2 (reverse) | 5' ITR mutant #4 |
| 13 | r3M3-B5IE | 3' ITR mutant #3 (reverse) | Full (BamHI insertion) |
| 14 | r3M3-5M2 | 3' ITR mutant #3 (reverse) | 5' ITR mutant #2 |
| 15 | r3M3-5M3 | 3' ITR mutant #3 (reverse) | 5' ITR mutant #3 |
| 16 | r3M3-5M4 | 3' ITR mutant #3 (reverse) | 5' ITR mutant #4 |

### 2. Observation of GFP expression in Jurkat cells7 days after transfection

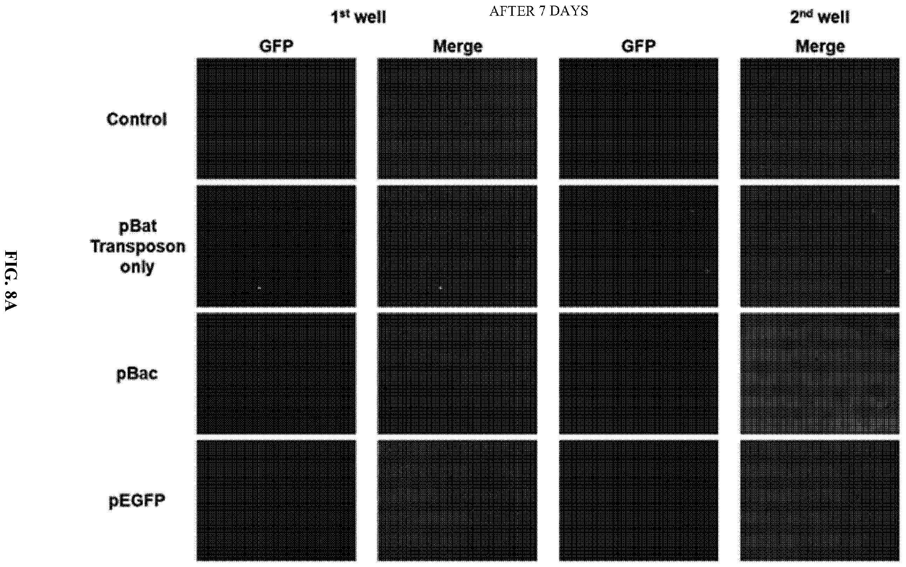

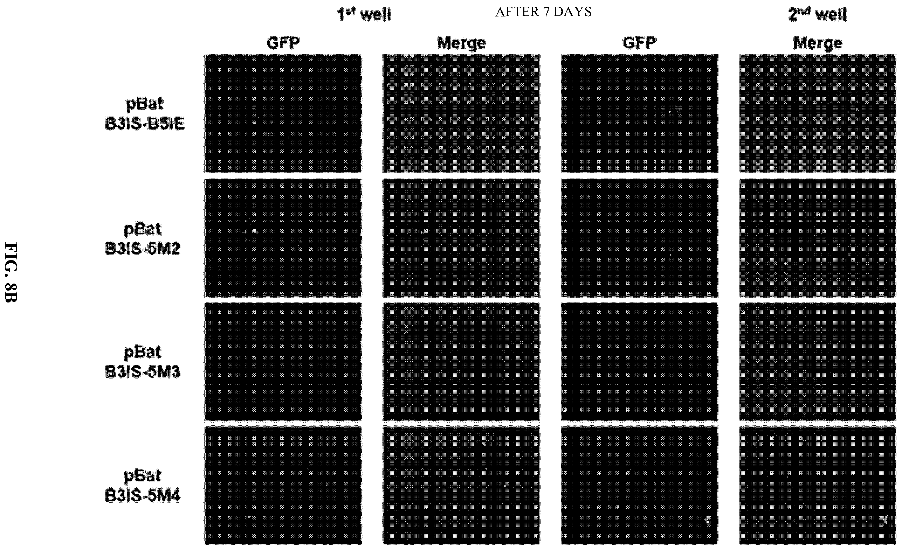

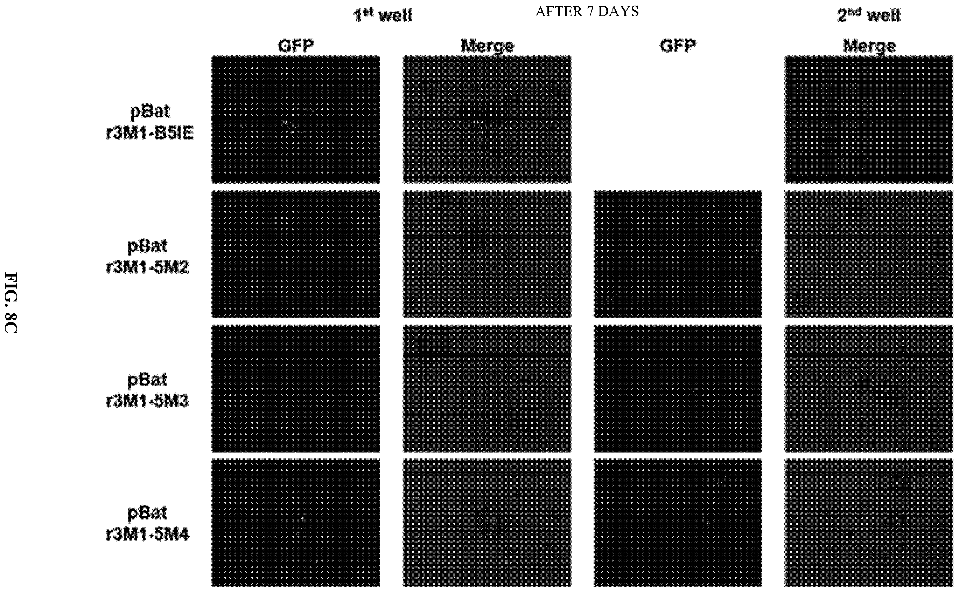

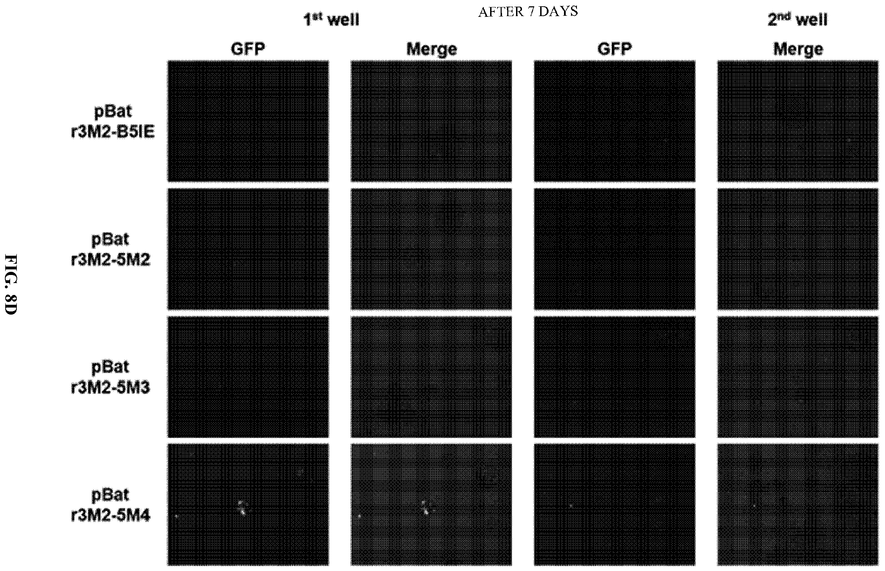

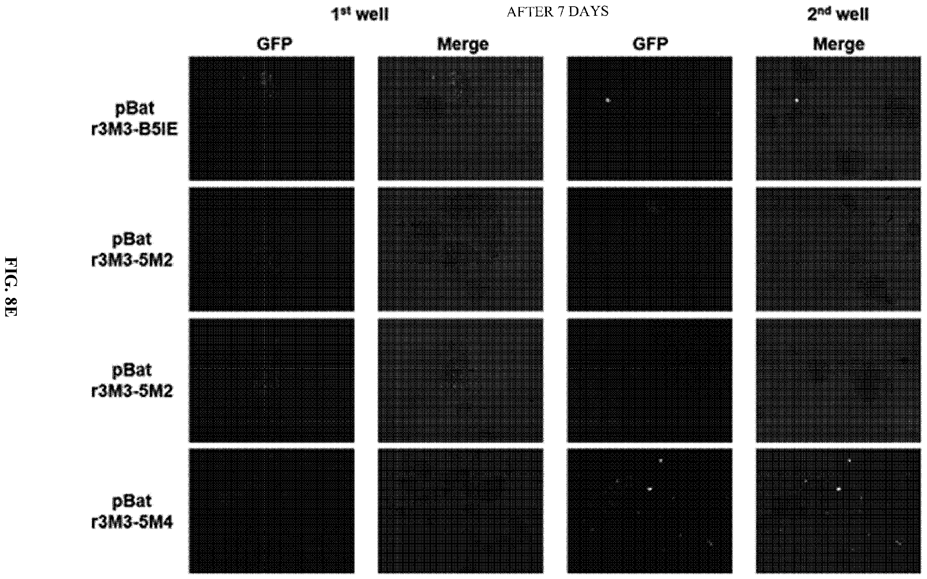

GFP expression in Jurkat cells was observed seven days after transfection using a fluorescence microscope, and analyzed by FACS. As shown in FIG. 8A, in a negative control where only electroporation was performed without a plasmid, GFP expression was not observed, and in a positive control in which electroporation was performed with a GFP-containing plasmid (pEGFP), it was confirmed that GFP was very weakly expressed. GFP expression was very low even in the group in which only a pBat transposon was transfected without a transposase, and GFP expression was not observed in a group (pBac) in which only a transposase is transfected without a piggyBac transposon. On the other hand, as shown in FIGS. 8B to 8E, high levels of GFP expression were confirmed in all groups including the 5M4 mutant form, and GFP expression was observed even with the other transposon mutant forms (reverse).

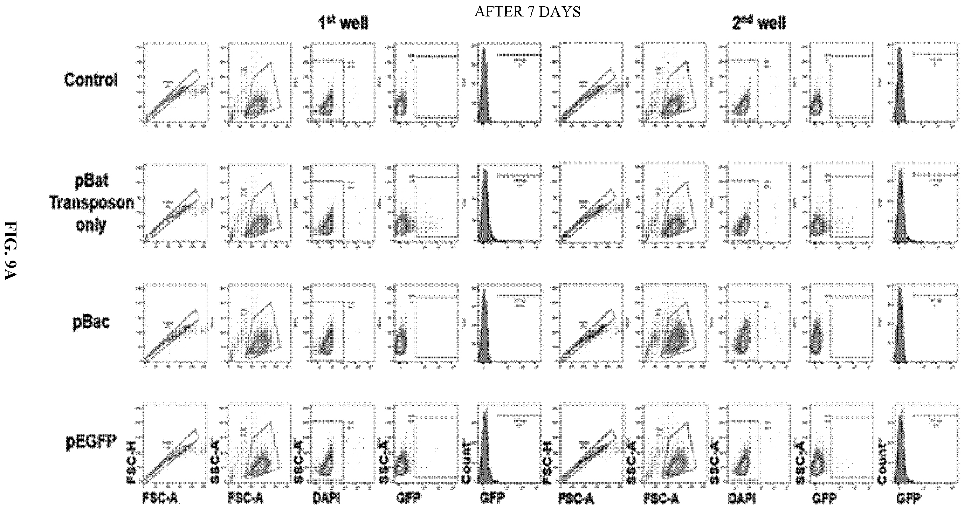

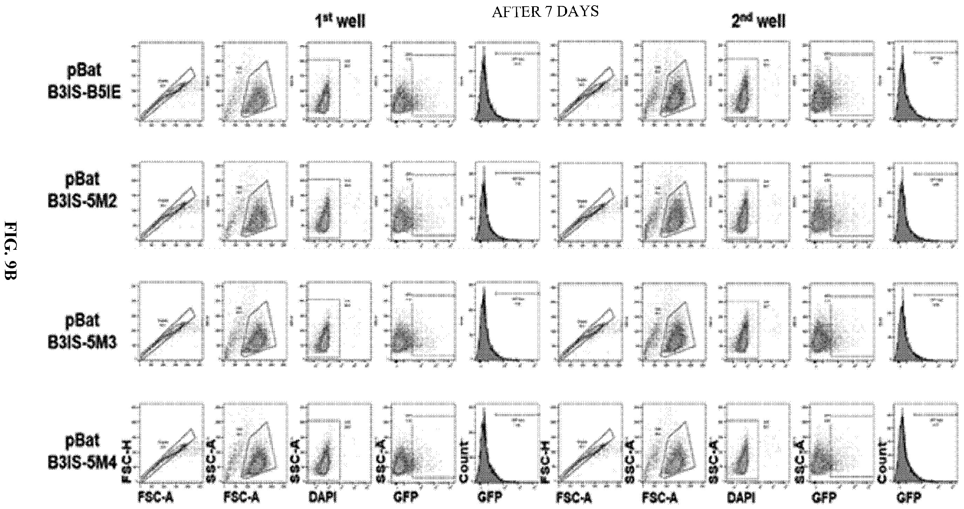

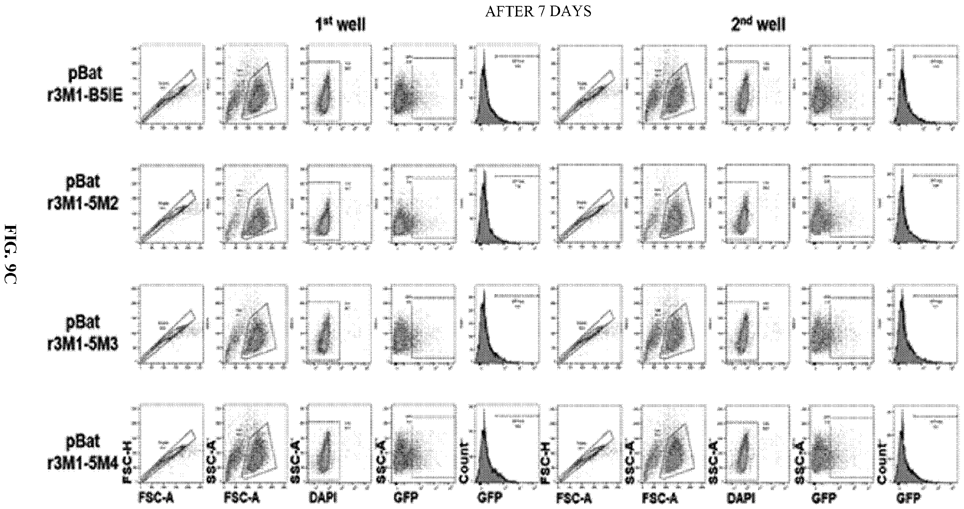

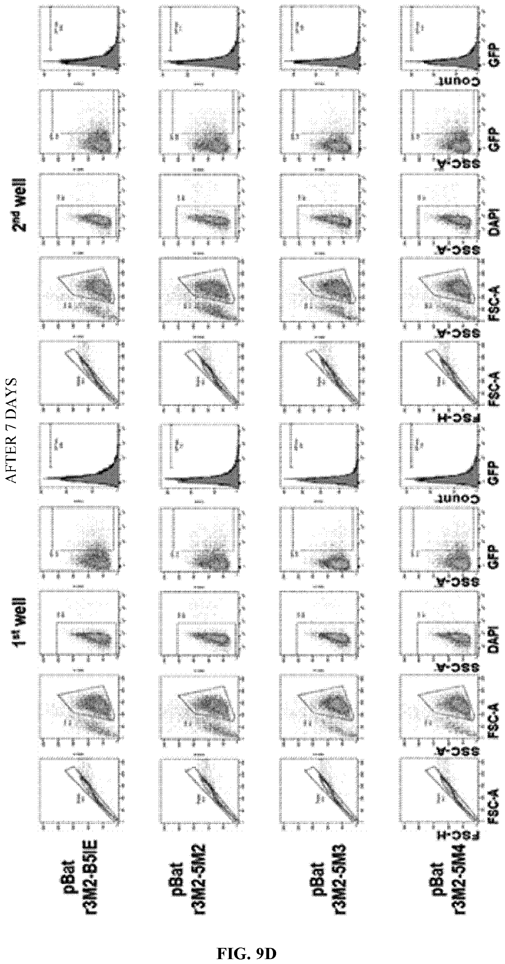

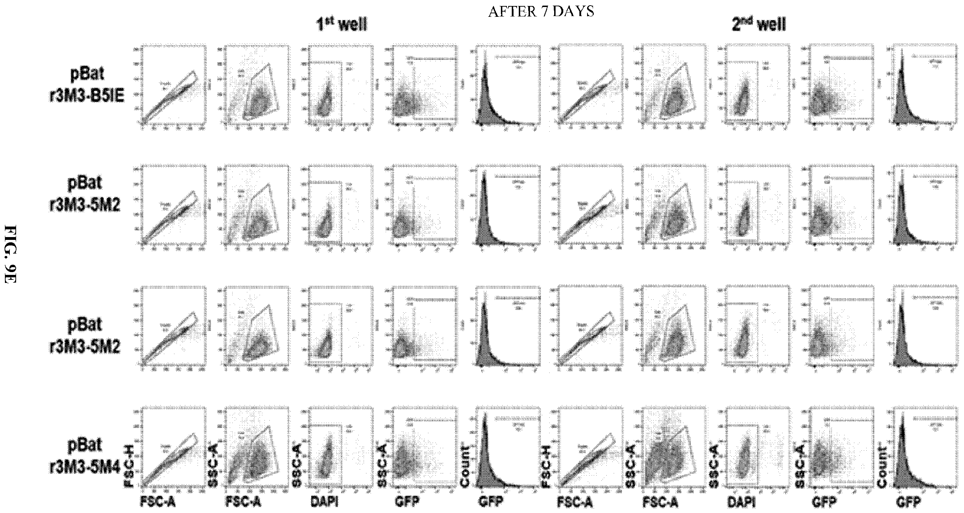

In addition, to confirm the proportions of GFP-expressing Jurkat cells 7 days after transfection, FACS analysis was performed. Analysis was performed by gating in the following order: singlets → cells → live cells → GFP⁺ cells, as shown in FIG. 9A. In addition, the proportion of GFP⁺ cells among live cells was analyzed using a histogram. As a result of the analysis, 7 days after transfection, the proportion of GFP-expressing cells was 0.5% in the pEGFP group, and 1.6% in the group in which only a pBat transposon was transfected, and the proportion of GFP-expressing cells was 0% in the pBac group, confirming that there were almost no stable GFP-expressing cells (FIG. 9A and Table 13). On the other hand, in the group in which a pBat mutant transposon according to the present invention was introduced, it was confirmed that GFP-expressing cells were present even 7 days after transfection (FIGS. 9B to 9E).

### 3. Observation of GFP expression in Jurkat cells 14 days after transfection

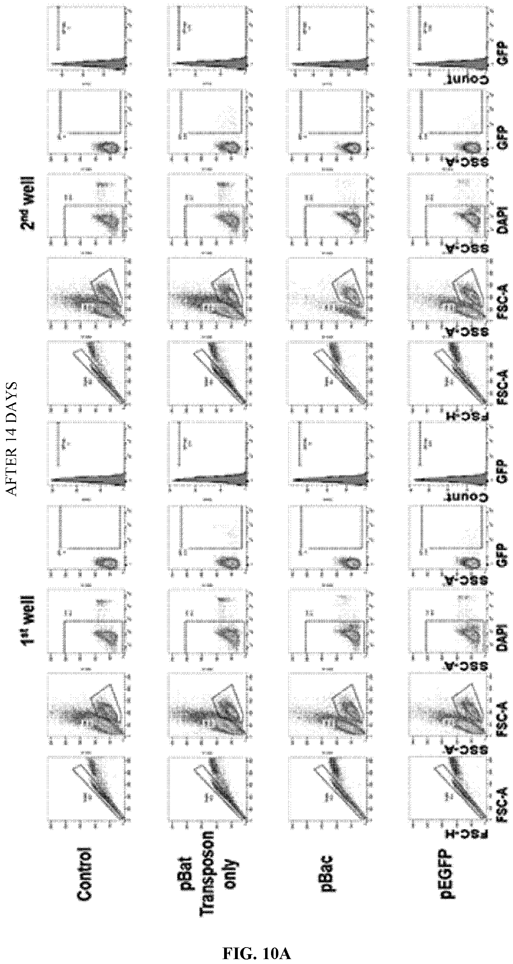

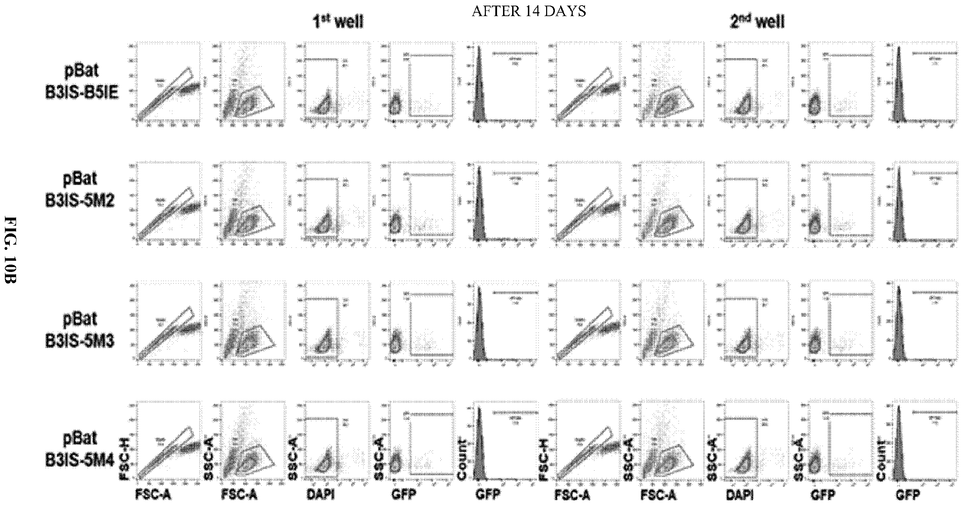

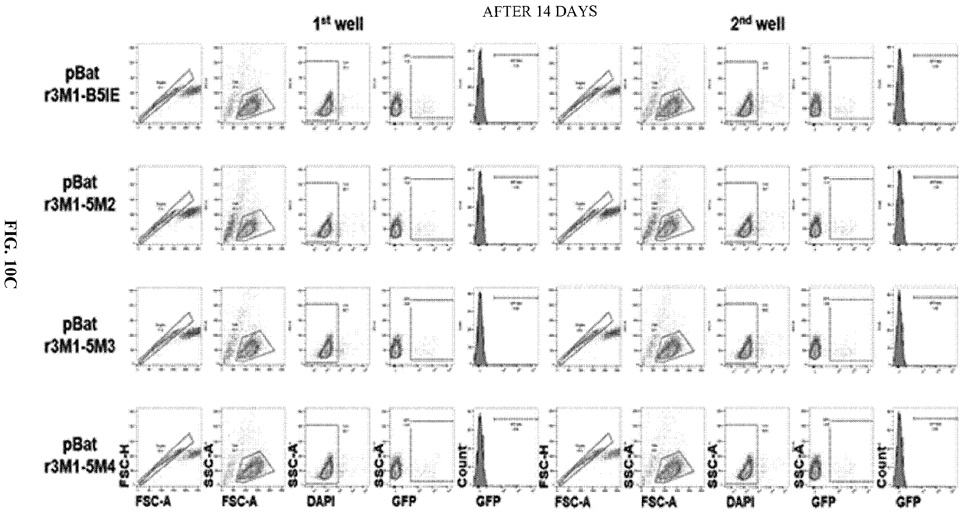

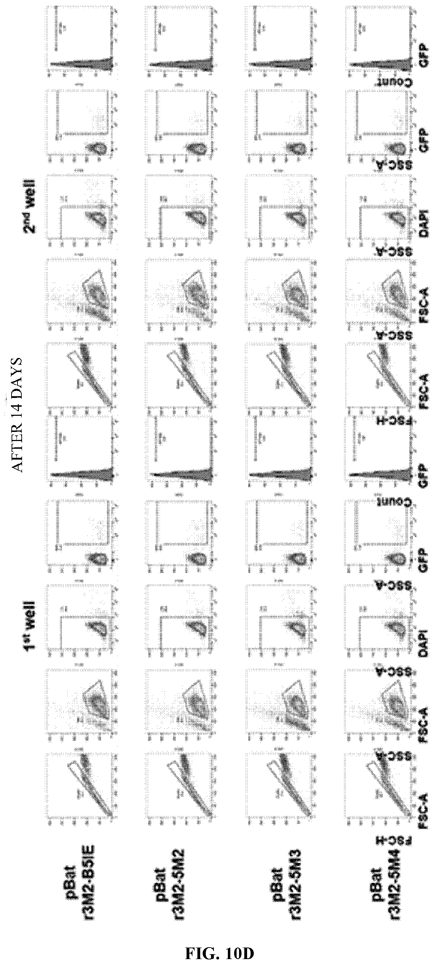

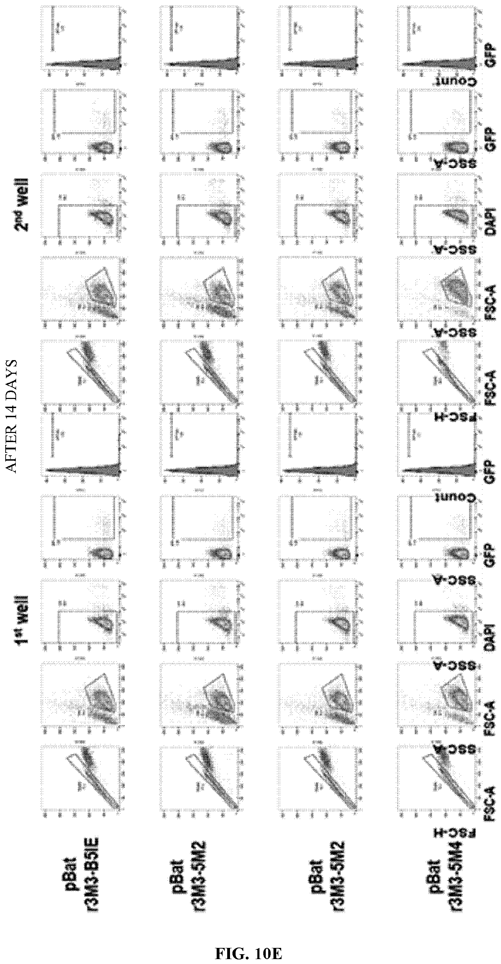

To confirm the proportions of Jurkat cells expressing GFP 14 days after transfection, FACS analysis was performed. As a result of the analysis, 14 days after transfection, the proportion of GFP-expressing cells was 0.4% in the pEGFP group and 0.8% in the group in which only a pBat transposon was transfected, and the proportion of GFP-positive cells in the pBac group was 0%, indicating that GFP-expressing cells hardly appear (FIG. 10A). On the other hand, in the group in which a pBat mutant transposon according to the present invention was introduced, it was confirmed that GFP-expressing cells were present even 14 days after transfection (FIGS. 10B to 10E).

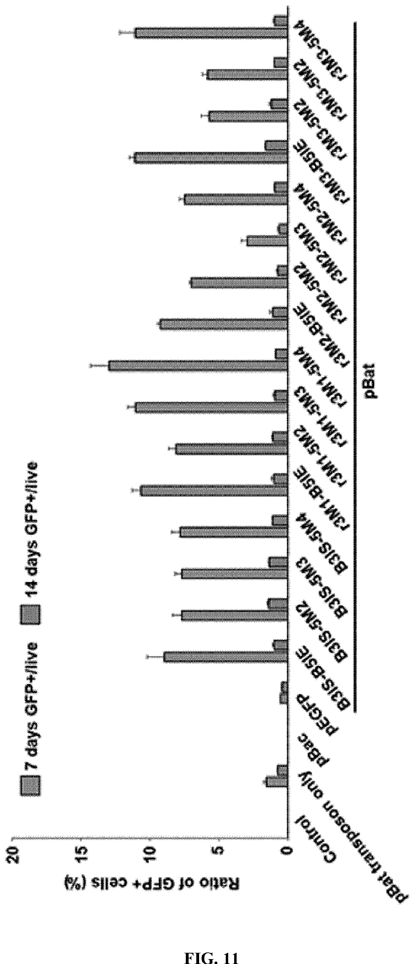

By combining the above results, the results of confirming the proportions of GFP-expressing cells by group over time after transfection are shown in Table 13 and FIG. 11.

**[Table 13]**

| **Group** | **Day 2** | **Day 7** | **Day 14** |
|---|---|---|---|
| | **GFP+/live** | **GFP+/live** | **GFP+/live** |
| control | 0.0 | 0.0 | 0.0 |
| pBat transposon only | 30.6 | 1.6 | 0.8 |
| pBac | 0.0 | 0.0 | 0.0 |
| pEGFP | 40.7 | 0.5 | 0.4 |
| B3IS-B5IE | 49.0 | 9.0 | 1.0 |
| B3IS-SM2 | 51.5 | 7.7 | 1.4 |
| B3IS-SM3 | 51.4 | 7.7 | 1.4 |
| B3IS-SM4 | 51.9 | 7.8 | 1.1 |
| r3M1-B5IE | 49.2 | 10.6 | 1.0 |
| r3M1-5M2 | 53.9 | 8.1 | 1.1 |
| r3M1-5M3 | 55.9 | 11.1 | 1.0 |
| r3M1-SM4 | 54.4 | 13.0 | 0.9 |
| r3M2-B5IE | 50.7 | 9.2 | 1.1 |
| r3M2-5M2 | 43.1 | 7.0 | 0.7 |
| r3M2-5M3 | 34.2 | 2.9 | 0.6 |
| r3M2-5M4 | 43.5 | 7.5 | 1.0 |
| r3M3-B5IE | 53.2 | 11.1 | 1.6 |
| r3M3-5M2 | 41.8 | 5.7 | 1.2 |
| r3M3-5M2 | 42.5 | 5.8 | 1.0 |
| r3M3-5M4 | 44.9 | 11.1 | 1.0 |

### 4. Confirmation of GFP expression after single cell sorting and culture

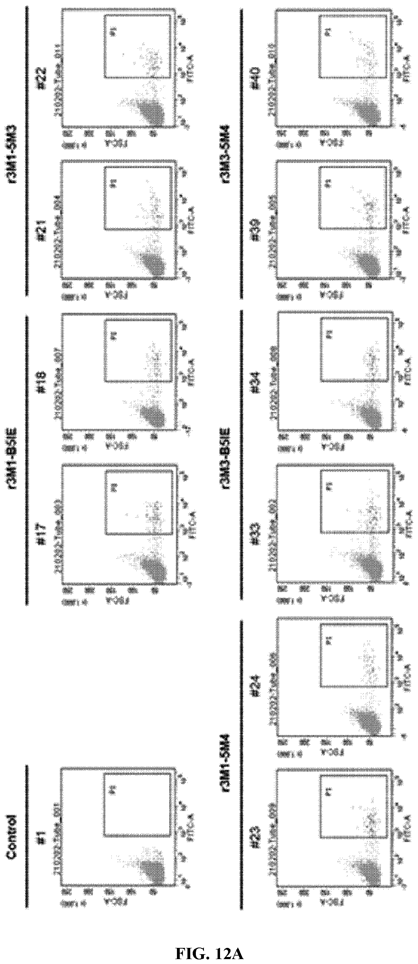

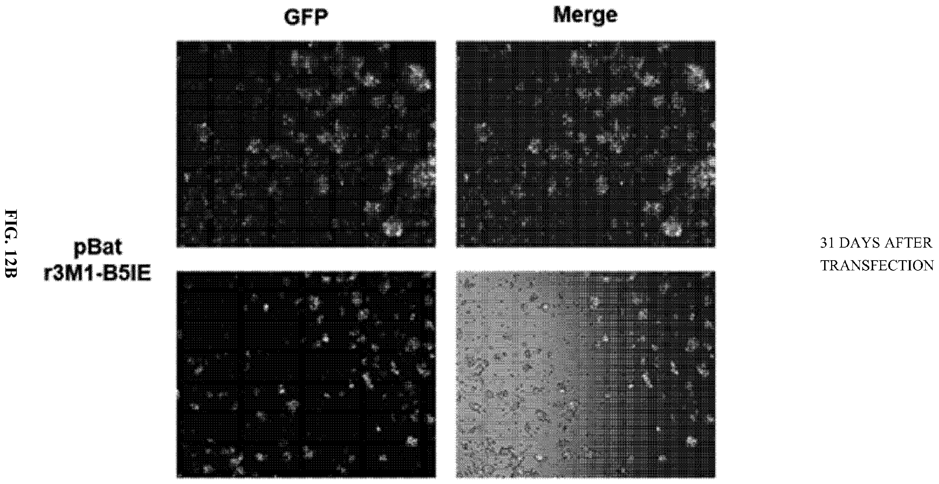

Fourteen days after transfection, in r3M1-BSIE-, r3M1-5M3-, r3M1-5M4-, r3M3-B5IE-, and r3M3-M4-treated groups, GFP-expressing Jurkat cells were each transferred to a 96 well plate, followed by single cell sorting (FIG. 12A). Subsequently, after further culturing the cells sorted into single cells for 14 days, GFP expression was observed. As a result, it was confirmed that, in the cells in the r3M1-B5IE group, GFP was stably expressed even 31 days after transfection with the transposon (FIG. 12B).

In this experimental example, transposon vectors having excellent chromosomal integration efficiency and excellent gene expression efficiency were produced based on the transposons discovered through Experimental Example A. To this end, four 5' ITR mutants and four 3' ITR mutants were produced by modifying the 5' ITR and 3' ITR of the transposon, respectively. However, in the case of the 3' ITR mutants, the 5' to 3' direction of the sequence of an antisense strand, not a sense strand, of the 3' ITR was cloned in the 5' to 3' direction of the sense strand of the transposon, thereby producing a plasmid starting with the 5'-ttaa-3' sequence at the 5' end of the 3' ITR (indicated with 'r' in the name). A total of 16 types of transposon vectors were produced by combining 5' ITR mutants and 3' ITR mutants, and their gene transfer efficiency was evaluated.

As shown in the experimental results, from day 7 after transfection of a transposon vector, GFP expression was hardly confirmed in the control group. On the other hand, in the cells in which the transposon mutants and transposase according to the present invention were introduced, it was confirmed that the GFP gene was inserted into the chromosome of the Jurkat cell, thereby stably expressing GFP, and particularly, it was confirmed that the cells into which a transposon vector containing 5M4 was introduced exhibited relatively high GFP expression.

In addition, it was confirmed that the cells into which a transposon vector according to the present invention was introduced maintained GFP expression even 14 days after transfection, and particularly, the cells transfected with a vector including the 3' ITR sequence in a reverse direction also stably expressed GFP.

The above results show that the transposon vectors according to the present invention can induce stable expression by introducing a target gene into the cell chromosome.

### Experimental Example C. Production of ITR mutant and identification of its function

In this experimental example, as in Experimental Example B, mutants of the transposons discovered by the present inventors were produced to produce mutants having more improved chromosomal integration efficiency and gene expression efficiency. Particularly, in this experimental example, unlike Experimental Example B, a mutant vector was produced by cloning a 3' ITR mutant with a sense strand sequence.

### 1. Production of mutant forms for ITR

Mutant forms were designed overall in the same manner as in Experimental Example B (FIGS. 13A and 13B). Based on the result of analyzing the alignment of the ITR sequences of pBat and piggyBac, four types of 5' ITR mutants and four types of 3' ITR mutants were produced.

The sequences of the four types of 5' ITR mutants are the same as shown in Table 10 (SM1, 5M2, 5M3, and 5M4), and the sequences of the four types of 3'ITR mutants are the same as shown in Table 11 (3M1, 3M2, 3M3, and 3M4).

By combining the 5' ITR mutants and the 3' ITR mutants, a total of 26 types of pBat transposon mutant forms were produced (Table 14). To this end, the gene of each mutant form was synthesized by Genscript, and to clone an ITR mutant into a transposon original plasmid, in a transposon vector, a BamHI site was inserted in front of the 5' ITR, and a SalI site was inserted after the 3' ITR. In addition, a 5' ITR mutant was produced using BamHI and EcoRV, and a 3' ITR mutant was produced using BmtI and Sall restriction enzymes.

In addition, in this experimental example, unlike Experimental Example B cloned with antisense sequences of the 3' ITR mutants, cloning was carried out with sense sequences of the 3' ITR mutants (3M1, 3M2, 3M3, and 3M4).

**[Table 14]**

| **No.** | **Name of mutant form** | **3' ITR** | **5' ITR** |
|---|---|---|---|
| 1 | Original | Full | Full |
| 2 | B3IS-B5IE | Full (SalI insertion) | Full (BamHI insertion) |
| 3 | B3IS-5M1 | Full (SalI insertion) | 5' ITR mutant #1 |
| 4 | B3IS-5M2 | Full (SalI insertion) | 5' ITR mutant #2 |
| 5 | B3IS-5M3 | Full (SalI insertion) | 5' ITR mutant #3 |
| 6 | B3IS-5M4 | Full (SalI insertion) | 5' ITR mutant #4 |
| 7 | 3M1-B5IE | 3' ITR mutant #1 | Full (BamHI insertion) |
| 8 | 3M1-5M1 | 3' ITR mutant #1 | 5' ITR mutant #1 |
| 9 | 3M1-5M2 | 3' ITR mutant #1 | 5' ITR mutant #2 |
| 10 | 3M1-5M3 | 3' ITR mutant #1 | 5' ITR mutant #3 |
| 11 | 3M1-5M4 | 3' ITR mutant #1 | 5' ITR mutant #4 |
| 12 | 3M2-B5IE | 3' ITR mutant #2 | Full (BamHI insertion) |
| 13 | 3M2-5M1 | 3' ITR mutant #2 | 5' ITR mutant #1 |
| 14 | 3M2-5M2 | 3' ITR mutant #2 | 5' ITR mutant #2 |
| 15 | 3M2-5M3 | 3' ITR mutant #2 | 5' ITR mutant #3 |
| 16 | 3M2-5M4 | 3' ITR mutant #2 | 5' ITR mutant #4 |
| 17 | 3M3-B5IE | 3' ITR mutant #3 | Full (BamHI insertion) |
| 18 | 3M3-5M1 | 3' ITR mutant #3 | 5' ITR mutant #1 |
| 19 | 3M3-5M2 | 3' ITR mutant #3 | 5' ITR mutant #2 |
| 20 | 3M3-5M3 | 3' ITR mutant #3 | 5' ITR mutant #3 |
| 21 | 3M3-5M4 | 3' ITR mutant #3 | 5' ITR mutant #4 |
| 22 | 3M4-B5IE | 3' ITR mutant #4 | Full (BamHI insertion) |
| 23 | 3M4-5M1 | 3' ITR mutant #4 | 5' ITR mutant #1 |
| 24 | 3M4-5M2 | 3' ITR mutant #4 | 5' ITR mutant #2 |
| 25 | 3M4-5M3 | 3' ITR mutant #4 | 5' ITR mutant #3 |
| 26 | 3M4-5M4 | 3' ITR mutant #4 | 5' ITR mutant #4 |

### 2. Observation of GFP expression in Jurkat cells 7 days after transfection

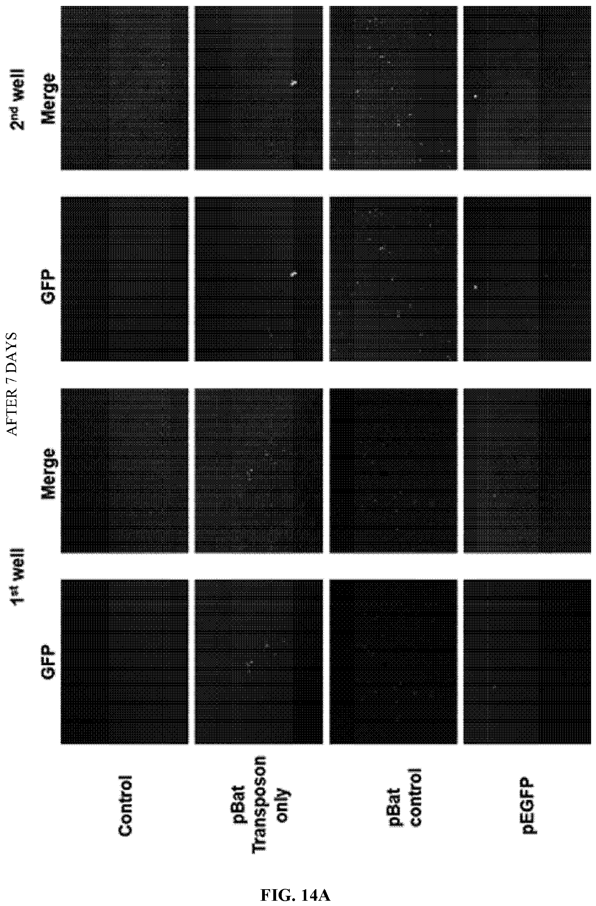

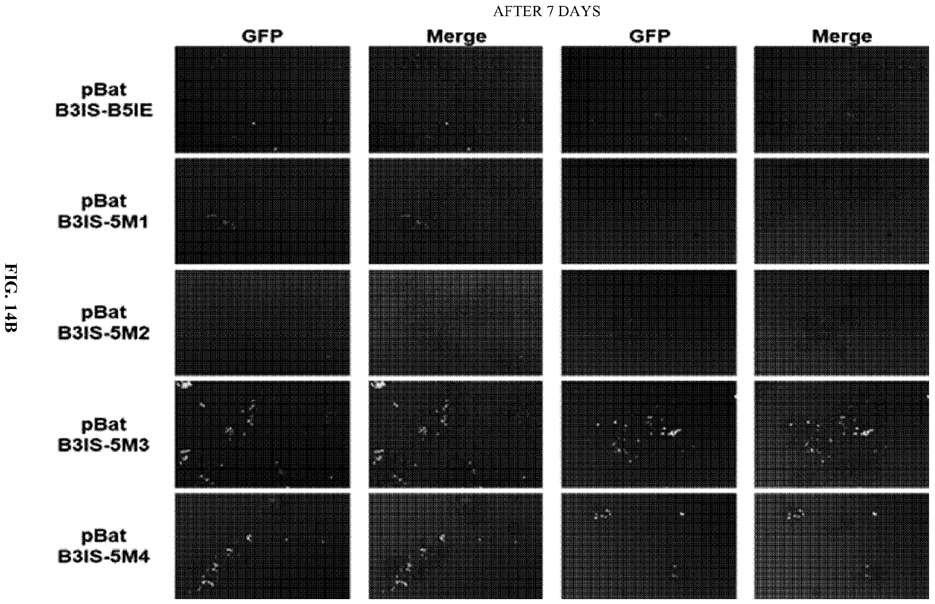

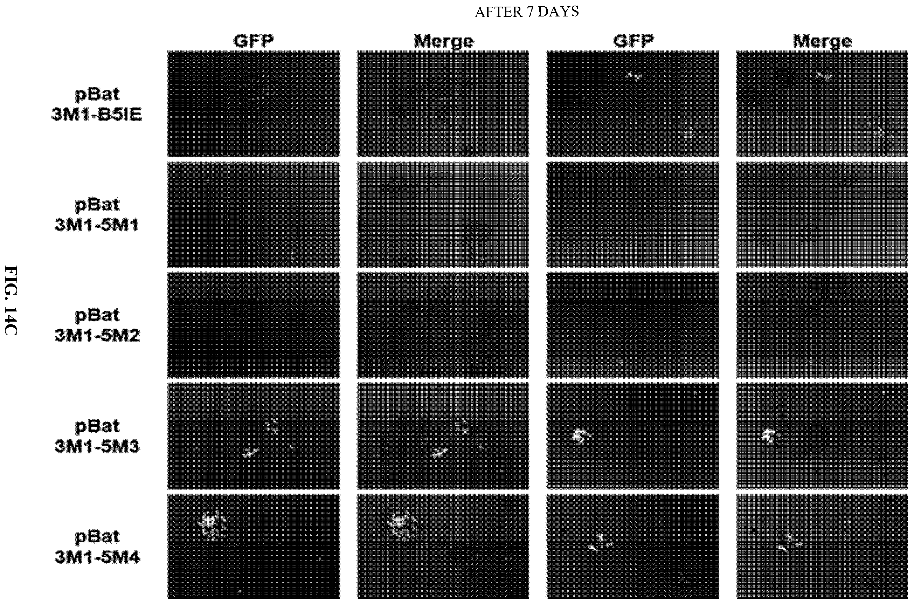

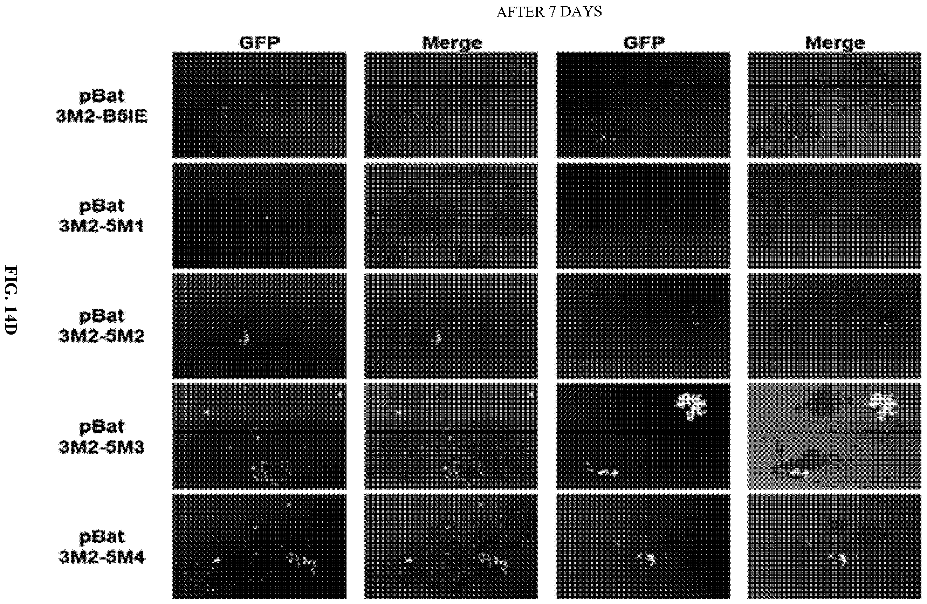

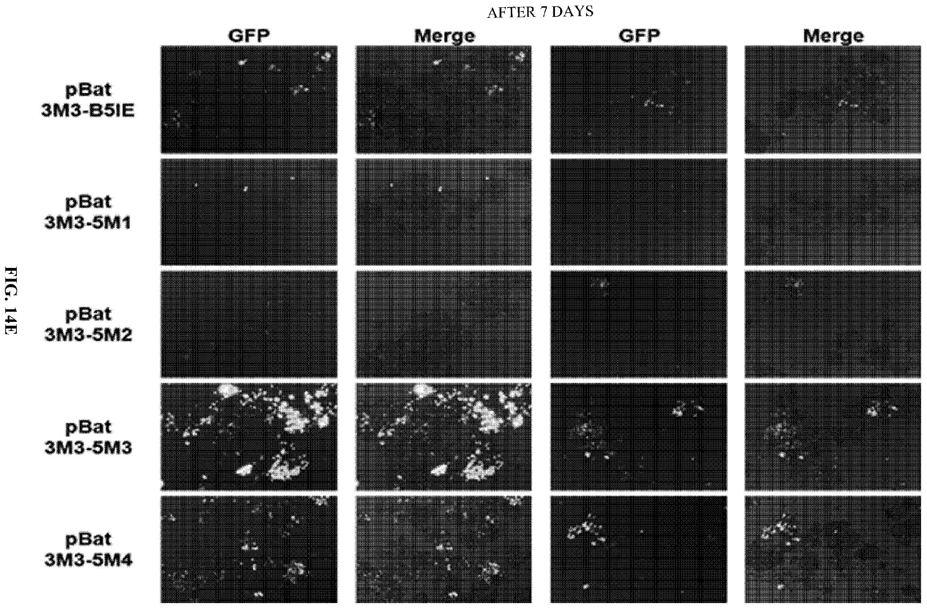

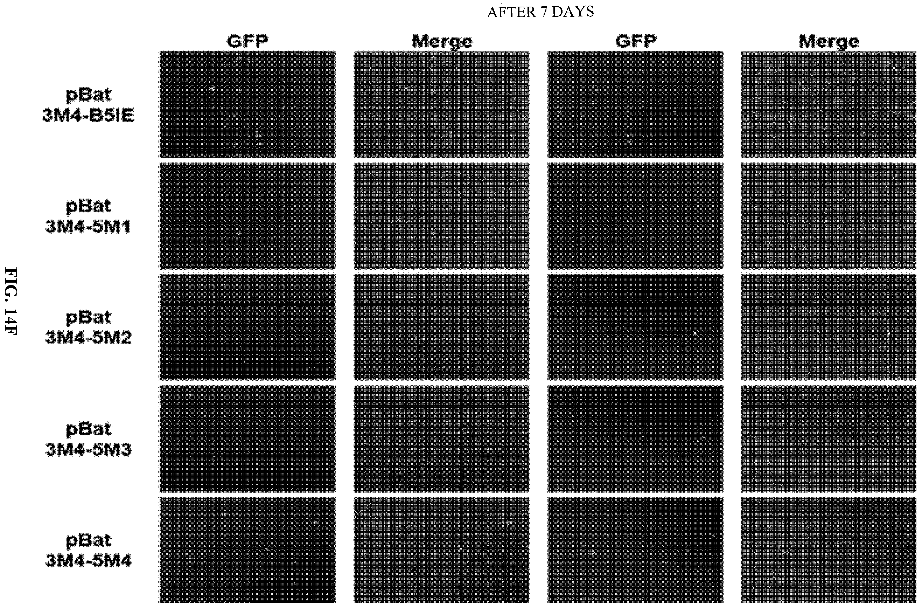

The produced transposon vector was introduced into Jurkat cells through electroporation (Neon transfection). Seven days after electroporation, to confirm the gene delivery efficiency of a transposon, GFP expression in Jurkat cells was observed using a fluorescence microscope, and analyzed by FACS. As a result of the analysis, as shown in FIG. 14A, it was confirmed that no GFP expression was observed in the negative control (Control) in which only electroporation was performed without a plasmid, and GFP expression was weakly exhibited in the positive control in which electroporation was performed with a GFP-expressing plasmid (pEGFP). It was observed that even in the group in which only a pBat transposon was transfected without a transposase, GFP expression was very low, and even in the group (pBat control) in which a transposase and intact pBat (original) were co-electroporated, GFP expression was low. On the other hand, in the groups in which a mutant transposon according to the present invention was electroporated, in general, GFP was detected, and particularly, in the groups containing the 5M3 or 5M4 mutant form, high levels of GFP expression were observed (FIGS. 14B to 14F).

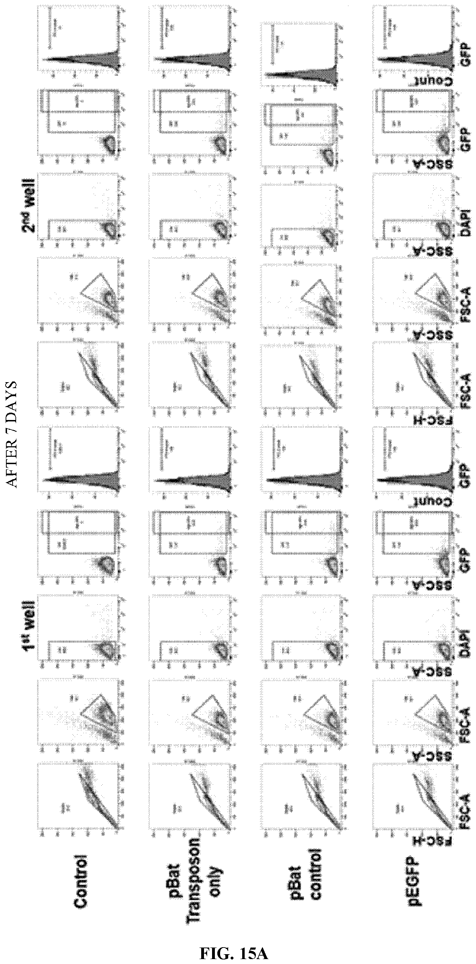

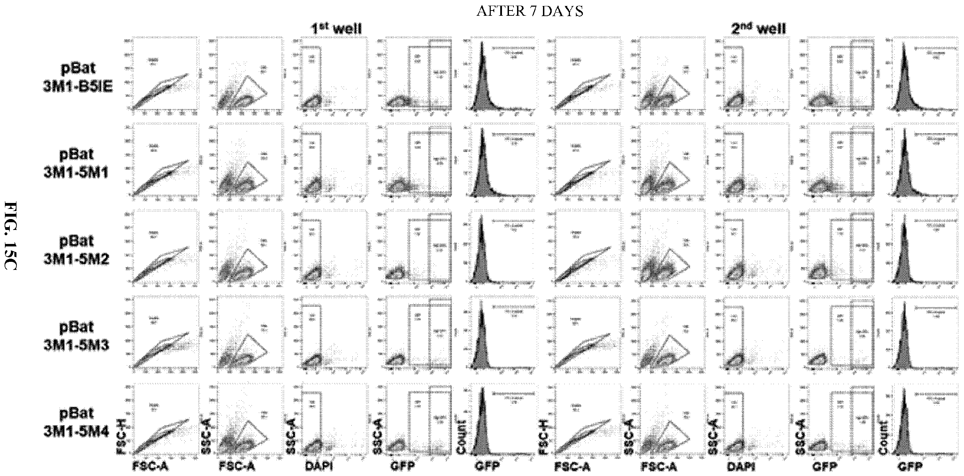

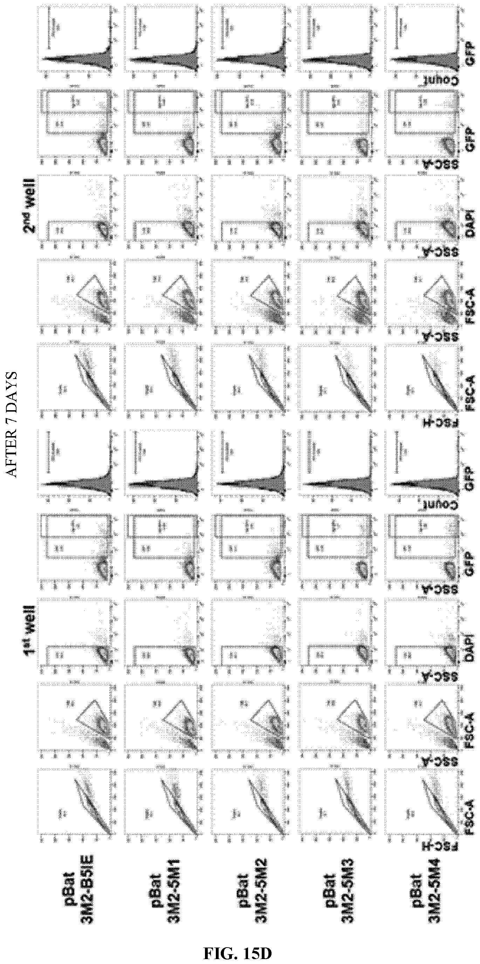

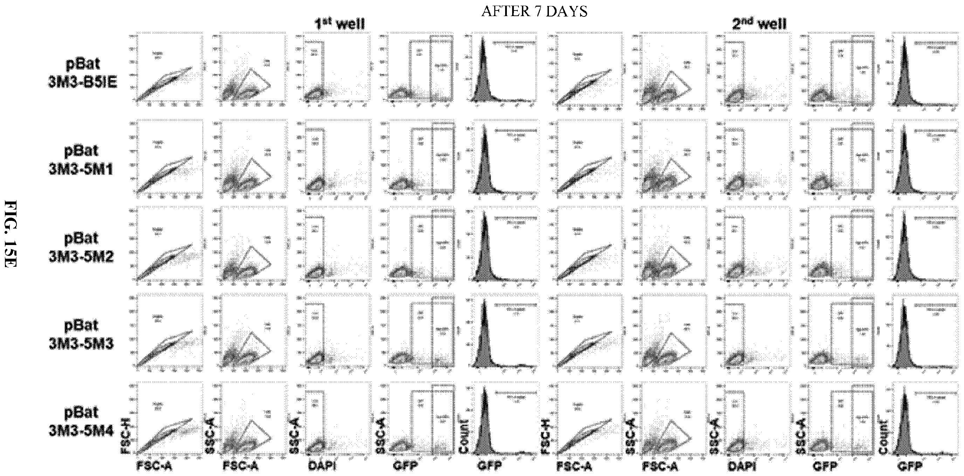

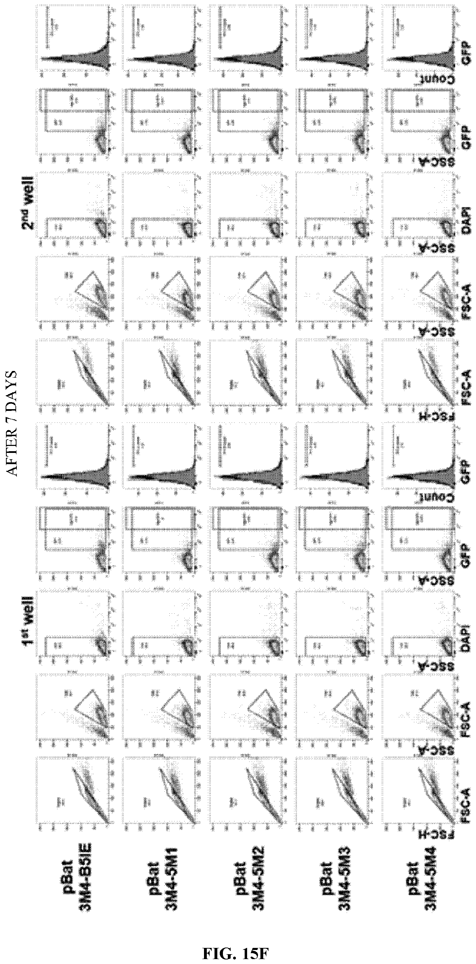

Seven days after electroporation, to confirm the proportion of GFP-expressing Jurkat cells, FACS analysis was performed. As shown in FIG. 15A, analysis was performed by gating in the following order: singlets → cells → live cells → GFP⁺ cells. In addition, the proportion of GFP⁺ cells among live cells was analyzed by a histogram. As a result of the analysis, seven days after transfection, in the pEGFP group, the proportion of GFP-expressing cells was approximately 2%, and in the group in which only a pBat transposon was transfected and the pBat control, the proportions of GFP-expressing cells were only approximately 1% (FIG. 15A).

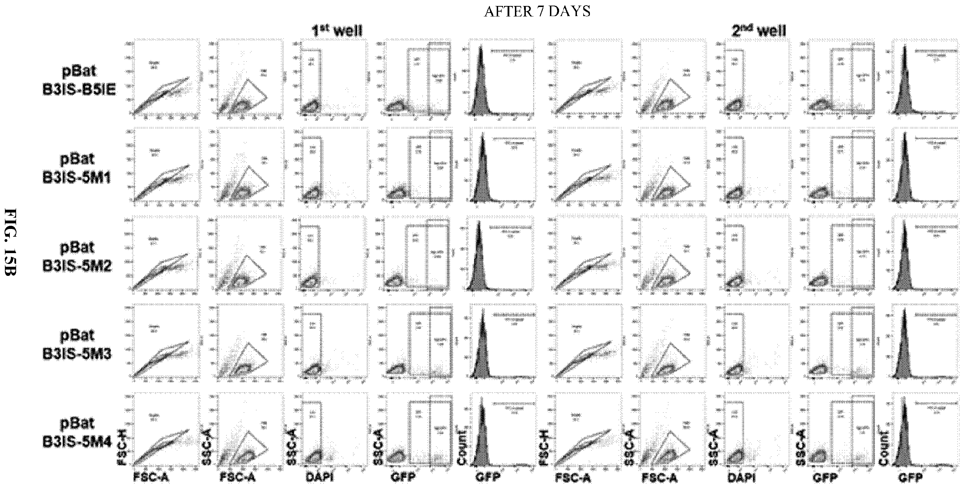

As a result of confirming the proportion of GFP⁺ cells by setting a standard close to GFP⁻ (negative), it was confirmed that the group in which the transposon mutant according to the present invention was introduced generally expressed a high level of GFP (FIGS. 15B to 15F). Particularly, in the group in which a 5M3 or 5M4 mutant-containing transposon was introduced, cell populations expressing GFP at high intensity were present. Accordingly, the proportion of GFP-expressing cells was confirmed by setting a standard close to high intensity. As a result, in the group in which a 5M3 or 5M4 mutant-containing transposon (including B3IS, 3M1, 3M2, or 3M3 as a 3' ITR) was introduced, GFP expression was confirmed, and compared with the group in which a B5IE, 5M1, or 5M2-containing transposon was treated, the proportion of high-intensity GFP⁺ cells was significantly higher.

GFP-expressing cells from the first well sample of each group were sorted into single cells and cultured in a 96-well plate, and proliferated cells were recovered, frozen, and then stored in a liquid nitrogen tank.

### 3. Observation of GFP expression in Jurkat cells 14 days after transfection

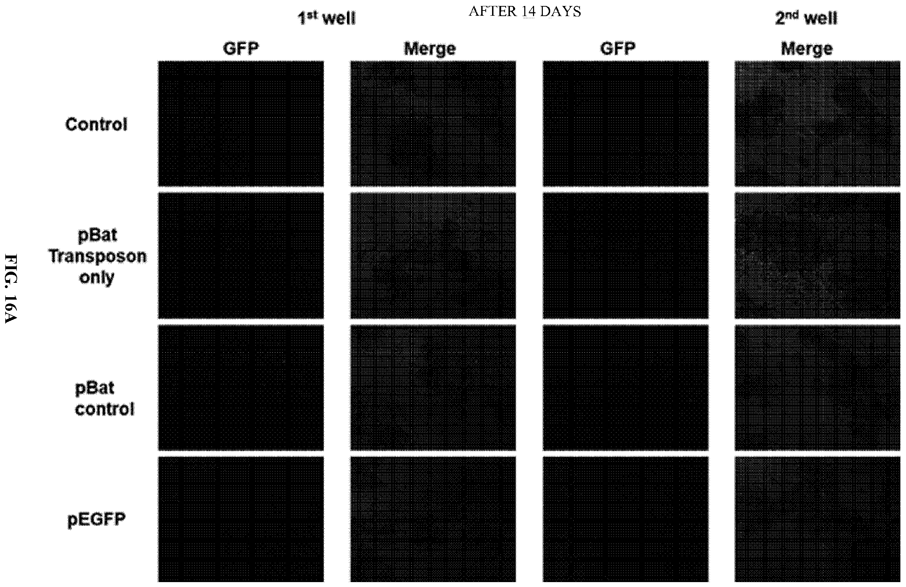

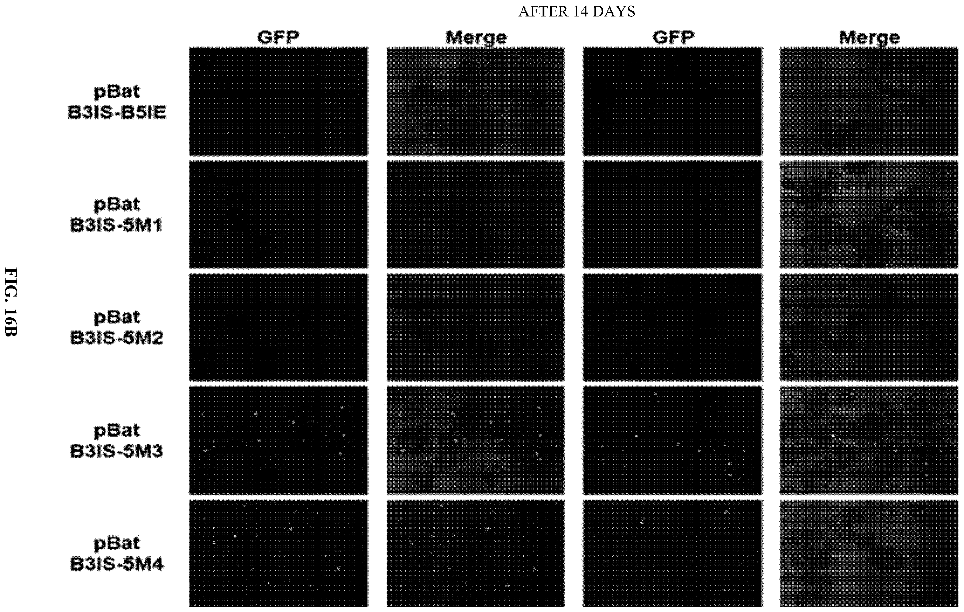

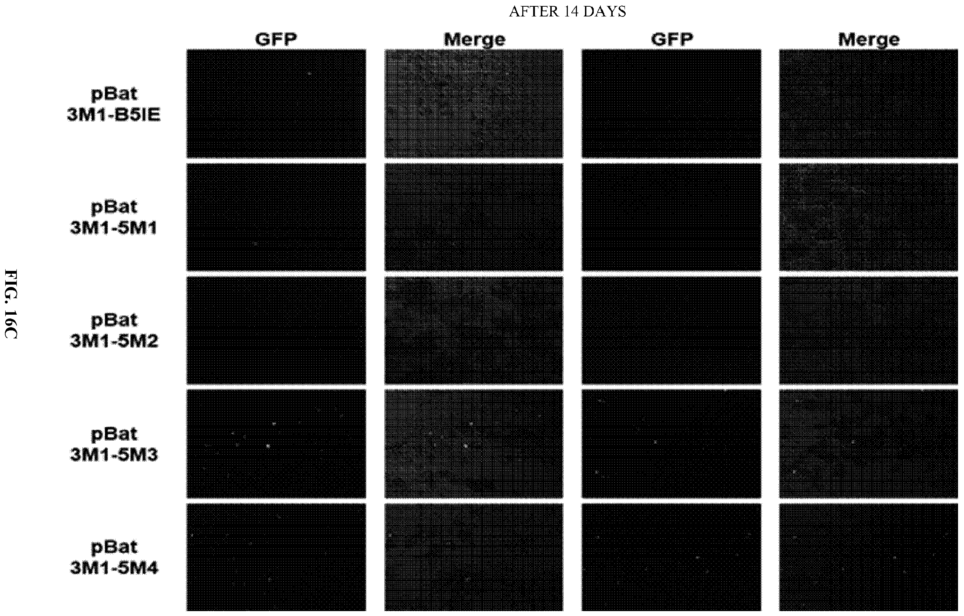

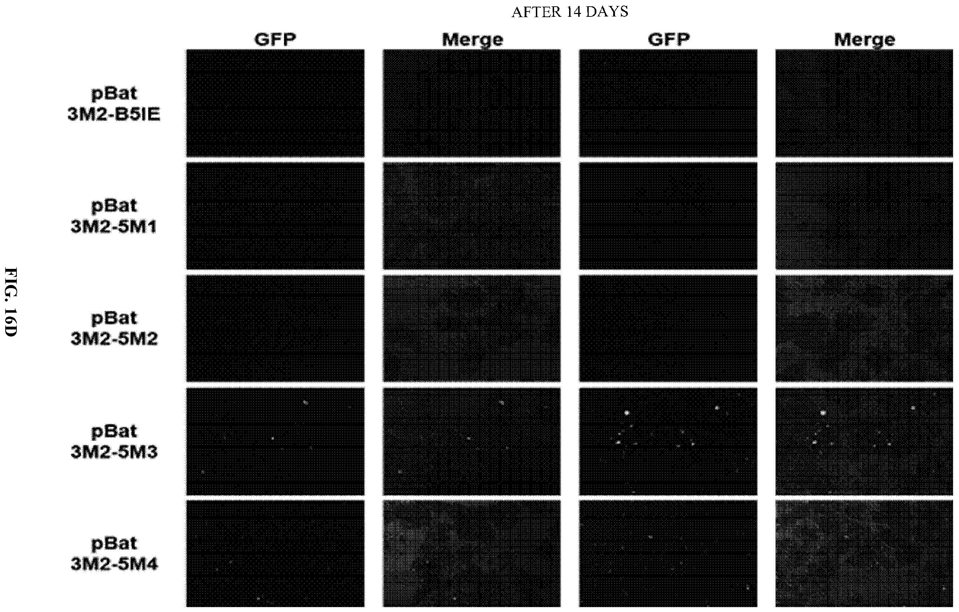

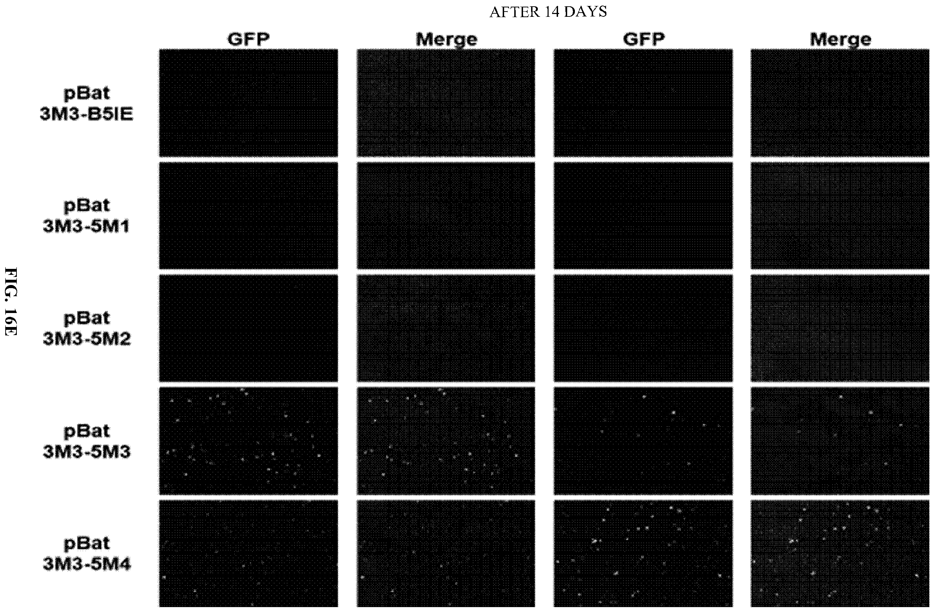

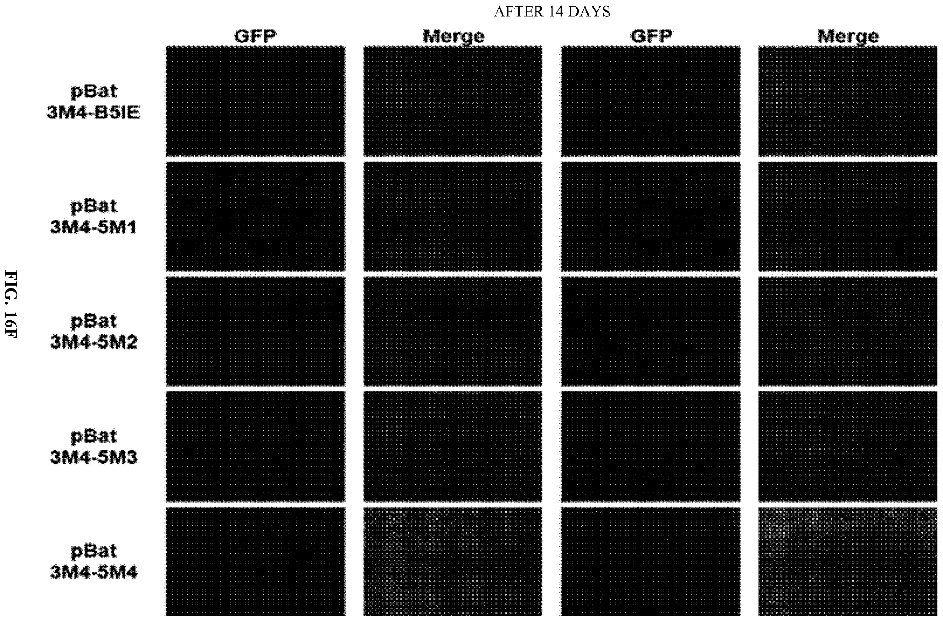

Fourteen days after transfection, GFP expression in Jurkat cells was observed using a fluorescence microscope, and analyzed by FACS. As a result of the analysis, as shown in FIG. 16A, in the negative control in which only electroporation was performed without a plasmid, GFP expression was not observed, and even in the positive control in which electroporation was performed with a GFP-containing plasmid (pEGFP) and the group in which only a pBat transposon was transfected without a transposase, GFP was hardly observed. In the group (pBat control) in which a transposase and intact pBat (original) were co-transfected, GFP was observed at a very low level. On the other hand, it was confirmed that, in the groups in which the transposon mutant according to the present invention was electroporated, generally, GFP was expressed, and particularly, in the groups containing the 5M3 or 5M4 mutant form, high levels of GFP expression were confirmed (FIGS. 16B to 16F).

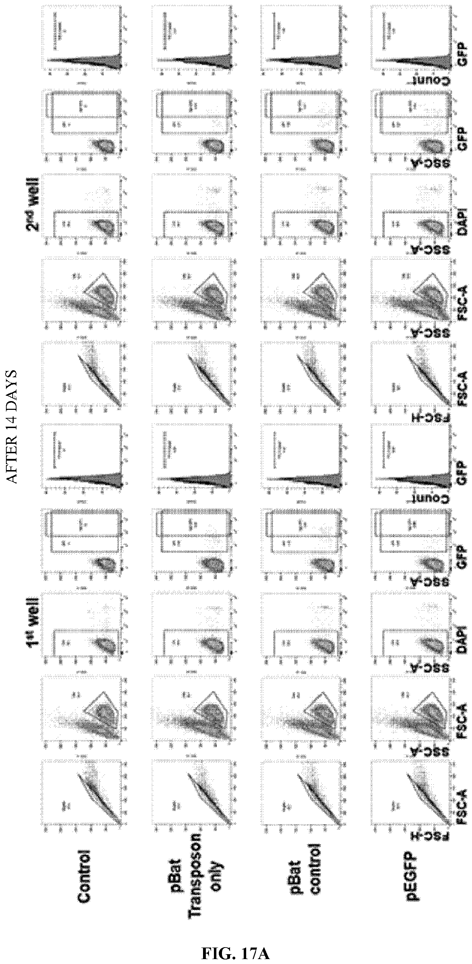

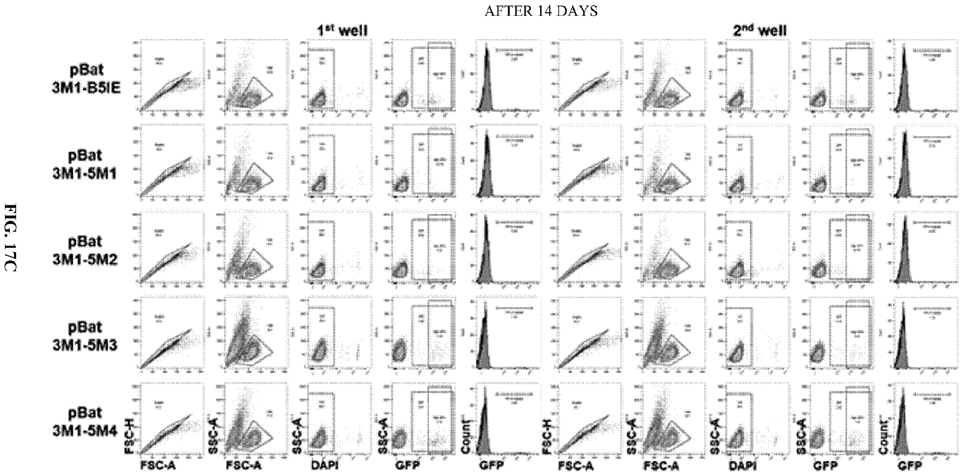

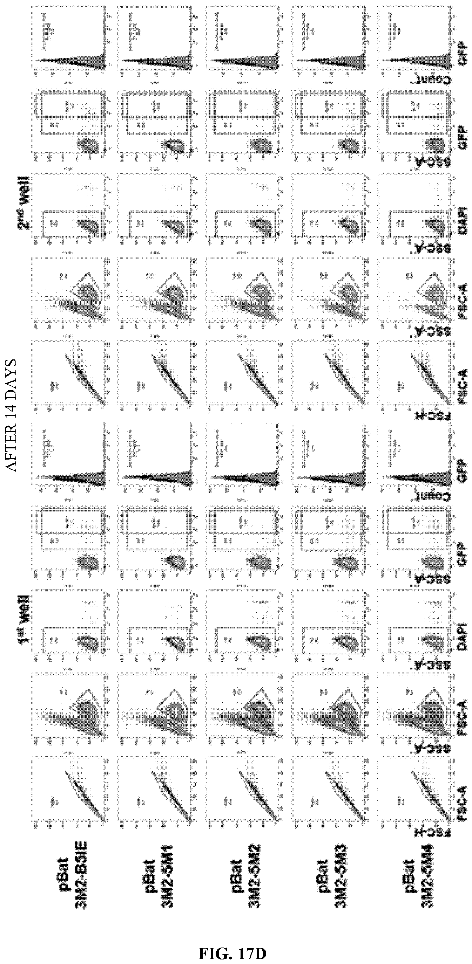

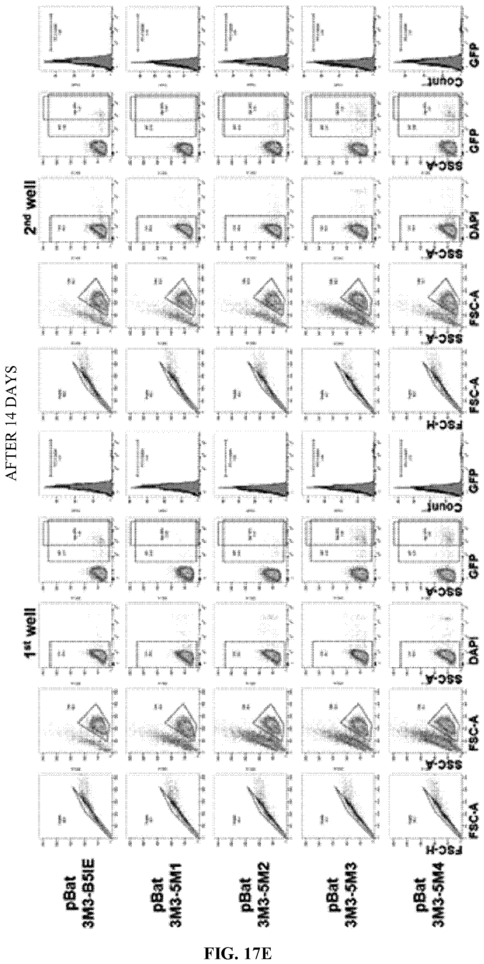

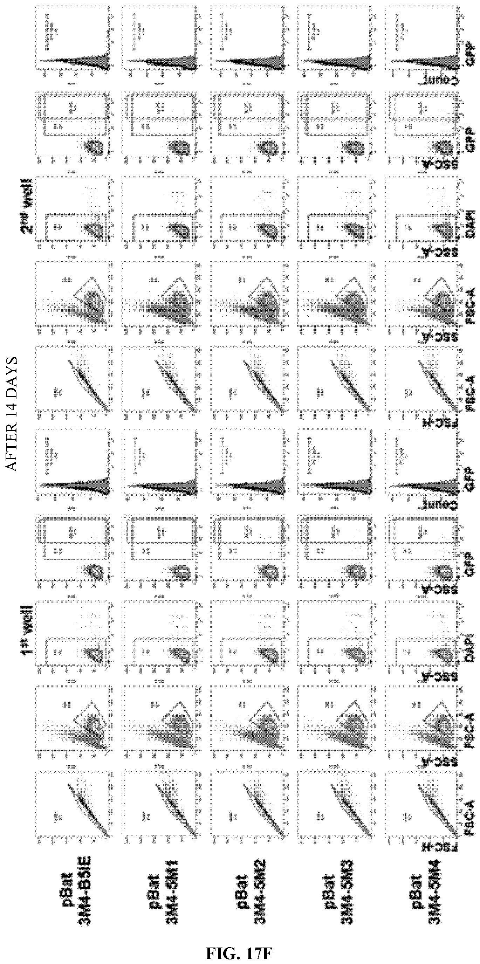

In addition, as a result of performing FACS analysis to confirm the proportion of GFP-expressing Jurkat cells, in all of the pEGFP group, the group in which only a pBat transposon was transfected, and the pBat control, the proportions of GFP-expressing cells were only approximately 1%, confirming that there were almost no GFP-expressing cells (FIG. 17A).

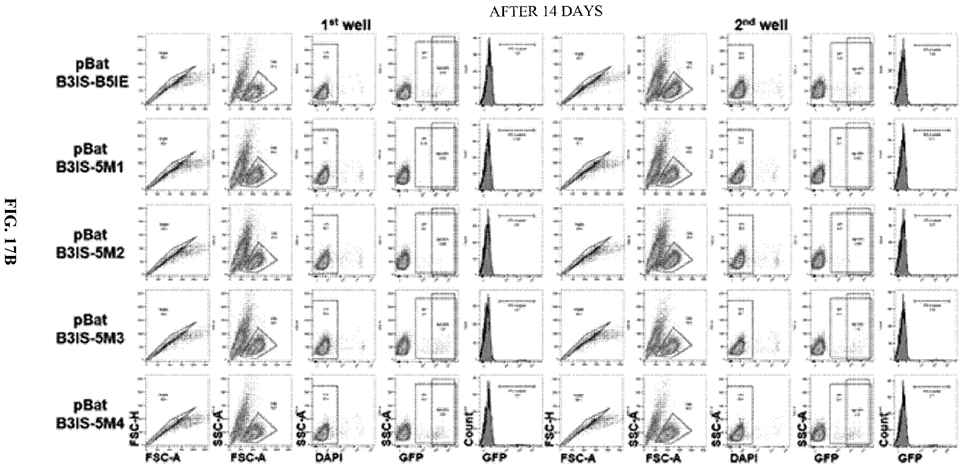

As a result of confirming the proportion of GFP⁺ cells by setting a standard close to GFP⁻ (negative), in the groups in which the transposon mutant according to the present invention was introduced, generally, GFP was expressed at a high level (FIGS. 17B to 17F). Particularly, compared with other groups, the groups in which a 5M3 or 5M4 mutant form-containing transposon was introduced exhibited high proportions of GFP-positive cells. In addition, it was confirmed that the same tendency was confirmed from the results of confirming the proportions of GFP⁺ cells by setting a standard close to high intensity.

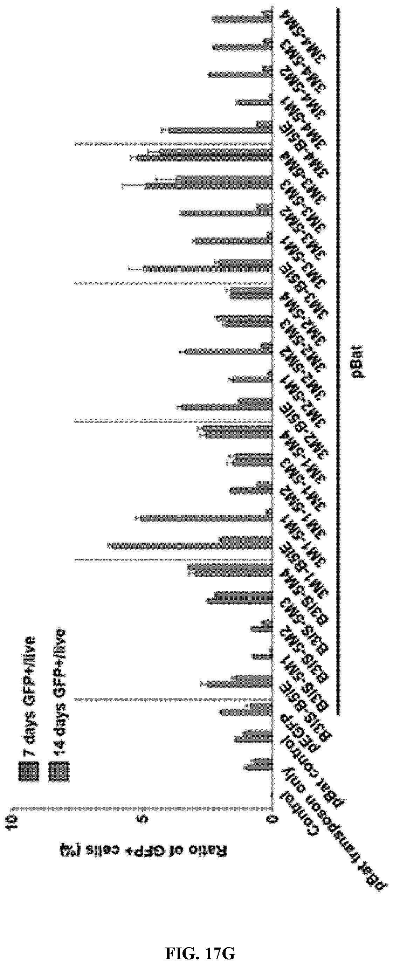

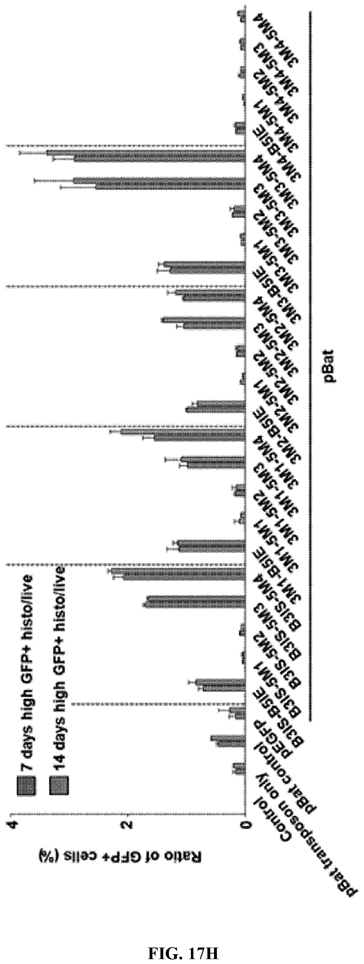

By combining the above results, the results of confirming the proportion of GFP-expressing cells by group over time after transfection are shown in Table 15, and FIGS. 17G and 17H. As can be confirmed in the table and the drawings, transposons containing the 5M3 or 5M4 mutant form exhibited particularly high gene delivery efficiency.

**[Table 15]**

| **Group** | **Day 1** | **Day 7** | | **Day 14** | |
|---|---|---|---|---|---|
| | **GFP+/live** | **GFP+/live** | **High GFP+/live** | **GFP+/live** | **High GFP+/live** |
| Control | 0.0 | 0.0 | 0.0 | 0.0 | 0.0 |
| pBat transposon only | 8.6 | 1.0 | 0.2 | 0.7 | 0.2 |
| pBat control | 9.2 | 1.4 | 0.5 | 1.1 | 0.6 |
| pEGFP | 27.5 | 2.0 | 0.2 | 0.8 | 0.3 |
| B3IS-BSIE | 11.5 | 2.5 | 0.7 | 1.4 | 0.8 |
| B3IS-5M1 | 5.9 | 0.7 | 0.0 | 0.1 | 0.0 |
| B3IS-5M2 | 7.4 | 0.8 | 0.1 | 0.3 | 0.1 |
| B3IS-5M3 | 9.5 | 2.5 | 1.7 | 2.2 | 1.6 |
| B3IS-SM4 | 8.0 | 3.0 | 2.1 | 3.2 | 2.3 |
| 3M1-B5IE | 17.2 | 6.2 | 1.1 | 2.0 | 1.2 |
| 3M1-5M1 | 11.7 | 5.1 | 0.1 | 0.2 | 0.1 |
| 3M1-5M2 | 9.7 | 1.6 | 0.2 | 0.6 | 0.1 |
| 3M1-5M3 | 7.0 | 1.5 | 1.0 | 1.4 | 1.1 |
| 3M1-5M4 | 8.3 | 2.6 | 1.6 | 2.7 | 2.1 |
| 3M2-B5IE | 12.1 | 3.5 | 1.0 | 1.3 | 0.8 |
| 3M2-5M1 | 7.4 | 1.5 | 0.1 | 0.1 | 0.0 |
| 3M2-5M2 | 12.6 | 3.3 | 0.1 | 0.4 | 0.1 |
| 3M2-5M3 | 4.7 | 1.8 | 1.0 | 2.1 | 1.4 |
| 3M2-5M4 | 3.2 | 1.6 | 1.0 | 1.6 | 1.2 |
| 3M3-B5IE | 14.4 | 4.9 | 1.3 | 2.0 | 1.4 |
| 3M3-5M1 | 10.2 | 2.9 | 0.1 | 0.2 | 0.1 |
| 3M3-5M2 | 15.2 | 3.5 | 0.2 | 0.6 | 0.2 |
| 3M3-5M3 | 13.0 | 4.9 | 2.5 | 3.7 | 2.9 |
| 3M3-5M4 | 12.9 | 5.2 | 2.9 | 4.3 | 3.4 |
| 3M4-B5IE | 16.7 | 4.0 | 0.2 | 0.6 | 0.2 |
| 3M4-5M1 | 5.3 | 1.3 | 0.0 | 0.1 | 0.0 |
| 3M4-5M2 | 8.9 | 2.4 | 0.1 | 0.3 | 0.1 |
| 3M4-5M3 | 8.7 | 2.3 | 0.1 | 0.3 | 0.1 |
| 3M4-5M4 | 6.8 | 2.3 | 0.1 | 0.3 | 0.1 |

In this experimental example, a transposon vector having excellent chromosomal integration efficiency and excellent gene expression efficiency was produced based on the transposon discovered through Experimental Example A. To this end, by modifying either the 5' ITR or 3' ITR of the transposon, four 5' ITR mutants and four 3' ITR mutants were produced, respectively. The produced 5' ITR mutants and 3' ITR mutants were the same as those produced in Experimental Example B, but as in Experimental Example B, the 3' ITR mutants were cloned with a sense strand sequence according to the original intension, not with a reverse complement sequence. A total of 26 types of transposon vectors were produced by combining the 5' ITR mutants and the 3' ITR mutants, and their gene delivery efficiencies were evaluated.

As shown in the experimental results, GFP expression was hardly detected in the control from day 7 after transfection of the transposon vector. One the other hand, it was confirmed that, in the cells into which the transposon mutants and transposase according to the present invention were introduced, generally the GFP gene was inserted into the chromosome of the Jurkat cell, thereby stably expressing GFP, and particularly, the cells into which a 5M3 or 5M4-containing transposon vector was introduced, compared to the cells into which other transposon vectors including the original transposon were introduced, relatively high GFP expression was confirmed. That is, this suggests that the areas corresponding to 5M3 and 5M4 in the ITR are essential regions for gene delivery of a transposon and the insertion into chromosomes, and it shows that the transposon mutant vectors according to the present invention can induce stable expression by introducing a target gene into the chromosome of a cell.

### Experimental Example D. Confirmation of gene delivery efficiency of pBat transposon to PBMCs

As described in Example D, it was intended to confirm the gene delivery efficiency by the pBat transposon system in PBMCs using Maxcyte equipment. To this end, the gene delivery efficiencies of mutant form transposons were compared using GFP gene-containing transposons (Naive-GFP, 3M3-5M3-GFP) and 1G4 TCR gene-containing transposons (B3IS-B5IE-1G4, 3M3-5M3-1G4).

### 1. Observation of GFP expression using fluorescence microscope

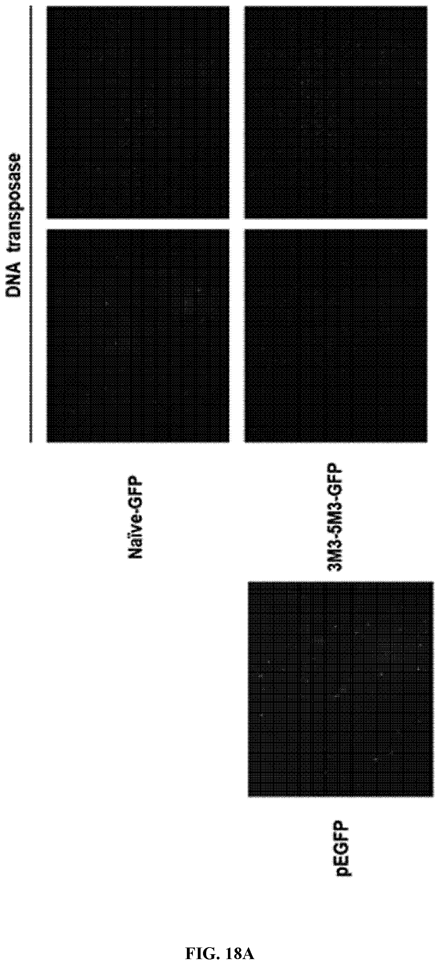

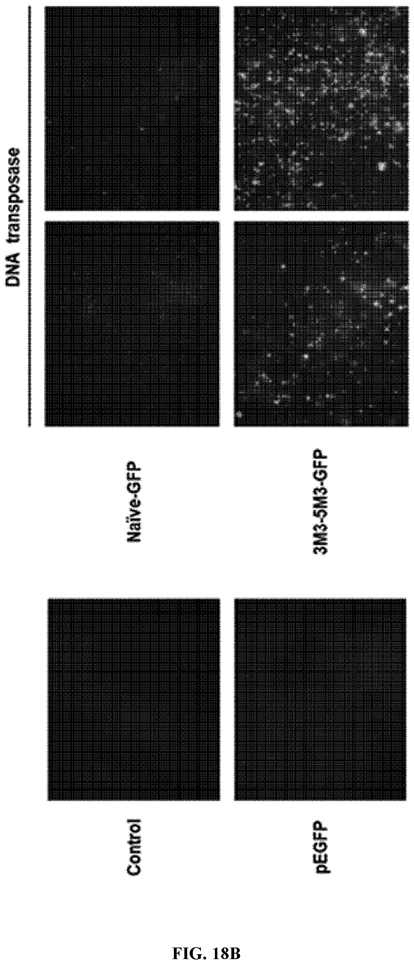

One day and 7 days after electroporation, whether GFP was expressed in the pBat-GFP group was observed using a fluorescence microscope. One day after electroporation, in both the pEGFP and pBat-GFP groups, GFP expression was confirmed as shown in FIG. 18A. As shown in FIG. 18B, seven days after electroporation, GFP was not observed in the pEGFP group, but GFP expression was observed in the pBat-GFP group, it is believed that this is because the GFP gene in the ITR of the transposon vector is integrated into the chromosomes of PBMCs by a transposase and thus stably expressed. In addition, compared to the Naive-GFP group, in the 3M3-5M3-GFP group, GFP was highly expressed, indicating that the 3M3-5M3 vector with mutated ITR sequences has higher gene insertion and expression efficiencies.

### 2. Observation of changes in cell viability and cell number

After Maxcyte electroporation, to confirm the changes in cell viability and cell number, one day and 14 days after electroporation, cell counting was performed. As shown in Table 16 below, one day after electroporation, in the NO EP group (group without electroporation) and the control (group with only electroporation), 90% or higher viability was shown, but in the pEGFP group, a viability of 69.5% was shown, and in all of the pBat transposon-introduced groups, a low viability of approximately 33% to 62% was shown. In the pEGFP group and the pBat transposon groups (excluding the 3M3-5M3-1G4 + DNA group, the cell number was 1×10⁶ or less, which was reduced by more than 75% compared to the cell number (4×10⁶) of initially seeded PBMCs. On the other hand, 14 days after electroporation, a high viability of 88% or more was shown in all groups, and the cell numbers of the pBat-GFP group and the pBat-1G4 group were approximately 2×10⁶ to 6×10⁶, and approximately 3.8×10⁶ to 12×10⁶, respectively, confirming that, as the cells are proliferated during the culture period, the overall cell number increases. Particularly, in the pBat-1G4 group, the cells appeared to have highly proliferated. The above results are believed to be because cells were damaged by electroporation and thus cell viability decreased significantly after one day, but the cells recovered and proliferated during the culture period and thus both the cell viability and cell number increased after 14 days. In addition, since added feeder cells (A375 cells irradiated with radiation) express HLA*02:01 and NY-ESO1, which are counter partners of the 1G4 TCR, it seems that T cells expressing the 1G4 TCR were activated and proliferated by the feeder cells.

**[Table 16]**

| No. | Sample | After one day | | After 14 days | |
|---|---|---|---|---|---|
| | | Cell viability (%) | Cell number (×10⁵) | Cell viability (%) | Cell number (×10⁵) |
| 1 | No EP | 98.6 | 30.96 | 88.5 | 68 |
| 2 | Control | 95.2 | 24.75 | 89.7 | 124.8 |
| 3 | Control | 94.4 | 27.45 | 88.4 | 133.6 |
| 4 | pEGFP | 69.5 | 7.38 | 89.1 | 84.8 |
| 6 | Naive-GFP + DNA | 36.2 | 2.25 | 96.8 | 48.8 |
| 7 | Naive-GFP + DNA | 42.3 | 1.98 | 96.3 | 61.6 |
| 9 | 3M3-5M3-GFP + DNA | 36.2 | 1.53 | 92.9 | 31.2 |
| 10 | 3M3-5M3-GFP + DNA | 57.8 | 4.32 | 90.3 | 22.4 |
| 12 | B3IS-B5IE-1G4 + DNA | 50.7 | 3.42 | 97.0 | 102.4 |
| 13 | B3IS-B5IE-1G4 + DNA | 47.7 | 2.79 | 95.4 | 66.4 |
| 16 | 3M3-5M3-1G4 + DNA | 51.2 | 3.96 | 93.6 | 116.8 |
| 17 | 3M3-5M3-1G4 + DNA | 44.4 | 2.16 | 96.0 | 76 |
| 18 | 3M3-5M3-1G4 + DNA | 40.0 | 2.70 | 93.1 | 43.2 |
| 21 | 3M3-5M3-1G4 + DNA | 39.0 | 3.51 | 90.6 | 38.4 |

### 3. FACS analysis of pBat-GFP group 7 days after electroporation

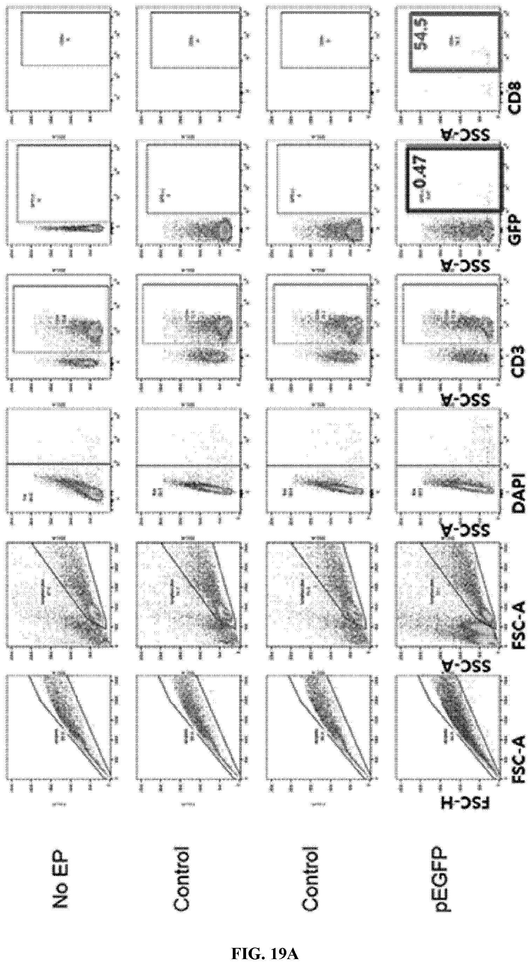

Seven days after electroporation, a GFP expression ratio was confirmed through FACS analysis. As shown in FIG. 19A, the gene delivery efficiency into CD3⁺ T cells was confirmed by gating in the following order: singlets → lymphocytes → live cells → CD3⁺ T cells → GFP⁺ T cells → CD8⁺ T cells, and the proportion of CD8⁺ cytotoxic T cells among the gene-delivered T cells was also analyzed.

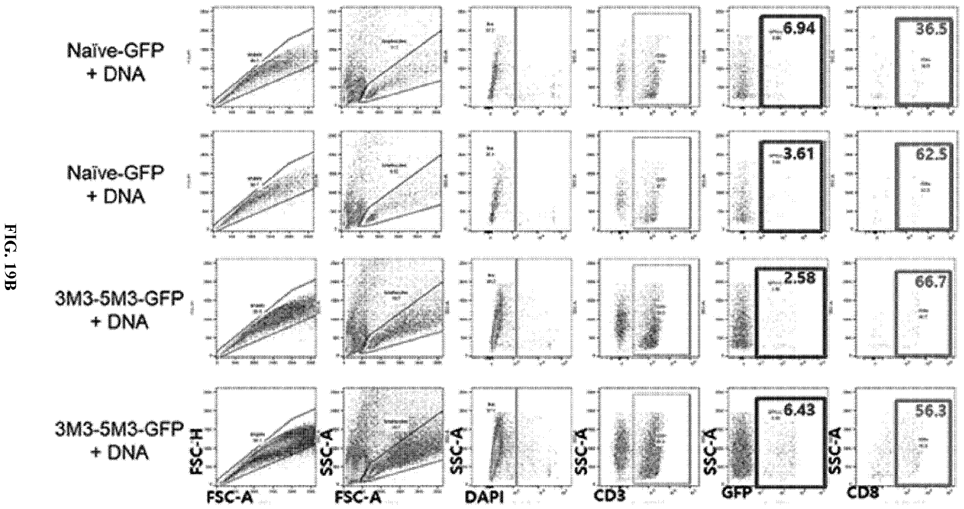

Seven days after electroporation, the proportion of GFP-expressing cells among the CD3+ T cells was 0% in the control, and 0.47% in the pEGFP group (FIG. 19A). On the other hand, it was confirmed that, in the Naive-GFP+DNA groups, the proportions of GFP⁺ T cells were 6.94% and 3.61%, and in the 3M3-5M3-GFP + DNA groups, the proportions of GFP⁺ T cells were 2.58% and 6.43%, and these values are higher than that of the control (FIG. 19B). In addition, as a result of confirming the proportion of CD8⁺ T cells among the GFP⁺ T cells, it was confirmed that in the pBat naive group, the proportion was approximately 35%, and in the pBat 3M3-5M3 group, the proportion was approximately 60%. Accordingly, it seems that, in the pBat-GFP group, GFP gene-delivered T cells did not specifically proliferate but proliferated randomly.

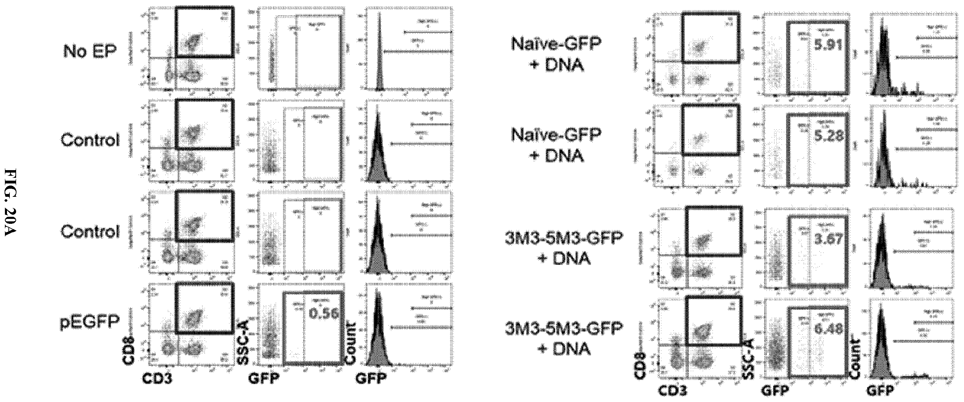

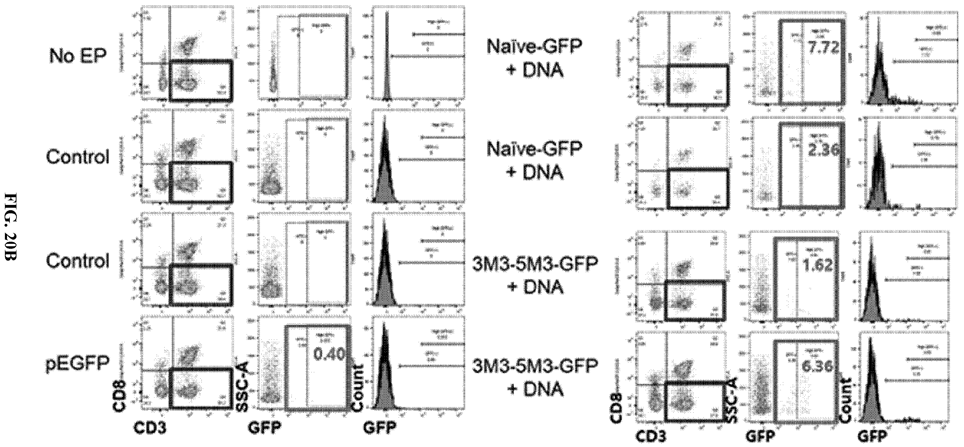

In addition, 7 days after electroporation, the gene delivery efficiencies in the CD3⁺CD8⁺ T cells and the CD3⁺CD8⁻ T cells by gating in the following order: singlets → lymphocytes → live cells → CD3⁺CD8⁺ T cells or CD3⁺CD8⁻ T cells → GFP⁺ cells. As a result, as shown in FIGS. 20A and 20B, the proportions of GFP⁺ cells among the CD3⁺CD8⁺ T cells and the CD3⁺CD8⁻ T cells were approximately 1% to 8% for each group, showing no significant difference.

### 4. FACS analysis of pBat 1G4 group 7 days after electroporation

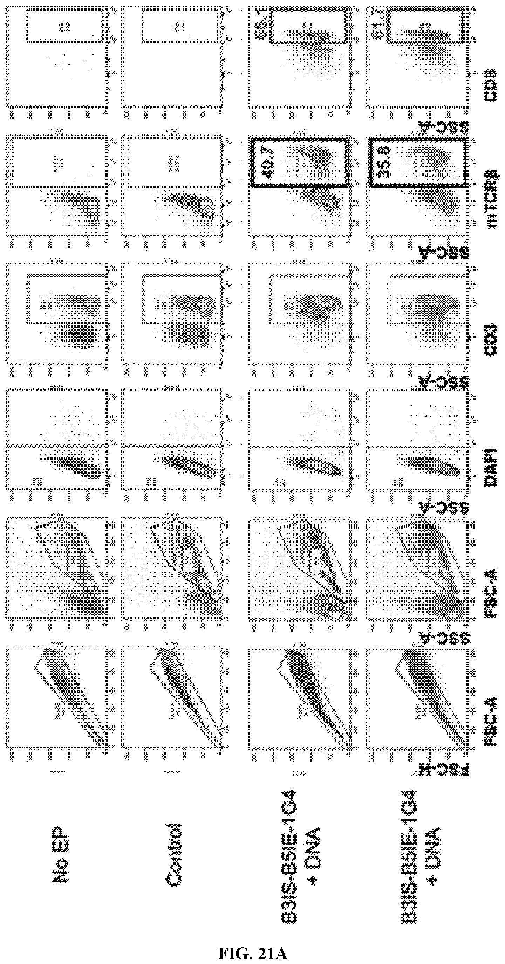

Seven days after electroporation, the 1G4 TCR expression ratio was confirmed through FACS analysis. According to the analysis method, as shown in FIG. 21A, the efficiency of gene delivery into CD3⁺ T cells was confirmed by gating in the following order: singlets → lymphocytes → live cells → CD3⁺ T cells → mTCRβ⁺ T cells → CD8⁺ T cells, and the proportion of CD8⁺ cytotoxic T cells among the gene-delivered T cells was also analyzed.

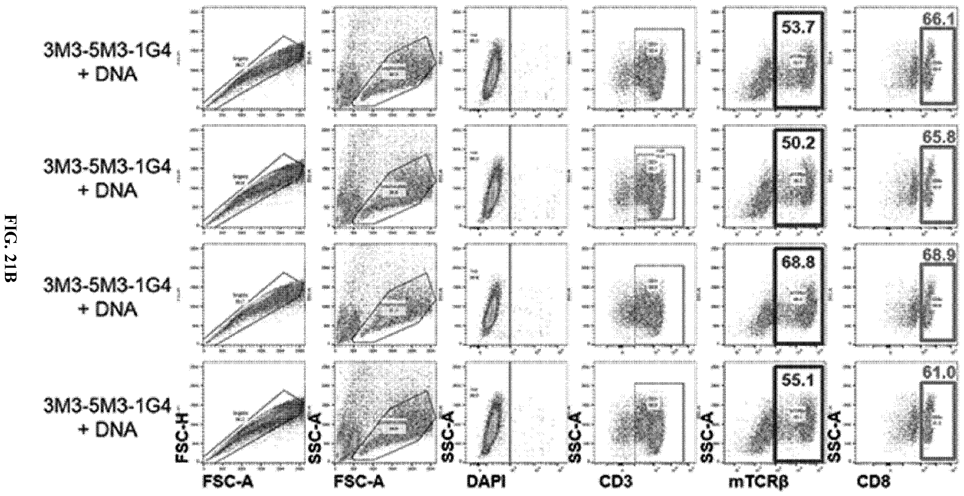

Seven days after electroporation, the proportion of mTCRβ⁺ cells among the CD3⁺ T cells was 0% in the control, but 40.7% and 35.8% in the B3IS-B5IE-1G4 + DNA groups, and 53.7%, 50.2%, 68.8%, and 55.1% in the 3M3-5M3-1G4 + DNA groups, confirming that the proportion of mTCRβ⁺ cells was the highest in the combination of 3M3-5M3-1G4 transposon and transposase DNA (FIGS. 21A and 21B). In addition, as a result of analyzing the proportion of CD8⁺ T cells in the mTCRβ⁺ T cell population, it was confirmed that these cells are present in overall similar proportions, approximately 61 to 69%.

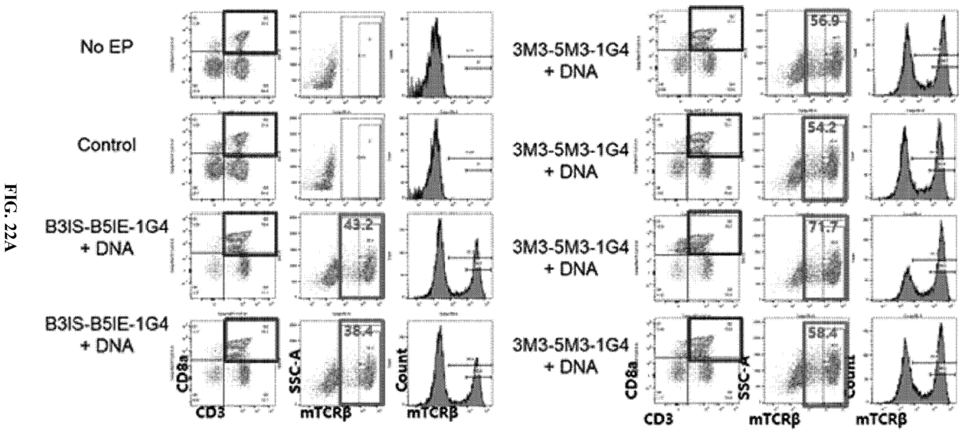

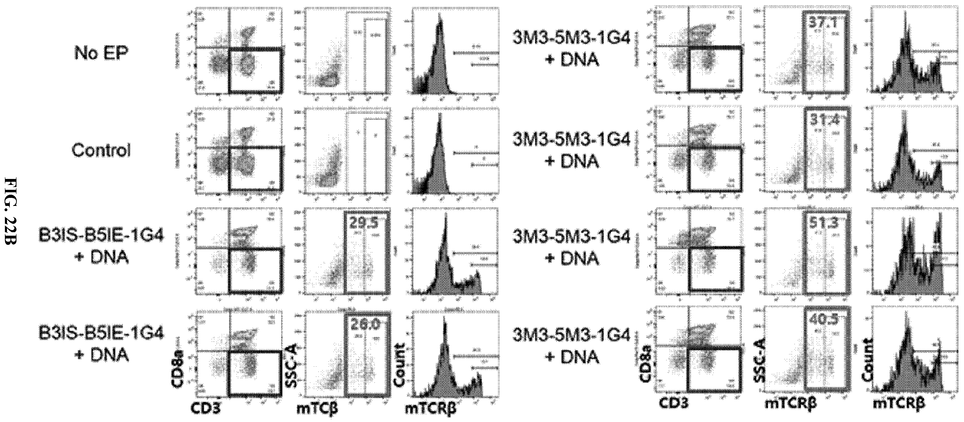

In addition, 7 days after electroporation, to analyze the proportions of mTCRβ-expressing CD8⁺ and CD8⁻ T cells, the gene delivery efficiencies in T cells were analyzed by gating in the following order: singlets → lymphocytes → live cells → CD3⁺CD8⁺ T cells or CD3⁺CD8⁻ T cells → mTCRβ⁺ cells. As a result, as shown in FIGS. 22A and 22B, in terms of the proportion of mTCRβ⁺ cells among the CD3⁺CD8⁺ T cells or CD3⁺CD8⁻ T cells, the proportion of mTCRβ⁺ cells in the CD3⁺CD8⁺ T cells was higher than that in the CD3⁺CD8⁻ T cells.

### 5. FACS analysis of pBat-GFP group 14 days after electroporation

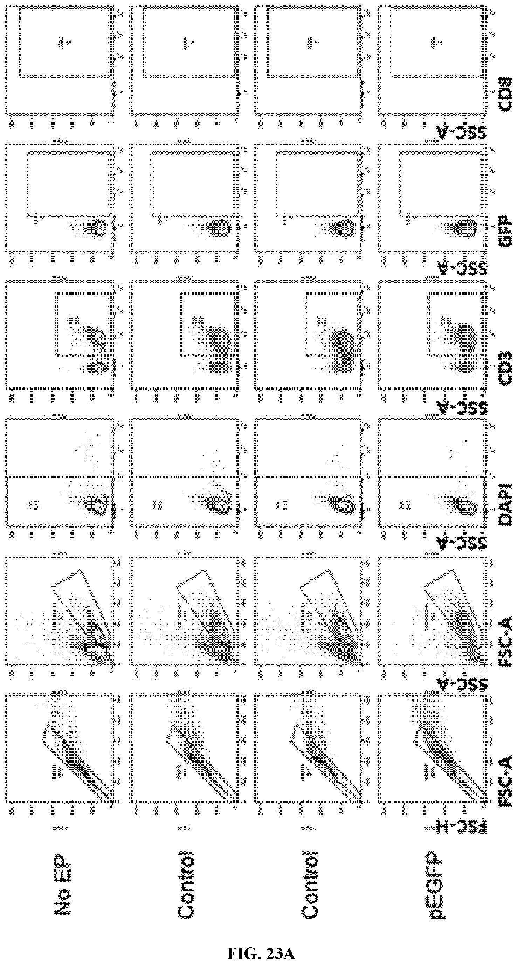

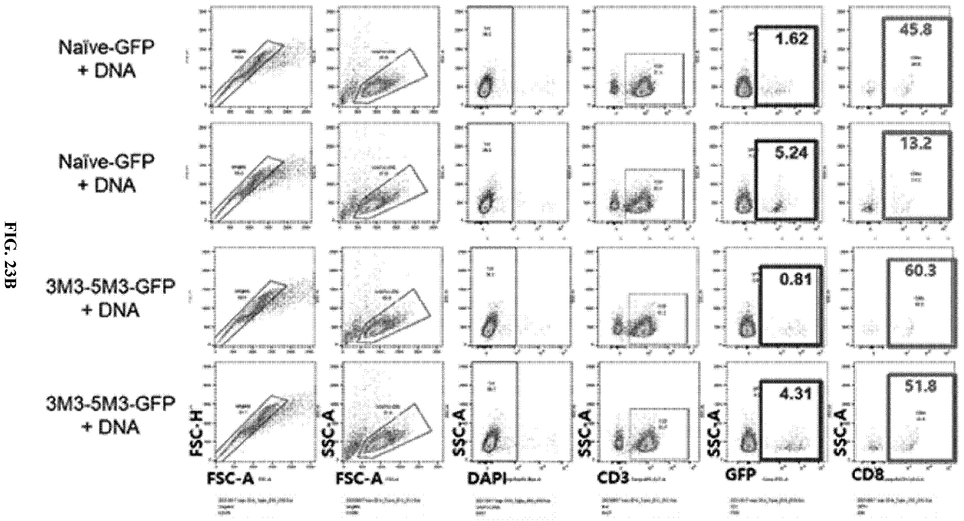

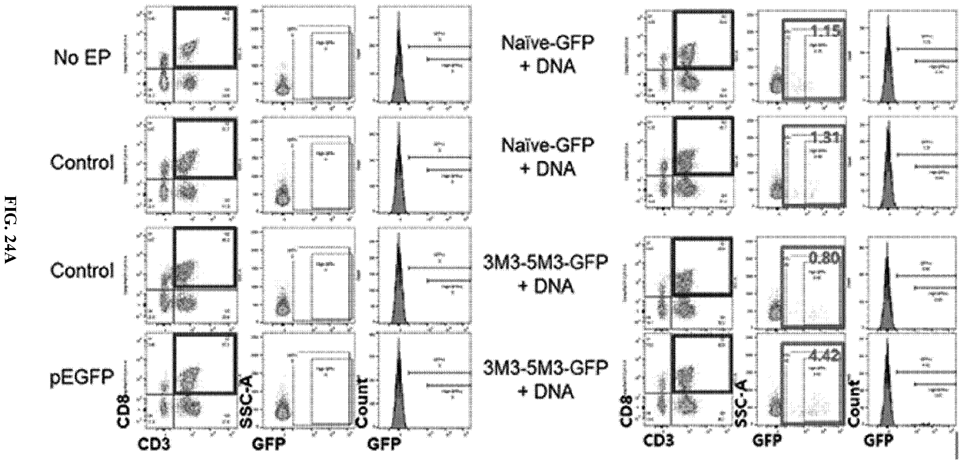

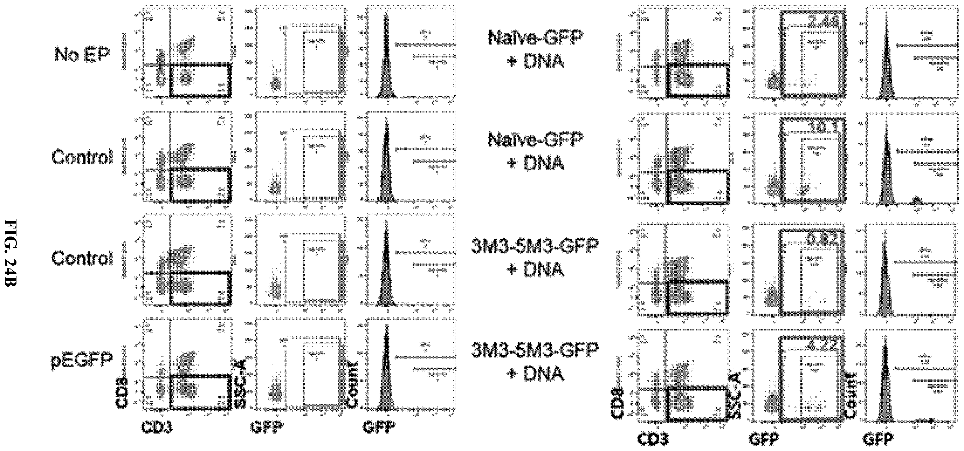

Fourteen days after electroporation, the proportion of GFP-expressing cells among CD3⁺ T cells was 0% in both the control and the pEGFP group. On the other hand, the proportions of GFP-expressing CD3⁺ T cells were 1.62% and 5.24% in the Naive-GFP+DNA groups, and 0.81% and 4.31% in the 3M3-5M3-GFP+DNA groups, thereby confirming a similar trend to that observed 7 days after electroporation (FIGS. 23A and 23B). There were no significant differences between the pBat naive groups and the pBat 3M3-5M3 groups, and the proportion of CD8⁺ T cells among GFP⁺ T cells was approximately 41% in the pBat naive groups and approximately 60% in the pBat 3M3-5M3 groups. In addition, 14 days after electroporation, as a result of analyzing the gene delivery efficiencies in CD3⁺CD8⁺ T cells and CD3⁺CD8⁻ T cells, as shown in FIGS. 24A and 24B, the proportions of GFP⁺ cells among the CD3⁺CD8⁺ and CD3⁺CD8⁻ T cells were almost the same, approximately 1 to 10%.

### 6. FACS analysis of pBat 1G4 group 14 days after electroporation

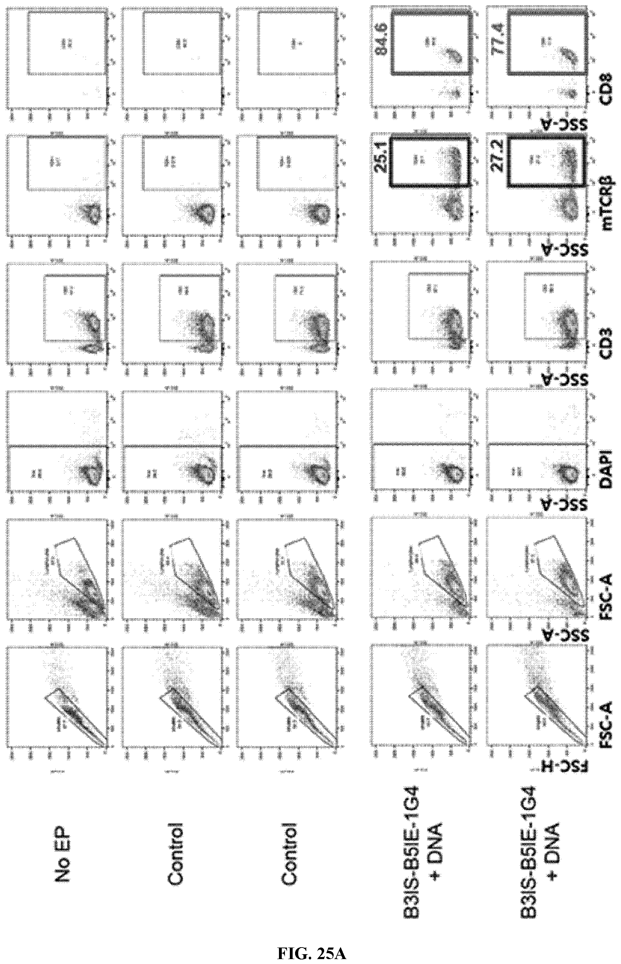

Fourteen days after electroporation, the 1G4 TCR expression ratio was confirmed through FACS analysis. According to the analysis method, as shown in FIG. 25A, the efficiency of gene delivery into the CD3⁺ T cells was confirmed by gating in the following order: singlets → lymphocytes → live cells → CD3⁺ T cells → mTCRβ⁺ T cells → CD8⁺ T cells, and the proportion of CD8⁺ cytotoxic T cells among the gene-delivered T cells was also analyzed.

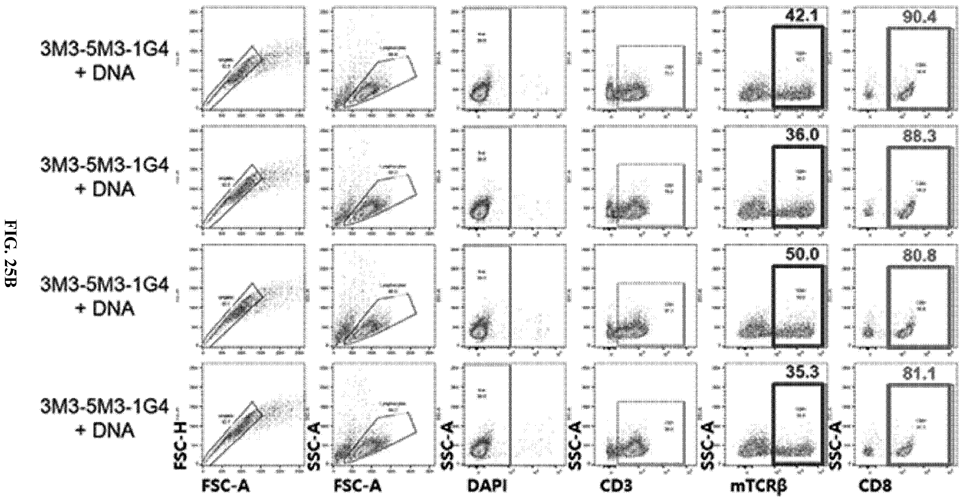

Fourteen days after electroporation, the proportion of mTCRβ⁺ cells among CD3⁺ T cells was 0% in the control, 25.1% and 27.2% in the B3IS-B5IE-1G4+DNA groups, and 42.1%, 36.0%, 50.0% and 35.3% in the 3M3-5M3-1G4+DNA groups, confirming that, overall, the proportions of mTCRβ⁺ cells were slightly lower than those obtained 7 days after electroporation, but highest in the combination of 3M3-5M3-1G4 transposon and transposase DNA (FIGS. 25A and 25B). In addition, as a result of analyzing the proportions of CD8⁺ T cells among mTCRβ⁺ T cells, overall, the proportions were approximately 75% to 90% with no significant difference between groups, and an overall increase compared to 7 days after electroporation was confirmed. This is considered to be a result of an increase in 1G4 TCR-expressing CD8⁺ T cells due to feeder cells added during culture.

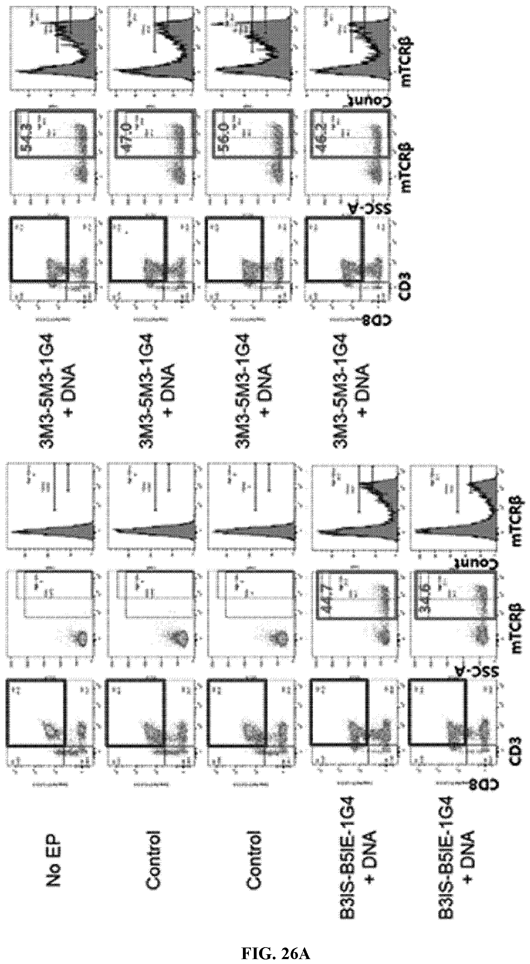

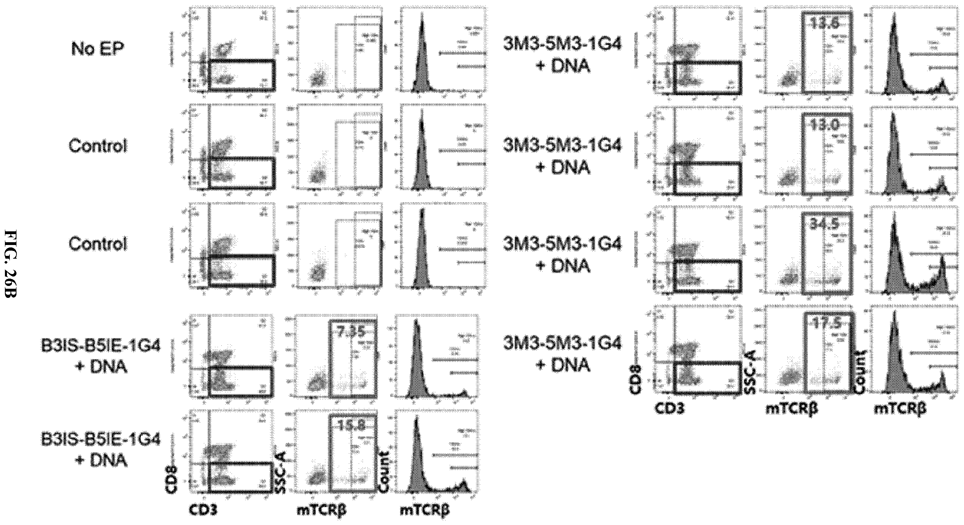

In addition, 14 days after electroporation, the gene delivery efficiencies in CD3⁺CD8⁺ T cells and CD3⁺CD8⁻ T cells were analyzed. As a result, as shown in FIGS. 26A and 26B, the proportion of mTCRβ⁺ cells in the CD3⁺CD8⁺ T cells was higher than that in the CD3⁺CD8⁻ T cells, and the proportion of mTCRβ⁺ cells among the CD3⁺CD8⁺ T cells was similar to that obtained 7 days after electroporation, but the proportion of mTCRβ⁺ cells among the CD3⁺CD8⁻ T cells greatly decreased in all groups compared to the results obtained on day 7.

As seen from the above results, one day after electroporation, the cell number and cell viability were relatively lower in the transposon + transposase (plasmid DNA)-containing group compared to the negative control. However, as the cells proliferated during culture, the number of transposon + transposase-introduced cells greatly increased 7 days after electroporation, and viability also increased compared to the negative control. One day after electroporation, as a result of observing GFP or 1G4 TCR expression after A375 cells irradiated with radiation were added as feeder cells and cultured, the proportion of GFP-expressing cells among CD3⁺ T cells was 1 to 7% on day 7 after electroporation, and 0.8 to 6% on day 14 after electroporation. On the other hand, the proportion of 1G4 TCR-expressing cells among CD3⁺ T cells was very high, ranging from 14 to 69% on day 7 after electroporation and 10 to 50% on day 14 after electroporation. As described above, it seems that the reason why the proportion of 1G4 TCR-expressing cells is particularly high compared to that of GFP-expressing cells is because A375 cells, which are feeder cells, express NY-ESO-1, the target antigen of the 1G4 TCR, and T cells activated by the antigen actively proliferate. This suggests that when a gene is delivered to primary T cells using a pBat transposon system, gene-introduced T cells can obtain higher gene delivery efficiency by stimulation with an antigen during the culture period.

In the case of the gene delivery into CD8⁺ or CD8⁻ T cells of the CD3⁺ T cells, the gene delivery efficiency into CD8⁺ T cells was higher than that into CD8⁻ T cells on day 7 and day 14 after electroporation. In addition, as a result of comparing gene delivery efficiency according to a transposon mutant form, it was confirmed that the proportion of 1G4 TCR-expressing T cells is the highest in the 3M3-5M3-1G4 transposon-introduced group. The above results show that the transposon system according to the present invention performs an excellent gene delivery function even in PBMCs.

### Experimental Example E. Confirmation of gene delivery efficiency according to T cell activation period in TCT-T production using pBat transposon system

In production of 1G4 TCR-T cells by delivering the 1G4 TCR gene to PBMCs of a healthy person using the pBat transposon system, it was examined whether there are differences in delivery and expression efficiency of the 1G4 TCR gene according to the T cell activation period.

### 1. Analysis of 1G4 TCR expression 7 days after electroporation

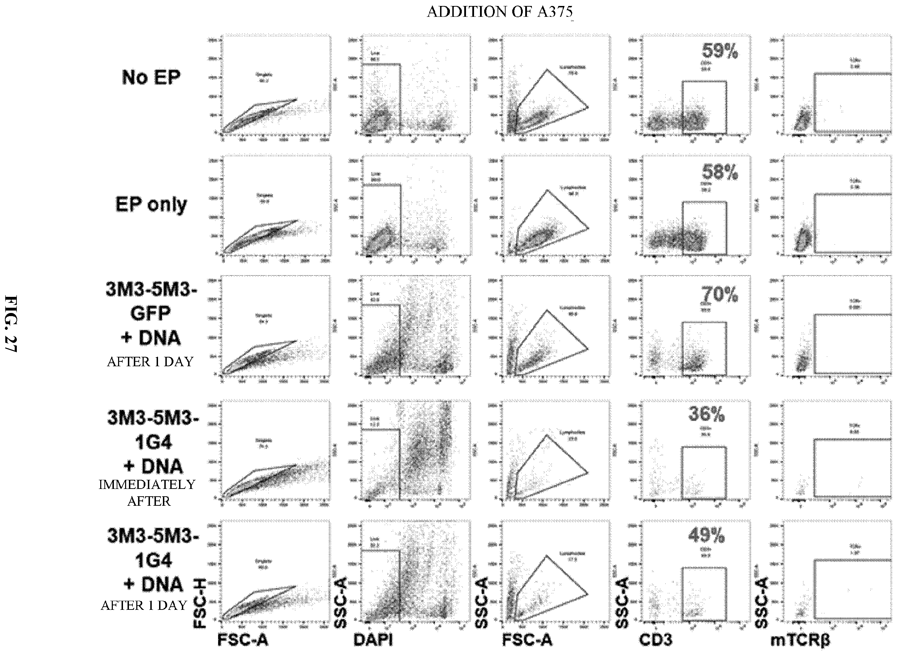

Since the constant region of the TCR used in the experiment was a mouse constant region, the 1G4 TCR expression rate was confirmed through FACS analysis using an antibody against this, and according to the analysis method shown in FIG. 27, the efficiency of gene delivery into CD3⁺ T cells was confirmed by gating in the following order: singlets → live cells → lymphocyte → CD3⁺ T cells → mTCRβ⁺ T cells.

Seven days after electroporation, when all of the groups in which T cells were activated using the irradiated A375 cells were observed with the naked eye, cell proliferation rates were low, and as shown in FIG. 27, mTCRβ⁺ T cells were hardly observed among CD3⁺ T cells.

### 2. Analysis of 1G4 TCR expression 10 days after electroporation

Ten days after electroporation, 1G4 TCR gene expression efficiency among CD3⁺ T cells was examined, and the proportion of CD8+ or CD4+ T cells among 1G4 TCR-expressing T cells (CD3⁺mTCRβ⁺ T cells) was analyzed using CD8 and CD4 markers, and the proportion of memory-type T cells was analyzed using CD45RA and CD62L markers.

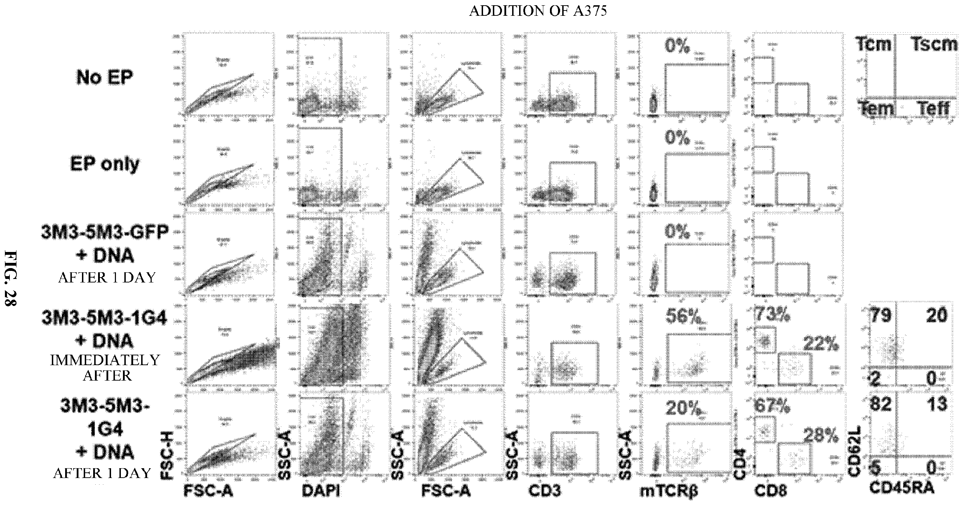

As shown in FIG. 28, ten days after electroporation, the proportion of mTCRβ-expressing cells among CD3⁺ T cells was 0% in all of the No EP, EP only, and 3M3-5M3-GFP + DNA (after one day) groups. On the other hand, it was confirmed that the proportion of mTCRβ-expressing cells was 56% in the 3M3-5M3-1G4+DNA (immediately after) group, and 20% in the 3M3-5M3-1G4+DNA (after one day) group, confirming that the proportion of mTCRβ-expressing cells is higher in the group in which the irradiated A375 cells are added immediately after electroporation.

In addition, as a result of measuring the proportions of CD8⁺ T cells and CD4⁺ T cells among the 1G4 TCR-expressing T cells, the proportions were measured at 22% and 73% in the 3M3-5M3-1G4+DNA (immediately after) group, respectively, and 28% and 67% in the 3M3-5M3-1G4+DNA (after one day) group, respectively, confirming that the proportion of CD4⁺ T cells is approximately 3-fold higher than that of CD8⁺ T cells.

The memory-type T cells may be distinguished using CD45RA and CD62L markers. When distinguishing the memory-type T cells using CD45RA and CD62L markers, CD45RA⁺CD62L⁻ T cells were classified as Teff cells, CD45RA⁻CD62L⁻ T cells were classified as Tem cells, CD45RA⁻CD62L⁺ T cells were classified as Tcm cells, and CD45RA⁺CD62L⁺ T cells were classified as Tscm cells. In terms of the proportions of memory-type T cells among the 1G4 TCR-expressing T cells, in the 3M3-5M3-1G4+DNA (immediately after) group, the proportions were 0% for Teff cells, 2% for Tem cells, 79% for Tcm cells, and 20% for Tscm cells, and in the 3M3-5M3-1G4+DNA (after one day) group, the proportions were 0% for Teff cells, 5% for Tem cells, 82% for Tcm cells, and 13% for Tscm cells, confirming that Tcm cells were the most abundant and Tscm cells were the second most abundant (Table 17).

**[Table 17]**

| | **Teff** | **Tem** | **Tcm** | **Tscm** |
|---|---|---|---|---|
| 3M3-5M3-1G4 + DNA (immediately after) group | CD45RA⁺CD62L⁻ | CD45RA⁻CD62L⁻ | CD45RA⁻CD62L⁺ | CD45RA⁺CD62L⁺ |
| | 0% | 2% | 79% | 20% |
| 3M3-5M3-1G4 + DNA (after one day) group | CD45RA⁺CD62L⁻ | CD45RA⁻CD62L⁻ | CD45RA⁻CD62L⁺ | CD45RA⁺CD62L⁺ |
| | 0% | 5% | 82% | 13% |

### 3. Analysis of 1G4 TCR expression 14 days after electroporation

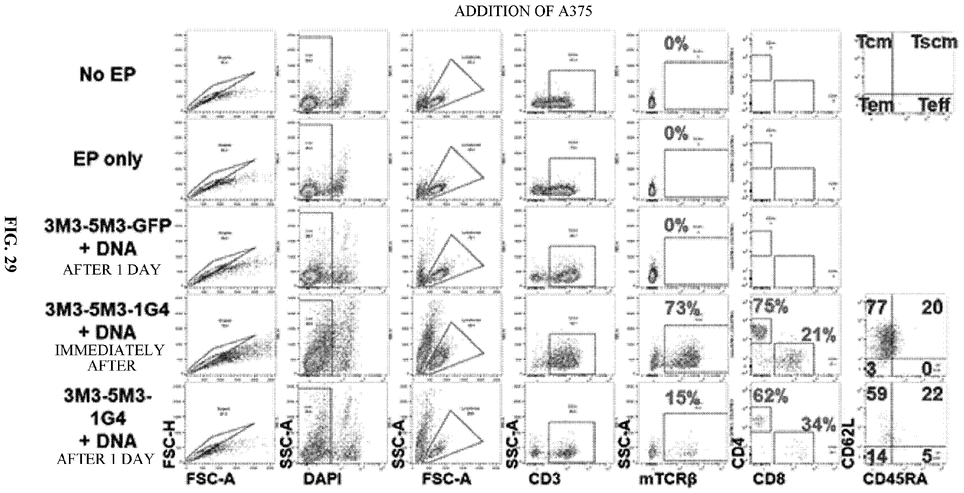

As shown in FIG. 29, fourteen days after electroporation, the proportion of mTCRβ-expressing cells among CD3⁺ T cells was 0% in all of the No EP, EP only, and 3M3-5M3-GFP+DNA (after one day) groups. On the other hand, the proportion of mTCRβ-expressing cells among CD3⁺ T cells was 73% in the 3M3-5M3-1G4+DNA (immediately after) group, and 15% in the 3M3-5M3-1G4 + DNA (after one day) group, confirming that, similar to the results obtained 10 days after electroporation, the proportion of mTCRβ-expressing cells was higher in the group in which the irradiated A375 cells were added immediately after electroporation.

The proportions of CD8⁺ T cells and CD4⁺ T cells in 1G4 TCR-expressing T cells were 21% and 75%, respectively, in the 3M3-5M3-1G4+DNA (immediately after) group, and 34% and 62%, respectively, in the 3M3-5M3-1G4+DNA (after one day) group, indicating that, similar to the results obtained on day 7 after electroporation, the proportion of CD4⁺ T cells is approximately 2 to 3-fold higher than that of CD8⁺ T cells.

When distinguishing the memory-type T cells using CD45RA and CD62L markers, the proportions of memory-type T cells in the 1G4 TCR-expressing T cells were 0% for Teff cells, 3% for Tem cells, 77% for Tcm cells, and 20% for Tscm cells in the 3M3-5M3-1G4+DNA (immediately after) group, and 5% for Teff cells, 14% for Tem cells, 59% for Tcm cells, and 22% for Tscm cells in the 3M3-5M3-1G4+DNA (after one day) group, demonstrating that Tcm cells were the most abundant, and the Tscm cells were the second most abundant (Table 18).

**[Table 18]**

| | **Teff** | **Tem** | **Tcm** | **Tscm** |
|---|---|---|---|---|
| 3M3-5M3-1G4+DNA | CD45RA⁺CD62L⁻ | CD45RA⁻CD62L⁻ | CD45RA⁻CD62L⁺ | CD45RA⁺CD62L⁺ |
| | 0% | 3% | 77% | 20% |
| (immediately after) group | | | | |
| 3M3-5M3-1G4+DNA (after one day) group | CD45RA⁺CD62L⁻ | CD45RA⁻CD62L⁻ | CD45RA⁻CD62L⁺ | CD45RA⁺CD62L⁺ |
| | 5% | 14% | 59% | 22% |

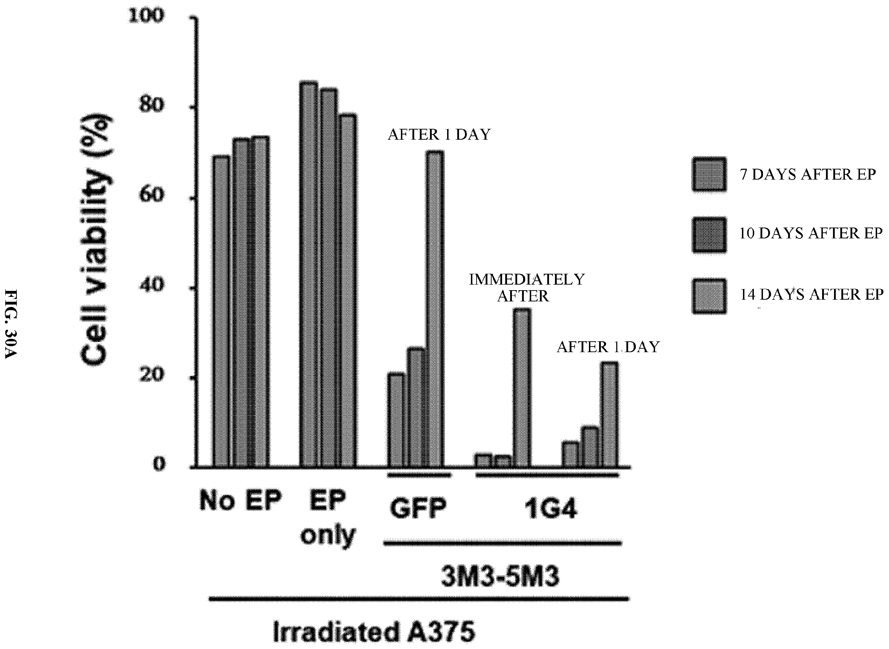

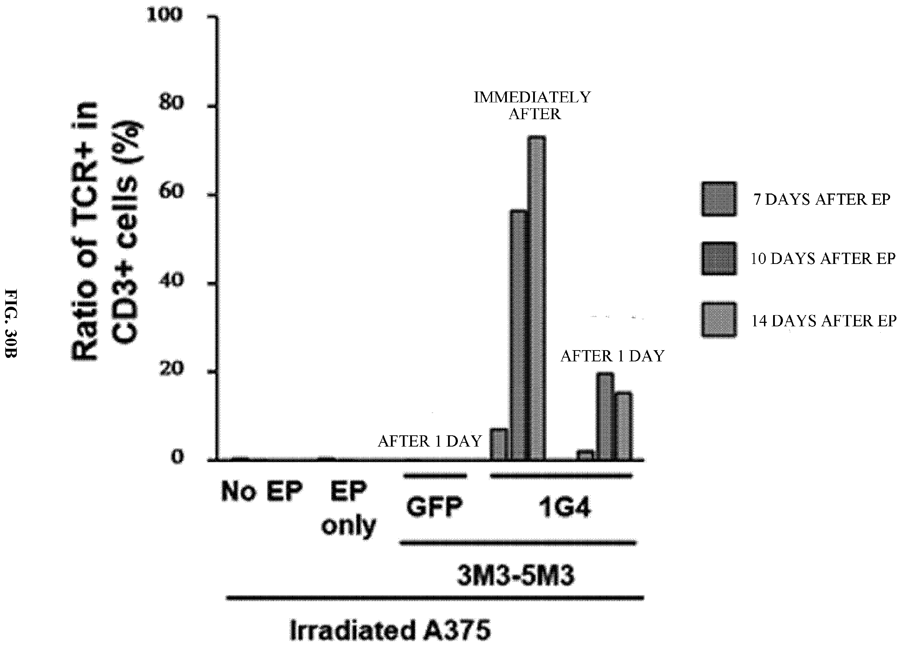

By combining the above results, it was confirmed that, comparing the cell viabilities of the cells that are co-cultured with feeder cells after vector introduction through electroporation 7, 10, and 14 days after electroporation, as shown in FIG. 30A, the viability decreased after 7 days gradually recovered in groups excluding the No EP and EP only groups. As shown in FIG. 30B, the proportion of 1G4 TCR-expressing T cells was the highest in the 3M3-5M3-1G4+DNA (immediately after) group after 10 and 14 days. The above results show that, rather than adding feeder cells one or more days after introducing a transposon and a transposase plasmid into the cells through electroporation, T cell activation by adding feeder cells immediately after electroporation is advantageous for gene delivery and expression.

### Experimental Example F. Production of CAR-T using pBat transposon system

In the previous experimental example, it was confirmed that TCR-T cells can be produced by delivering the TCR gene into PBMCs using the pBat transposon system. Subsequently, this experimental example was intended to examine whether the transposon system of the present invention also effectively delivers other genes, in addition to the TCR gene, to produce genetically modified T cells. To this end, in this experimental example, a 3M3-5M3-CD19 CAR vector was constructed by replacing the TCR gene between the 5' ITR and 3' ITR with the CD19 CAR gene in a 3M3-5M3 transposon vector. The efficiency of CAR-T production mediated by the pBat transposon system was confirmed by measuring the proportion of CD19-expressing CAR-T cells during the electroporation and culture of PBMCs with the 3M3-5M3-CD19 CAR transposon vector and the transposase vector for gene delivery using Maxcyte equipment.

### 1. Analysis of CD19 CAR expression 7 days after electroporation

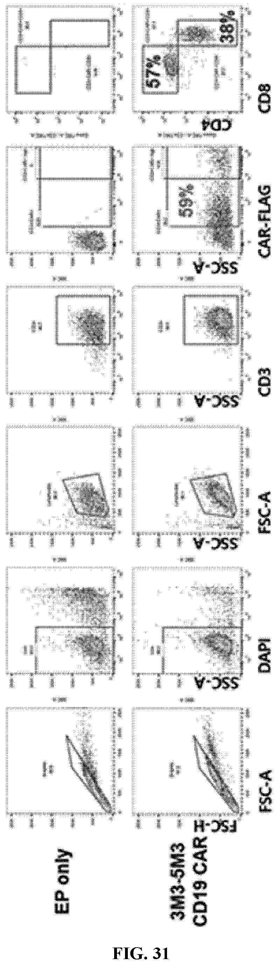

Seven days after electroporation, CD19 CAR expression was confirmed by FACS analysis. According to the analysis method, as shown in FIG. 31, the efficiency of CD19 CAR gene delivery into CD3⁺ T cells was confirmed by gating in the following order: singlets → live cells → lymphocytes → CD3⁺ T cells → FLAG⁺ T cells → CD8⁺ or CD4⁺ T cells, and the proportion of CD8⁺ or CD4⁺ T cells among the gene-delivered T cells was analyzed. Since the FLAG tag sequence was located between the leader sequence and CD19scFv of the CD19 CAR gene of the transposon vector, CD19 CAR protein expression was identified using an anti-FLAG tag antibody.

Seven days after electroporation, it was confirmed that the proportion of FLAG-expressing cells among the CD3⁺ T cells was 0% regardless of the time of T cell activation in the EP only negative control in which electroporation (EP) was only performed without a plasmid. On this other hand, in the group (3M3-5M3 CD19 CAR) in which T cells were activated after introducing 3M3-5M3 CD19 CAR through electroporation, the proportion of FLAG positive CD3⁺ cells was very high at 59%. The proportions of CD8⁺ cells and CD4⁺ cells in the FLAG-expressing T cells were 38% and 57%, respectively, confirming that there were more CD4⁺ T cells (FIG. 31).

### 2. Confirmation of proportion of CD19 CAR-expressing cells 7 days after electroporation

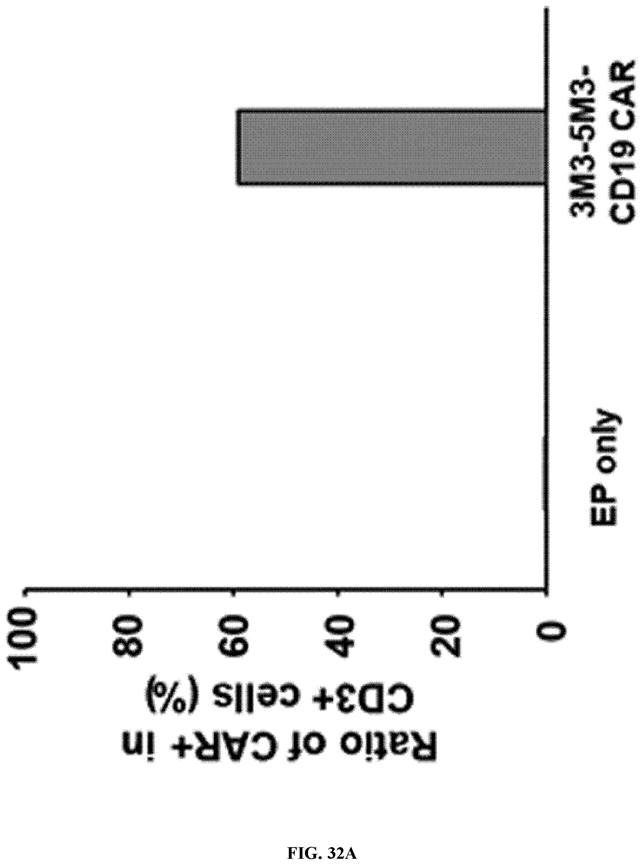

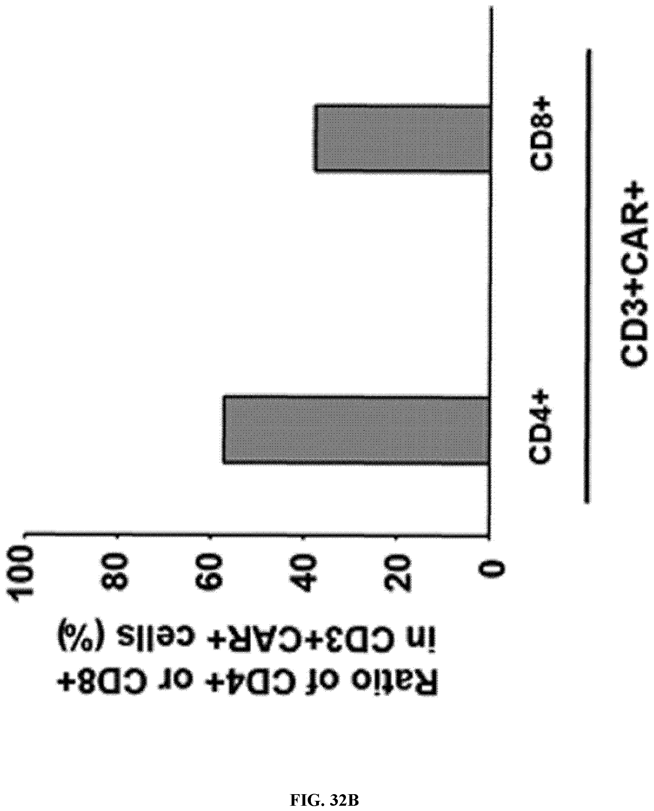

Seven days after electroporation, as a result of confirming the proportion of CD19 CAR-expressing T cells in the 3M3-5M3-CD19 CAR group, as shown in FIG. 32A, a very high proportion of approximately 59% was confirmed in the 3M3-5M3-CAR-introduced group. In addition, as a result of comparing the proportion of CD8⁺ or CD4⁺ cells in the CD19 CAR-expressing T cells, the proportion of CD4⁺ cells was higher than that of CD8⁺ cells (FIG. 32B).

From the above results, it was confirmed that the transposon system according to the present invention can express the CAR gene as well as the TCR gene by effectively delivering them into cells. Particularly, it is considered that, when T cells are activated by introducing a transposon and a transposase through electroporation, gene delivery and expression efficiency can be further improved.

### Experimental Example G. Additional verification of CAR-T production using pBat transposon system

As in the previous experimental example, the gene was introduced into PBMCs using the transposon system of the present invention, which contains the CAR gene, and whether CAR-T cells were effectively produced was further verified.

### 1. Confirmation of total cell number after electroporation

The results that confirm the viability and total cell number of the cultured cells 7 days after electroporation are shown in Table 19 below.

**[Table 19]**

| Group | After 7 days | |
|---|---|---|
| | Cell viability (%) | Cell number (x10⁷) |
| EP only | 96.4 | 3 |
| CD19 CAR + DNA (transposase) | 93.8 | 0.7 |

### 2. Analysis of CD19 CAR protein expression 7 days after electroporation

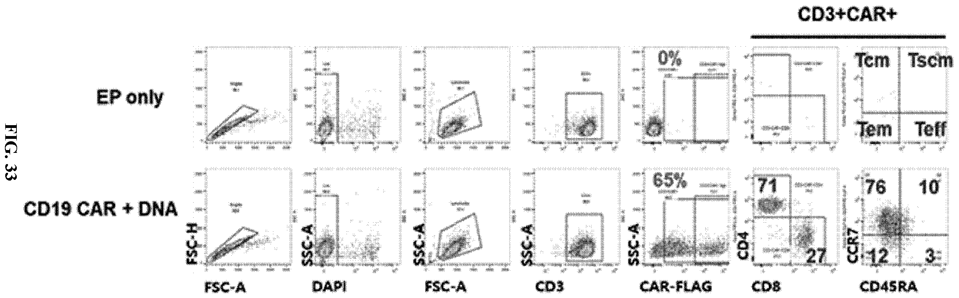

Since the FLAG tag gene is present between the leader sequence and CD19scFv of the CD19 CAR gene inserted into a transposon vector, cells in which the gene was introduced and expressed were detected by FACS using an anti-FLAG tag antibody. According to the analysis method, as shown in FIG. 33, the efficiency of CD19 CAR gene delivery into CD3⁺ T cells was confirmed by gating in the following order: singlets → live cells → lymphocytes → CD3⁺ T cells → FLAG⁺ T cells. In addition, the proportion of CD8⁺ or CD4⁺ T cells in CD19 CAR-expressing T cells (CD3⁺FLAG⁺ T cells) was analyzed using CD8 and CD4 markers, and the proportions of memory-type T cells was also analyzed using CD45RA and CCR7 markers. When distinguishing the memory-type T cells using CD45RA and CCR7 markers, CD45RA⁺CCR7⁻ T cells were classified as effector T cells (Teff), CD45RA⁻CCR7⁻ T cells were classified as effector memory T cells (Tern), CD45RA⁻CCR7⁺ T cells were classified as central memory T cells (Tern), and CD45RA⁺CCR7⁺ T cells were classified as stem cell-like memory T cells (Tscm).

As shown in FIG. 33, seven days after electroporation, the proportion of FLAG-expressing cells among the CD3⁺ T cells was 0% in the EP only group, which is a negative control in which electroporation (EP) was only performed without a plasmid. On the other hand, in the 3M3-5M3 CD19 CAR group ('CD19 CAR+DNA') into which a CD19 CAR gene-containing transposon and a transposase vector were introduced, the proportion of FLAG-expressing cells was 65%. In addition, in the CD19 CAR + DNA group, the proportion of CD8⁺ T cells and CD4⁺ T cells in the FLAG-expressing T cells were 27% and 71%, respectively, confirming that there were relatively more CD4⁺ T cells. In addition, in the CD19 CAR + DNA group, the proportions of memory-type T cells in the FLAG-expressing T cells were 3% for Teff cells, 12% for Tem cells, 76% for Tcm cells, and 10% for Tscm cells, confirming that the Tcm cells were the most abundant, and Tscm cells were also present at approximately 10%.

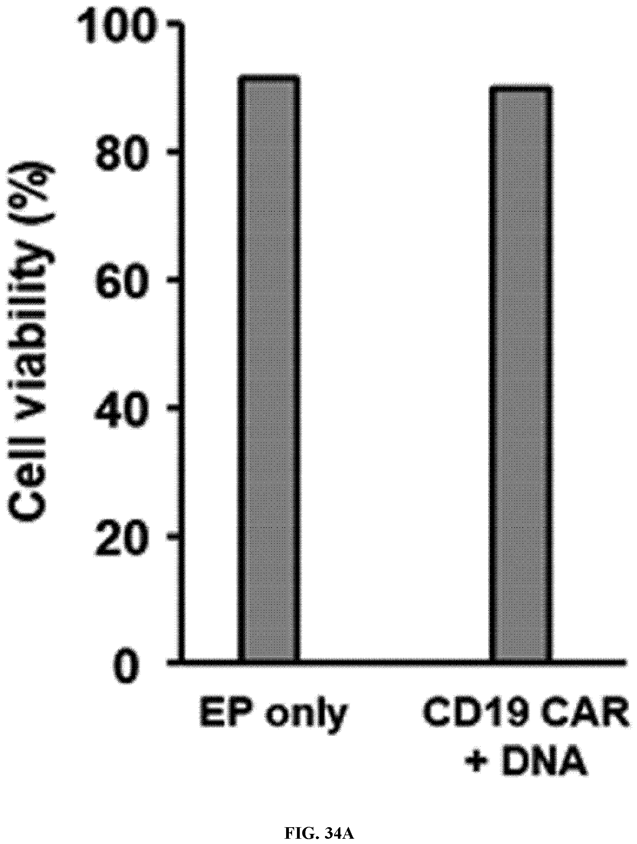

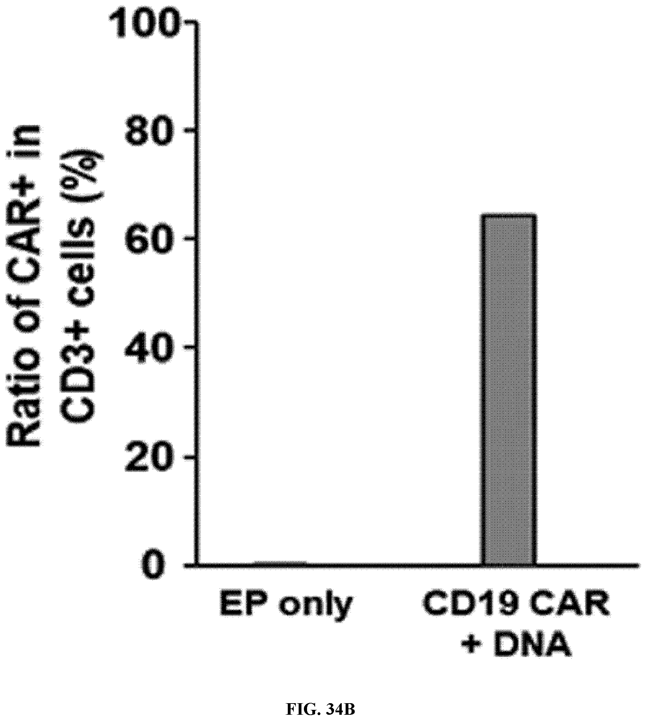

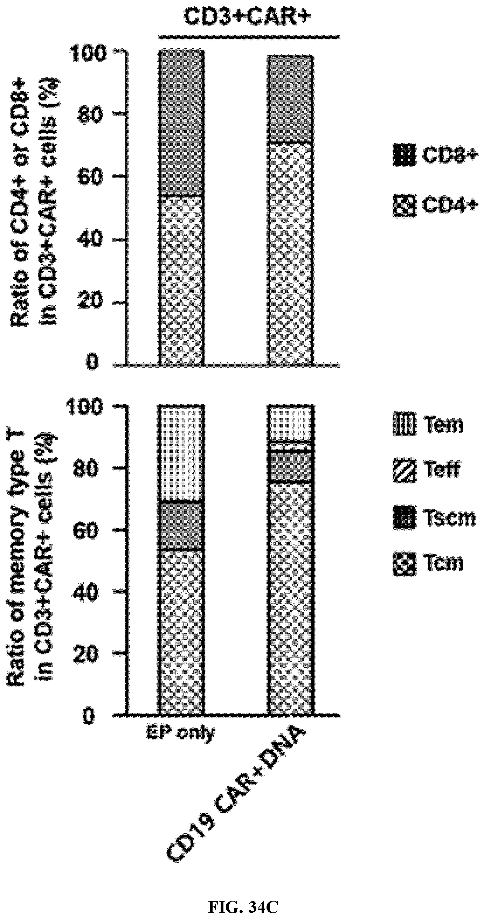

After electroporation of a CD19 CAR gene-containing transposon vector and a transposase vector, T cells were activated and then cell viability was measured on day 7 after electroporation. The results are shown in FIG. 34A, the results of confirming the proportion of CD19 CAR-expressing T cells is shown in FIG. 34B, and the results of confirming the proportions of memory-type T cells is shown in FIG. 34C.

From the above results, the transposon system according to the present invention can effectively delivery the CAR gene as well as the TCR gene into cells, and it was confirmed that the delivered gene was normally expressed. Accordingly, by using the transposon system of the present invention, it is expected that CAR-T cells can be produced at high yield.

### Experimental Example H. Confirmation of in vitro efficacy of CAR-T cells produced using pBat transposon system

According to the previous Experimental Example, it was confirmed that the pBat transposon system may effectively deliver TCR or CAR gene to target cells, and CAR-T cells can be produced with excellent yield using the system. Therefore, in this experimental example, it was examined whether the CAR-T cells produced by the pBat transposon system according to the present invention actually perform normal functions by checking the reactivity of the CAR-T cells produced by the transposon system to a target antigen.

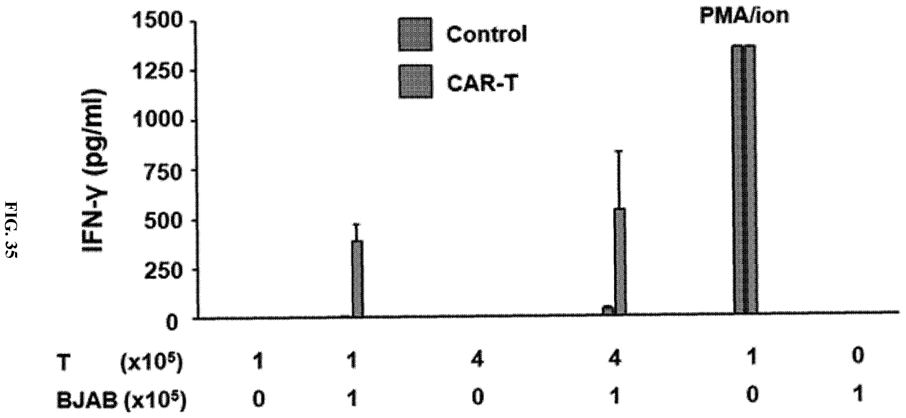

Specifically, after co-culturing control T cells or CD19 CAR-T cells with a BJAB cell line, that is, CD19-expressing B cells, for 24 hours, an IFN-γ ELISA assay was conducted with 100 µL of the culture solution to confirm the reactivity of the CAR-T cells to an antigen. As can be seen in FIG. 35, when the control T cells or BJAB cells are cultured alone, IFN-γ was not detected. In addition, in the group in which the control T cells and the BJAB cells were co-cultured, IFN-γ was detected, but its concentration was very low, 40 pg/mL or less. On the other hand, when the CD19 CAR-T cells produced by the transposon system according to the present invention and the BJAB cells were co-cultured, the IFN-γ concentration was found to significantly increase. Particularly, when the ratio of BJAB cells to CAR-T cells is 1:1, the average concentration of IFN-γ is 385 pg/mL, and when the ratio is 1:4, the average concentration is 535 pg/mL, indicating that the higher the proportion of CAR-T cells, the higher the IFN-γ concentration.

That is, even when the control T cells were co-cultured with the BJAB cells, IFN-γ was produced at a very low level, and the T cells did not show any significant reactivity to the antigen. However, when the CAR T cells produced by the transposon of the present invention were co-cultured with the BJAB cells, the IFN-γ level significantly increased, confirming that the CAR-T cells effectively recognized the antigen and were activated. The above results support the fact that the CAR-T cells showing excellent antigen reactivity can be produced using the transposon system of the present invention.

### Experimental Example 1. Confirmation of gene delivery efficiency according to delivery type of pBat transposon

According to the previous experimental example, it was confirmed that the transposon system according to the present invention can effectively deliver a gene and foreign gene-introduced cells such as CAR-T cells can be produced. Therefore, in the present experimental example, it was examined whether there are differences in gene delivery efficiency according to the type of transposon when using various types of transposons.

### 1. Observation of GFP expression in Jurkat cells 7 days after electroporation

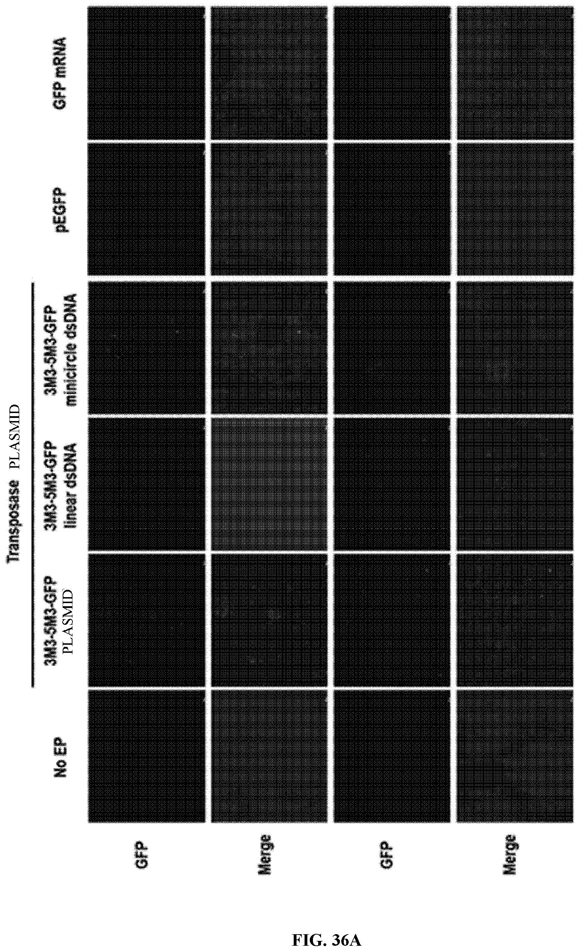

Seven days after introducing various transposons into cells through electroporation, whether GFP was expressed in a group in which a GFP-containing transposon was introduced was observed using a fluorescence microscope. As shown in FIG. 36A, in the No EP group (negative control) in which electroporation (EP) was not performed, GFP was not expressed like the results shown on day 1, and in the pEGFP group (positive control 1) in which a GFP was introduced, GFP was very weakly expressed, but in the GFP mRNA group (positive control 2) in which GFP mRNA was introduced, no GFP signal was detected. In the groups in which a GFP-containing transposon was introduced (3M3-5M3-GFP plasmid, 3M3-5M3-GFP linear dsDNA, 3M3-5M3-GFP minicircle dsDNA), weak GFP signals were detected.

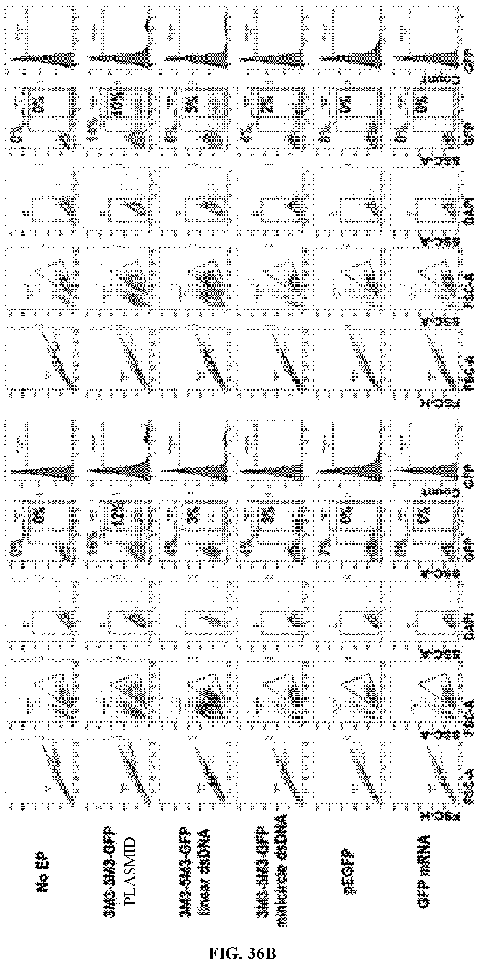

In addition, 7 days after electroporation, GFP expression in Jurkat cells was confirmed by FACS analysis. Specifically, as shown in FIG. 36B, the FACS analysis was performed by gating in the following order: singlets → cells → live cells → GFP⁺ cells. As a result of analyzing the proportion of GFP-expressing cells by setting a standard close to GFP⁻ (negative), as shown in FIG. 36B, in the No EP group, no GFP-expressing cells were observed, and the proportion of GFP-expressing cells was 8% in the pEGFP group, and 0% in the GFP mRNA group. In the case of the 3M3-5M3-GFP transposon-introduced groups, the proportion of GFP-expressing cells was 15% in the plasmid group, 5% in the linear dsDNA group, and 4% in the minicircle dsDNA group, confirming that the group in which the plasmid-type transposon was introduced shows the largest proportion of GFP-expressing cells.

In addition, because there were cells with high GFP intensity 7 days after electroporation, the proportions of GFP-expressing cells were compared by setting a standard close to high intensity. As a result of confirming the proportion of high intensity GFP-expressing cells according to the delivery type of the 3M3-5M3-GFP transposon, the proportion was 11% in the plasmid group, 4% in the linear dsDNA group, and 3% in the minicircle dsDNA group. That is, similar to the above-mentioned results, the group in which the plasmid-type transposon was introduced showed the highest GFP gene delivery efficiency.

### 2. Observation of GFP expression in Jurkat cells 14 days after electroporation

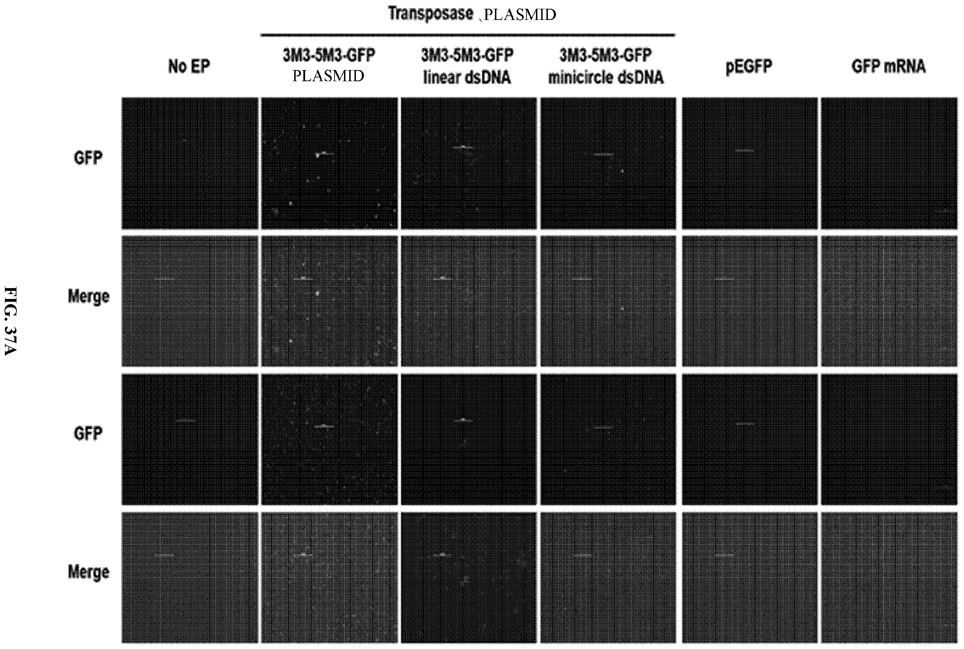

Subsequently, 14 days after electroporation, whether GFP was expressed in the group in which a GFP gene-containing transposon was introduced was observed using a fluorescence microscope. As shown in FIG. 37A, in the No EP group and the GFP mRNA group, GFP was not detected, and in the pEGFP group, GFP was hardly observed. On the other hand, in all of the 3M3-5M3-GFP transposon-introduced groups, high levels of GFP signals were detected. Particularly, it was confirmed that GFP was expressed at the highest level in the plasmid group, and relatively weakly expressed in the linear dsDNA group and the minicircle dsDNA group, compared to the plasmid group.

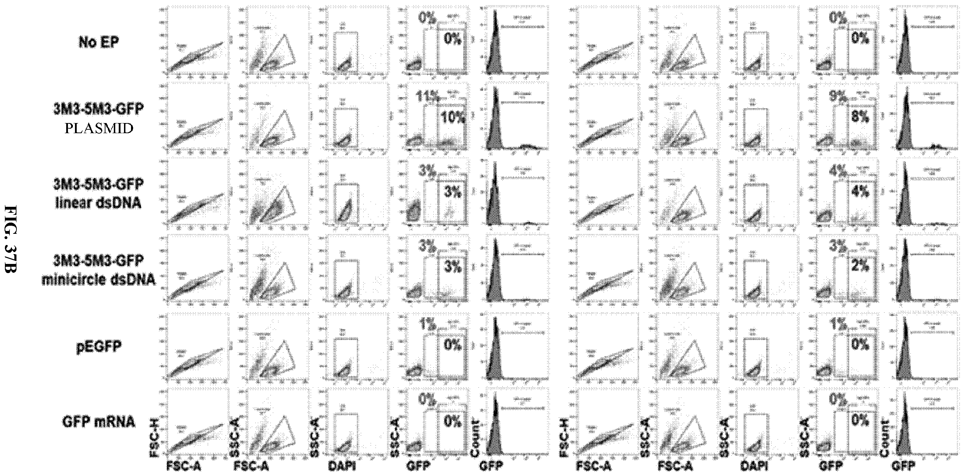

In addition, 14 days after electroporation, GFP expression in Jurkat cells was confirmed by FACS analysis. Specifically, as shown in FIG. 37B, FACS analysis was performed by gating in the following order: singlets → cells → live cells → GFP⁺ cells. As a result of analyzing the proportion of GFP-expressing cells by setting a standard close to GFP⁻ (negative), as shown in FIG. 37B, no GFP-expressing cells were observed in the No EP group, and GFP was hardly expressed in the pEGFP group and the GFP mRNA group. On the other hand, in the 3M3-5M3-GFP transposon-introduced groups, GFP expression was detected. Specifically, it was confirmed that the proportion of GFP-expressing cells was 10% in the plasmid group, 4% in the linear dsDNA group, and 3% in the minicircle dsDNA group, which were similar to the results and tendency on day 7. Particularly, it was confirmed that the proportion of GFP-expressing cells in the group in which the plasmid-type transposon was introduced was the highest.

Subsequently, as observed 7 days after electroporation, the proportions of GFP-expressing cells were compared by setting a standard close to high GFP intensity. The proportions of high intensity GFP-expressing cells according to the delivery type of the 3M3-5M3-GFP transposon were 9% in the plasmid group, 4% in the linear dsDNA group, and 3% in the minicircle dsDNA group, also confirming that the proportion was the highest in the group in which the plasmid-type transposon was introduced, and GFP-expressing cells were maintained as is even on day 7.

### 3. Comparison of GFP expression by period after electroporation

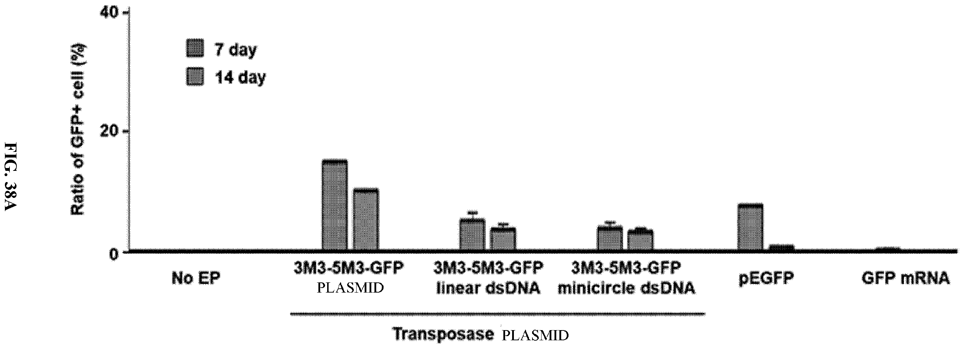

As a result of comparing the proportions of GFP-expressing cells in the GFP-transposon groups 7 days or 14 days after electroporation by setting a standard close to GFP⁻ (negative), the proportions ranged from 4% to 24% on day 7, and 3 to 19% after 14 days, confirming that the proportions of GFP-expressing cells gradually decreased (FIG. 38A). As a result of comparing the proportions of GFP-expressing cells according to the delivery type of the 3M3-5M3-GFP transposon, the proportion of GFP-expressing cells in the plasmid group was approximately 2.5-fold higher than those of the linear dsDNA group and the minicircle dsDNA group, demonstrating that gene delivery efficiency was best when the transposon was delivered in the form of a plasmid.

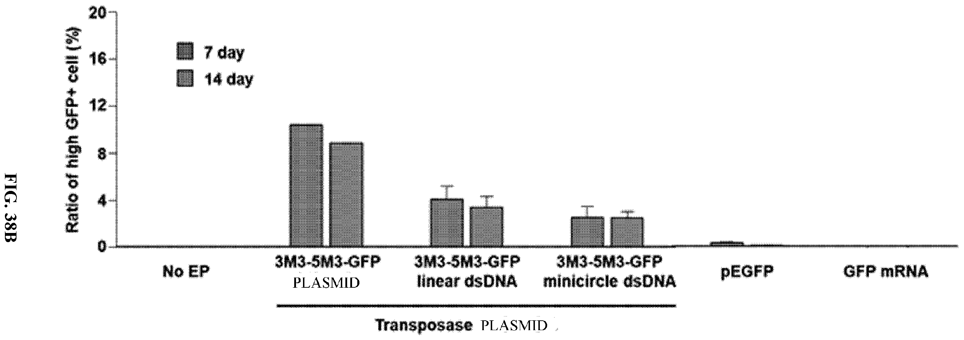

In addition, as a result of comparing the proportions of GFP-expressing cells in the GFP-transposon groups 7 days or 14 days after electroporation by setting a standard close to GFP high intensity, it was confirmed that the proportions ranged from 2 to 18% on day 7, and 2 to 16% after 14 days, confirming that the proportions of GFP-expressing cells were similar (FIG. 38B). The proportion of GFP-expressing cells according to the delivery type of the 3M3-5M3-GFP transposon in the plasmid group was approximately 2-fold higher than those in the linear dsDNA group and the minicircle dsDNA group, reconfirming that, when a transposon was delivered in the form of a plasmid, gene delivery efficiency was best. In addition, in all groups, after 7 days and 14 days, the proportions of cells exhibiting high GFP intensity were almost similar, demonstrating that Jurkat cells in which GFP gene was integrated into the chromosomes by a transposase on day 7 were maintained in the same state until day 14.

As above, as a result of comparing the gene delivery efficiency according to the delivery type of the 3M3-5M3-GFP transposon on day 7 and day 14 when a gene was inserted into the cell chromosome after electroporation and stably expressed, it was confirmed that all of minicircle DNA-, linear DNA-, and plasmid-type transposons effectively delivered a gene into target cells, and when the gene was delivered by a plasmid-type transposon, particularly, delivery efficiency was higher.

### Experimental Example J. Confirmation of antibody gene delivery efficiency by pBat transposon system

As a result of confirming the gene delivery and expression efficiency of the pBat transposon mutants through the above experimental examples, it was confirmed that the transposon system according to the present invention can effectively deliver a gene of a receptor protein such as TLR or a gene of a chimeric antibody such as CAR as well as GFP gene to target cells (Jurkat cells or PBMCs). In addition, the gene delivery efficiency of the 3M3-5M3 or 3M3-5M4 mutant transposon was found to be particularly high. Accordingly, it was intended to examine whether these improved mutant transposon vehicles can effectively deliver other antibody genes such as the JWW-2 antibody gene, and it was also intended to examine whether gene delivery and expression efficiency was high, particularly even in HEK293 cells, which were widely used in the mass production of proteins.

### 1. Observation of GFP expression by period after electroporation in HEK293 cells

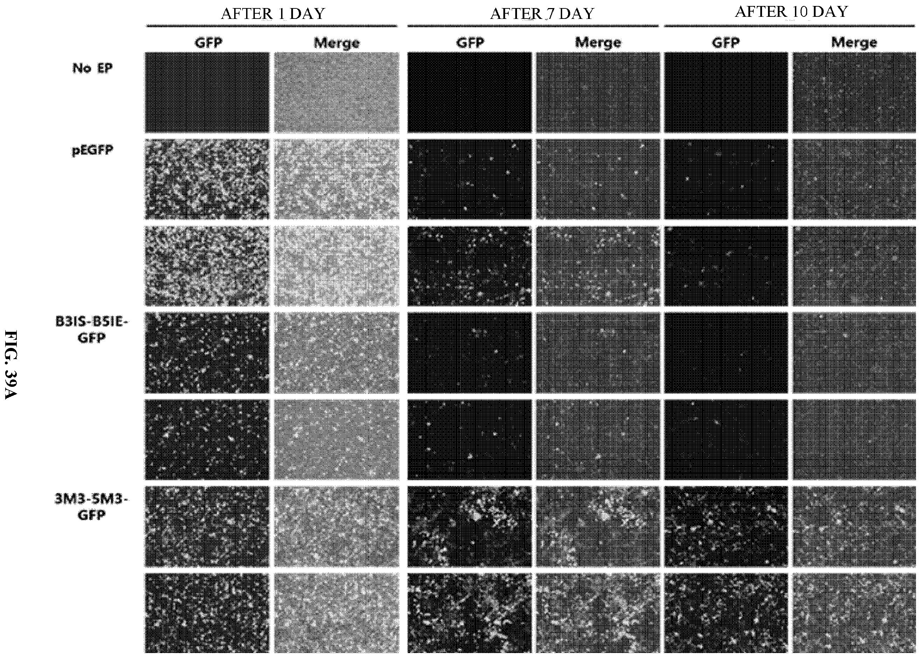

One day, 7 days, and 10 days after electroporation, GFP expression was observed using a fluorescence microscope. As a result of analysis, as shown in FIG. 39A, it was confirmed that, in the No EP group, which is a negative control in which only electroporation (EP) was performed without a plasmid, GFP expression was not observed at any time, and in a positive control (pEGFP group) in which electroporation was performed with a pEGFP plasmid, one day after electroporation, GFP was well expressed, but after 7 days and 10 days, GFP greatly decreased. As a result of confirming the GFP gene-containing transposon groups, it was confirmed that, GFP was well expressed on day 1 in the B3IS-B5IE-GFP group, but after 7 days and 10 days, GFP expression levels were slightly reduced. However, in the group treated with a mutant transposon 3M3-5M3-GFP (3M3-5M3-GFP group), it was confirmed that high levels of GFP expression were shown throughout the entire period from after one day to after 10 days. The above results show that the transposon system according to the present invention exhibits an excellent gene delivery function.

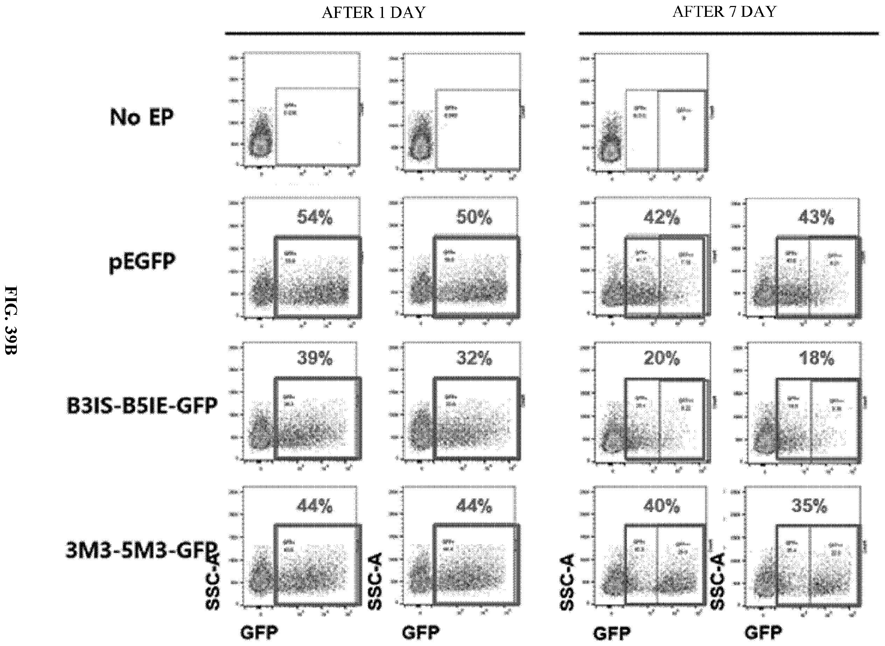

In addition, as a result of confirming GFP expression in HEK293 cells through FACS analysis, as shown in FIG. 39B, it was confirmed that one day after electroporation, in the No EP group, no GFP was expressed, and the proportion of GFP-expressing cells was approximately 52% on average in the pEGFP group, approximately 36% in the B3IS-B5IE-GFP group, and approximately 44% in the 3M3-5M3-GFP group. In addition, 7 days after electroporation, as a result of measuring the proportion of GFP-expressing cells again, the proportion was approximately 43% on average in the pEGFP group, approximately 19% in the B3IS-B5IE-GFP group, and approximately 38% in the 3M3-5M3-GFP group. Particularly, it was confirmed that the B3IS-B5IE-GFP group showed weak GFP intensity, but the 3M3-5M3-GFP group showed a relatively higher GFP expression intensity and a large proportion of expressing cells.

### 2. Observation of mRNA expression of JWW antibody after electroporation in HEK293 cells

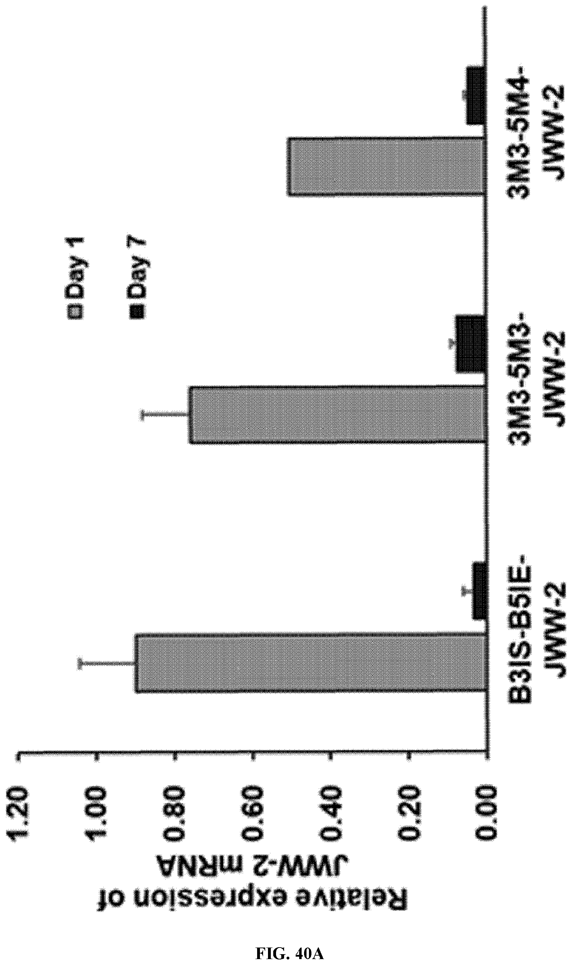

One day and 7 days after electroporation, the mRNA expression of JWW-2 was confirmed through qPCR by isolating total RNA from HEK293 cells. As a result of the analysis, as shown in FIG. 40A, it was confirmed that the JWW-2 gene was amplified only in the JWW-2 gene-containing transposon group. One day after electroporation, the mRNA expression of the JWW-2 gene was highest in the order of B3IS-B5IE-JWW-2 group, the 3M3-5M3-JWW-2 group, and the 3M3-5M4-JWW-2 group. However, 7 days after electroporation, it was confirmed that the level of JWW-2 mRNA expression was highest in the order of the 3M3-5M3-JWW-2 group, the 3M3-5M4-JWW-2 group, and the B3IS-B5IE-JWW-2 group.

### 3. Observation of protein expression of JWW antibody after electroporation in HEK293 cells

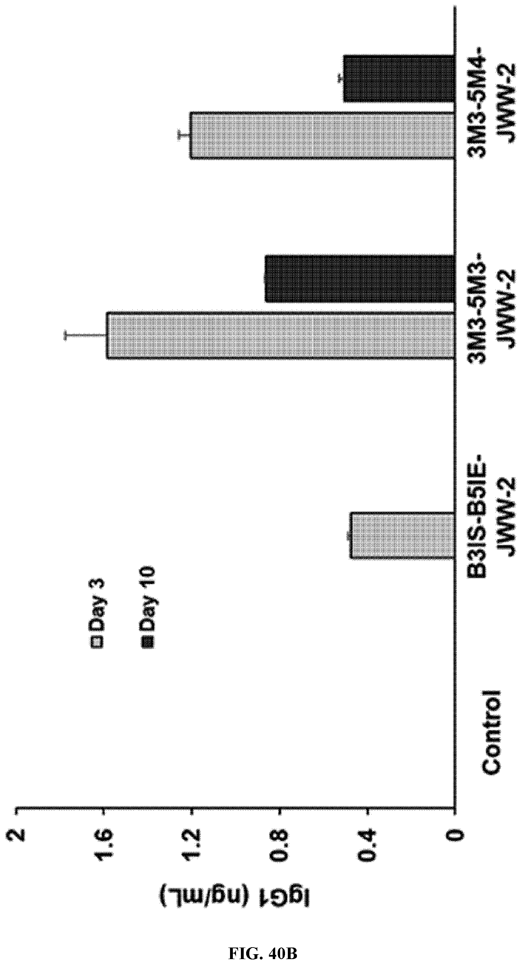

Three days and 10 days after electroporation, a medium in which HEK293 cells were cultured was recovered, and the JWW-2 antibody protein secreted from the HEK293 cells was quantified. Specifically, an ELISA assay using an antibody targeting human IgG1, which is the Fc region of the JWW-2 antibody, was performed. As shown in FIG. 40B, three days after electroporation, in all transposon groups containing the JWW-2 antibody gene, except the EP only group, human IgG1 was detected. Particularly, it was confirmed that, in the 3M3-5M3-JWW-2 group, the levels of JWW-2 protein were the highest of 1.6 ng/mL three days after electroporation, and 0.9 ng/µL 10 days after electroporation, and in the 3M3-5M4-JWW-2 group, the levels of the JWW-2 protein were 1.2 ng/mL 3 days after electroporation, and 0.5 ng/mL 10 days after electroporation. In addition, it was confirmed that, in the B3IS-B5IE-JWW-2 group, the levels of JWW-2 protein were 0.5 ng/mL and 0 ng/mL 3 days and 10 days after electroporation, respectively. Accordingly, the cells that deliver the JWW-2 gene using the transposon system according to the present invention all express the JWW-2 antibody at high levels, and particularly, it was confirmed that the gene delivery efficiency was the highest in the groups using mutant transposons.

In the previous experimental example, as a result of confirming the gene delivery and expression efficiency research for pBat transposon mutants using the GFP gene, it was confirmed that all of the transposon systems according to the present invention showed an excellent gene delivery function, and particularly, the high gene delivery efficiency of the 3M3-5M3 or 3M3-5M4 mutant transposon was confirmed in Jurkat cells. Accordingly, it was intended to confirm whether the improved mutant transposon vehicle exhibited high gene delivery and expression efficiency for the JWW-2 antibody gene, not the GFP gene, in HEK293 cells widely used for protein production, rather than Jurkat cells. As described above, it was confirmed that, 7 days and 10 days after electroporation, the proportion of GFP-expressing cells is higher in the case of the 3M3-5M3-GFP transposon vector compared to the B3IS-B5IE-GFP transposon vector, confirming that the gene delivery efficiency of mutant transposons was high in HEK293 cells as well as Jurkat cells.

In addition, one day after electroporation, since the expression of JWW-2 mRNA was transient, the JWW-2 mRNA was highly expressed in all of the B3IS-B5IE-JWW-2, 3M3-5M3-JWW-2, and 3M3-5M4-JWW-2 groups. However, it was confirmed that the expression rate 7 days after electroporation in which the JWW-2 mRNA was stably expressed by integrating the JWW-2 gene into the chromosomes of HEK293 cells by a transposase was lower than the transient expression rate on day 1, but expression was maintained. It was observed that, in the case of the 3M3-5M3-JWW-2 transposon vector, which is a mutant transposon vector, compared to the B3IS-B5IE-JWW-2 transposon vector, the mRNA level of JWW-2 expressed by integration into the chromosomes was slightly higher. In addition, in terms of the protein expression of the JWW-2 antibody, the concentration of the JWW-2 antibody protein expressed by insertion into chromosomes was higher in the case of the 3M3-5M3-JWW-2 and 3M3-5M4-JWW-2 transposon vectors, which are mutant transposon vectors, compared to the B3IS-B5IE-JWW-2 transposon vector, which were observed in the same way 3 days and 10 days after electroporation. Particularly, it was confirmed that the concentration of the JWW-2 antibody protein was the highest in the case of the 3M3-5M3-JWW-2 transposon vector. Therefore, it was confirmed that the improved transposon vectors (3M3-5M3 and 3M3-5M4) produced by ITR mutations along with the antigen gene were delivered into target cells and inserted into chromosomes, and also induce the expression of an antibody protein.

### Experimental Example K. Confirmation of gene delivery efficiency according to size of pBat transposon vector

Through the previous experimental examples, it was confirmed that the transposon vector according to the present invention can effectively deliver various genes such as an antibody gene, a receptor gene, and a CAR gene into target cells and induce the expression thereof.

When DNA is delivered into cells, generally, it was known that the smaller the size of DNA, the higher the delivery efficiency into nuclei (McLenachan et al., 2007). Accordingly, to confirm whether the size of the vector affects the gene delivery efficiency of the transposon system of the present invention, after minimizing the size of the transposon vector, it was confirmed whether gene delivery efficiency increased. To minimize the size of the transposon vector, IRES and a puromycin resistance gene were removed, one ori site was removed to reduce a total of two ori sites to one, a CAG promoter was changed to an EF1α promoter, and for use in clinical trials, an ampicillin resistance gene was changed to a kanamycin resistance gene. As a result, the size of the transposon vector of the present invention was reduced from the original size of 7,562 bp to 4,149 bp.

### 1. Observation of GFP expression in Jurkat cells one day after electroporation

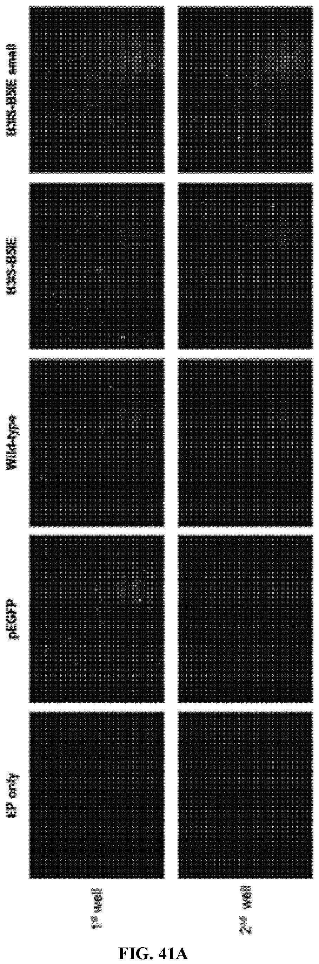

GFP expression one day after electroporation was observed using a fluorescence microscope. As a result, as shown in FIG. 41A, it was confirmed that GFP was expressed in all groups except the EP only group in which electroporation (EP) was performed without a plasmid.

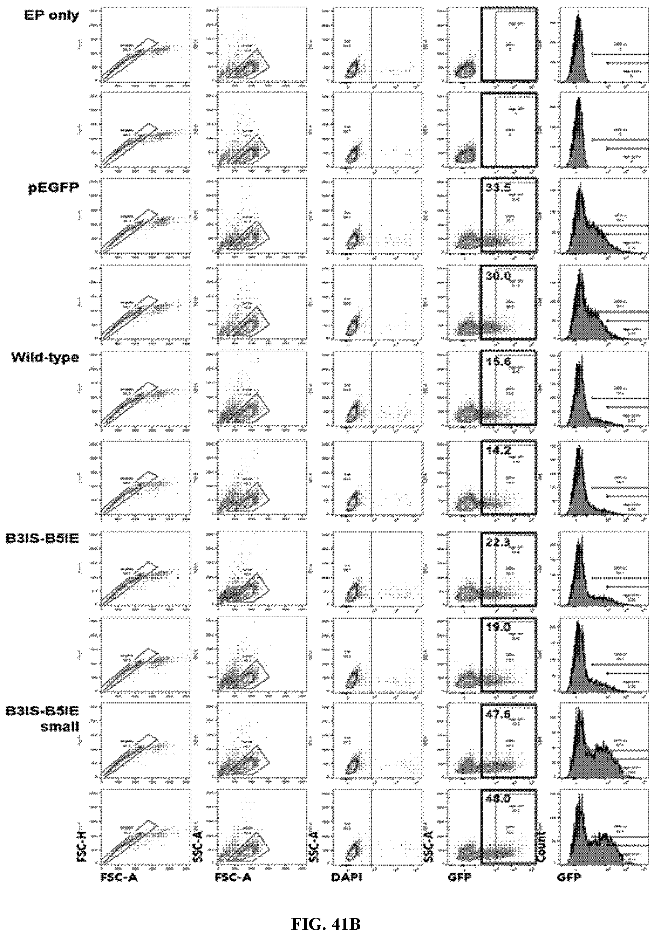

In addition, the GFP expression in Jurkat cells one day after electroporation was confirmed by FACS analysis. As shown in FIG. 41B, the GFP expression was analyzed by gating in the following order: singlets → cells → live cells → GFP+ cells. As a result of the analysis, it was confirmed that, in the EP only group, no GFP was observed, and in the pEGFP group, the proportion of GFP expression cells was approximately 31.8%. In the groups in which a pBat transposon vector and a transposase vector were electroporated, there was a difference in GFP expression according to the size of a transposon vector, and in the group (wild-type) in which the wild-type transposon vector was electroporated, the proportion of GFP-expressing cells was 14.9%, and in the group (B3IS-B5IE) in which a vector with enzyme sites added to the genes outside the 5'ITR and 3'ITR of the transposon vector was electroporated, gene expression was 20.7%, and in the group (B3IS-B5IE small) in which a transposon vector reduced in size from 7,562 bp to 4,149 bp was electroporated, GFP expression was 47.8%. That is, it was confirmed that, when the size of the transposon vector is small, the proportion of GFP-expressing cells was high.

### 2. Observation of GFP expression in Jurkat cells 7 days after electroporation

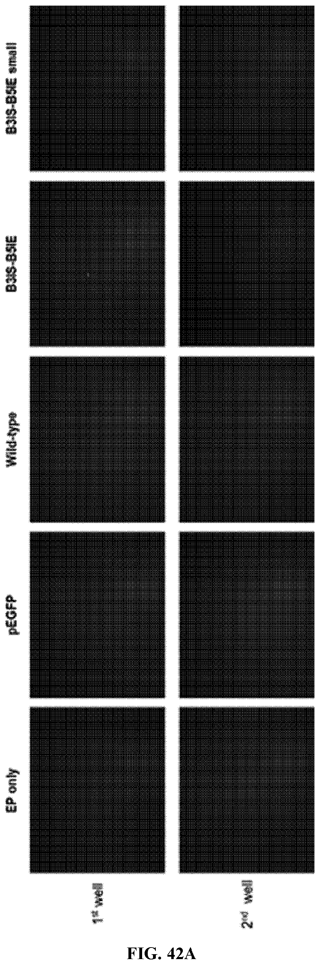

GFP expression 7 days after electroporation was observed using a fluorescence microscope. As a result, as shown in FIG. 42A, in all groups except the EP only group, it was confirmed that GFP was expressed (FIG. 42A).

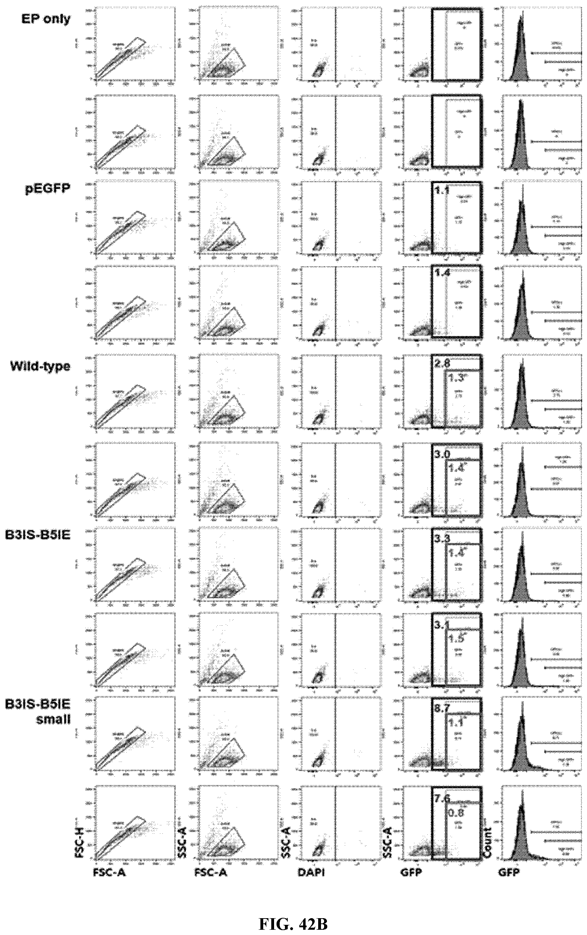

As a result of confirming the GFP expression in Jurkat cells 7 days after electroporation through FACS analysis (FIG. 42B), it was confirmed that no GFP was observed in the EP only group, and the proportion of GFP-expressing cells was approximately 1.3% in the pEGFP group. On the other hand, it was confirmed that the proportion of GFP-expressing cells in the wild-type group was 2.9%, 3.2% in the B3IS-B5IE group, 8.2% in the B3IS-B5IE small group, indicating that the GFP expression levels of these groups were higher than that of the control. Particularly, in the B3IS-B5IE small group in which the size of transposon vector is small, the proportion of GFP-expressing cells was high.

### 3. Observation of GFP expression in Jurkat cells 14 days after electroporation

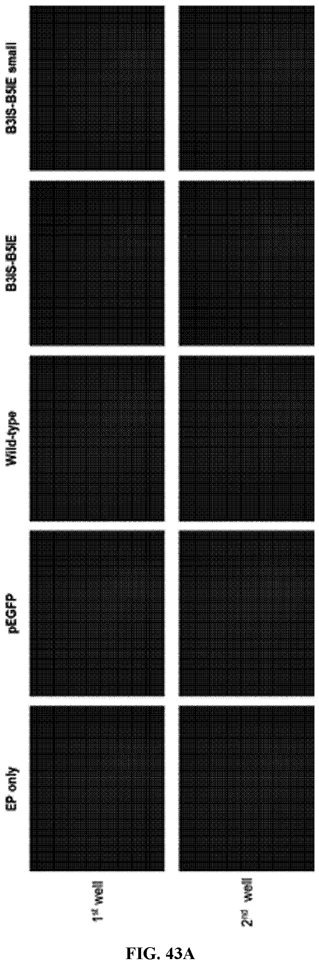

GFP expression 14 days after electroporation was observed using a fluorescence microscope. As a result, as shown in FIG. 43A, in the EP only group and the pEGFP group, no GFP expression was observed, and in all groups using transposons, GFP-expressing cells were detected.

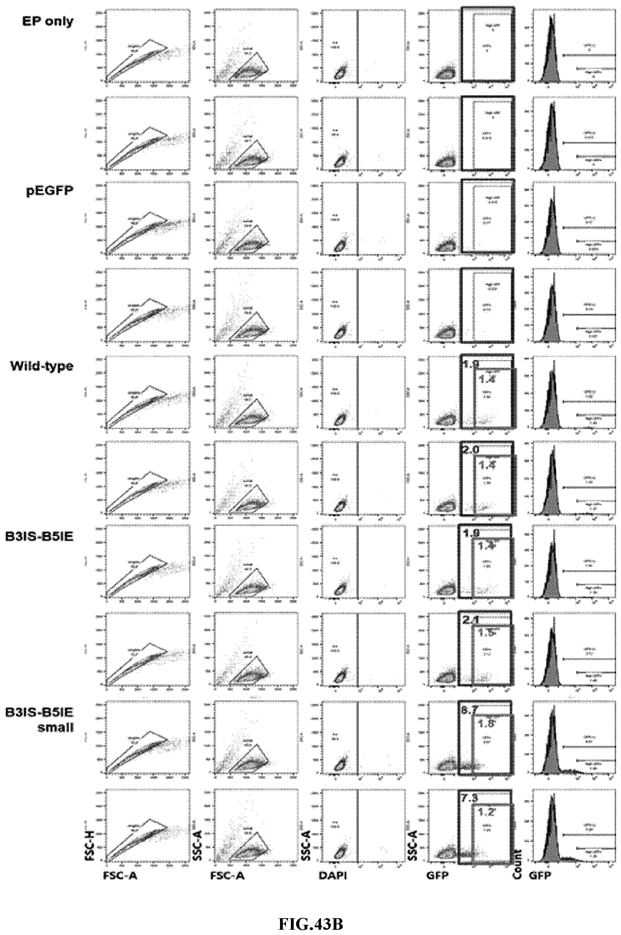

GFP expression in Jurkat cells 14 days after electroporation was confirmed through FACS analysis (FIG. 43B). As a result of the analysis, no GFP expression was observed in the EP only group and the pEGFP group. On the other hand, in all of the groups using transposons, GFP expression was detected, showing that the gene is integrated into the chromosomes of the cells by a transposon and stably expressed. Particularly, the proportion of GFP-expressing cells was 2.0% in the wild-type group, and 2.0% in the B3IS-B5IE group, but 8.0% in the B3IS-B5IE small group, confirming that the smaller the size of a transposon vector, the larger the proportion of GFP-expressing cells.

### 4. Comparison of GFP expression rate in Jurkat cells

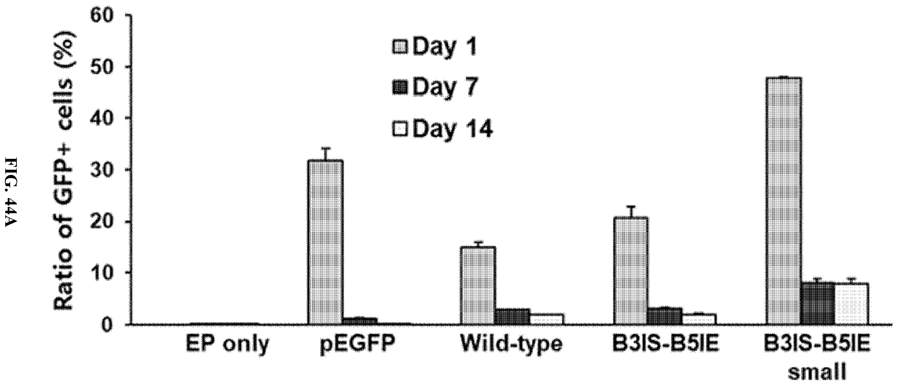

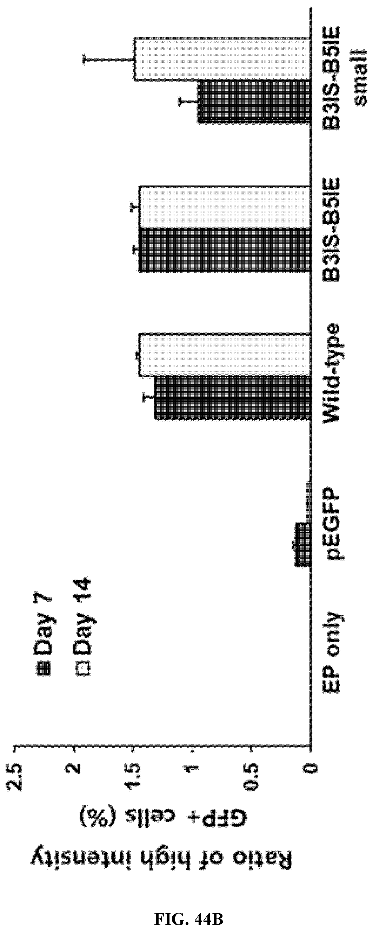

Comparing the proportions of GFP-expressing cells over time after electroporation, as shown in FIG. 44A, in all groups, there is a tendency to gradually decrease from 10 to 32% on day 1 to 4 to 17% on day 7 to 0 to 10% on day 14. However, it was confirmed that, in all of the transposon-treated groups, even 14 days after electroporation, the GFP gene was stably expressed. Particularly, it was confirmed that, on days 1, 7, and 14, in the B3IS-B5IE small group in which the size of transposon vector is small, the proportion of GFP-expressing cells was the highest. In addition, it was confirmed that, the proportion of high GFP intensity-expressing cells was also significantly high in the groups using transposons compared to the control (FIG. 44B).

As can be seen above, until 14 days after electroporation, in the group treated with a small-sized transposon vector, the proportion of target gene (GFP)-expressing cells was the highest. Specifically, 7 days and 14 days after electroporation when the GFP gene was integrated into the chromosomes of Jurkat cells and stably expressed, in the B3IS-B5IE small group using a relatively small-sized vector, compared to the B3IS-B5IE group, the proportion of GFP-expressing cells was increased two- and four-fold, respectively. This demonstrates that the smaller the size of the transposon vector, the higher the gene delivery efficiency of the pBat transposon.

Table 20 shows the information on main sequences described in the specification.

**[Table 20]**

| **Classification** | **Sequence of sense strand (5' → 3')** | **SEQ ID NO:** |
|---|---|---|
| 85IE | ttaacacttggattgcgggaaacgagttaagtcggctcgcgtgaattgcgcgtactccgcgggagccgtcttaa ctcggttcatatagatttgcggtggagtgcgggaaacgtgtaaactcgggccgattgtaactgcgtattaccaa [?] atatttgtt | |
| B3IS | aattatttatgtactgaatagataaaaaaatgtctgtgattgaataaattttcattttttacacaagaaaccgaaaattt catttcaatcgaacccatacttcaaaagatataggcattttaaactaactctgattttgcgcgggaaacctaaataa[?] ttgcccgcgccatcttatattttggcgggaaattcacccgacaccgtagtgttaa | |
| 5M1 (13mer) | ttaacacttggat | 3 |
| 5M2 (33mer) | ttaacacttggattgcgggaaacgagttaagtc | 4 |
| 5M3 (71mer) | ttaacacttggattgcgggaaacgagttaagtcggctcgcgtgaattgcgcgtactccgcgggagccgtct | 5 |
| 5M4 (110mer) | ttaacacttggattgcgggaaacgagttaagtcggctcgcgtgaattgcgcgtactccgcgggagccgtcttaa [?] ctcggttcatatagatttgcggtggagtgcgggaaa | |
| 5' ITR_26 | ttaacacttggattgcgggaaacgag | 7 |
| 5' ITR_8 | acacttgg | Not numbered |
| 5' ITR_15 (ITR) | tgcgggaaacgagtt | 8 |
| 3M1 (66mer) | aacctaaataattgcccgcgccatcttatattttggcgggaaattcacccgacaccgtagtgttaa | 9 |
| 3M2 (91mer) | aaactaactctgattttgcgcgggaaacctaaataattgcccgcgccatcttatattttggcgggaaattcacccg acaccgtagtgttaa | 10 |
| 3M3 (151 mer) | cacaagaaaccgaaaatttcatttcaatcgaacccatacttcaaaagatataggcattttaaactaactctgatttt gcgcgggaaacctaaataattgcccgcgccatcttatattttggcgggaaattcacccgacaccgtagtgttaa | 11 |
| 3M4 (37 mer) | attttggcgggaaattcacccgacaccgtagtgttaa | 12 |
| 3' ITR_30 | ttggcgggaaattcacccgacaccgtagtg | 13 |
| 3' ITR_18 | aactctgattttgcgcgg | 14 |
| r3M1 | ttaacactacggtgtcgggtgaatttcccgccaaaatataagatggcgcgggcaattatttaggtt | 15 |
| r3M2 | ttaacactacggtgtcgggtgaatttcccgccaaaatataagatggcgcgggcaattatttaggtttcccgcgca [?] aaatcagagttagttt | 16 |
| r3M3 | ttaacactacggtgtcgggtgaatttcccgccaaaatataagatggcgcgggcaattatttaggtttcccgcgca aaatcagagttagtttaaaatgcctatatcttttgaagtatgggttcgattgaaatgaaattttcggtttcttgtgt | 17 |
| Transposase amino acid | Met Ser Gln His Ser Asp Tyr Ser Asp Asp Glu Phe Cys Ala Asp Lys Leu Ser Asn Tyr Ser Cys Asp Ser Asp Leu Glu Asn Ala Ser Thr Ser Asp Glu Asp Ser Ser Asp Asp Glu Val Met Val Arg Pro Arg Thr Leu Arg Arg Arg Arg Ile Ser Ser Ser Ser Ser Asp Ser Glu Ser Asp Ile Glu Gly Gly Arg Glu Glu Trp Ser His Val Asp Asn Pro Pro Val Leu Glu Asp Phe Leu Gly His Gln Gly Leu Asn Thr Asp Ala Val Ile Asn Asn Ile Glu Asp Ala Val Lys Leu Phe Ile Gly Asp Asp Phe Phe Glu Phe Leu Val Glu Glu Ser Asn Arg Tyr Tyr Asn Gln Asn Arg Asn Asn Phe Lys Leu Ser Lys Lys Ser Leu Lys Trp Lys Asp Ile Thr Pro Gln Glu Met Lys Lys Phe Leu Gly Leu Ile Val Leu Met Gly Gln Val Arg Lys Asp Arg Arg Asp Asp Tyr Trp Thr Thr Glu Pro Trp Thr Glu Thr Pro Tyr Phe Gly Lys Thr Met Thr Arg Asp Arg Phe Arg Gln Ile Trp Lys Ala Trp His Phe Asn Asn Asn Ala Asp Ile Val Asn Glu Ser Asp Arg Leu Cys Lys Val Arg Pro Val Leu Asp Tyr Phe Val Pro Lys Phe Ile Asn Ile Tyr Lys Pro His Gln Gln Leu Ser Leu Asp Glu Gly Ile Val Pro Trp Arg Gly Arg Leu Phe Phe Arg Val Tyr Asn Ala Gly Lys Ile Val Lys Tyr Gly Ile Leu Val Arg Leu Leu Cys Glu Ser Asp Thr Gly Tyr Ile Cys Asn Met Glu Ile Tyr Cys Gly Glu Gly Lys Arg Leu Leu Glu Thr Ile Gln Thr Val Val Ser Pro Tyr Thr Asp Ser Trp Tyr His Ile Tyr Met Asp Asn Tyr Tyr Asn Ser Val Ala Asn Cys Glu Ala Leu Met Lys Asn Lys Phe Arg Ile Cys Gly Thr Ile Arg Lys Asn Arg Gly Ile Pro Lys Asp Phe Gln Thr Ile Ser Leu Lys Lys Gly Glu Thr Lys Phe Ile Arg Lys Asn Asp Ile Leu Leu Gln Val Trp Gln Ser Lys Lys Pro Val Tyr Leu Ile Ser Ser Ile His Ser Ala Glu Met Glu Glu Ser Gln Asn Ile Asp Arg Thr Ser Lys Lys Lys Ile Val Lys Pro Asn Ala Leu Ile Asp Tyr Asn Lys His Met Lys Gly Val Asp Arg Ala Asp Gln Tyr Leu Ser Tyr Tyr Ser Ile Leu Arg Arg Thr Val Lys Trp Thr Lys Arg Leu Ala Met Tyr Met Ile Asn Cys Ala Leu Phe Asn Ser Tyr Ala Val Tyr Lys Ser Val Arg Gln Arg Lys Met Gly Phe Lys Met Phe Leu Lys Gln Thr Ala Ile His Trp Leu Thr Asp Asp Ile Pro Glu Asp Met Asp Ile Val Pro Asp Leu Gln Pro Val Pro Ser Thr Ser Gly Met Arg Ala Lys Pro Pro Thr Ser Asp Pro Pro Cys Arg Leu Ser Met Asp Met Arg Lys His Thr Leu | 18 |
| | Gln Ala Ile Val Gly Ser Gly Lys Lys Lys Asn Ile Leu Arg Arg Cys Arg Val Cys Ser Val His Lys Leu Arg Ser Glu Thr Arg Tyr Met Cys Lys Phe Cys Asn Ile Pro Leu His Lys Gly Ala Cys Phe Glu Lys Tyr His Thr Leu Lys Asn Tyr | |
| Transposase DNA | atttccagaccatctccctgaaaaagggtgaaactaagttcattcgcaaaaacgacatcctcctgcaagtctgg cagtctaaaaagcctgtatatctgatctcatctattcacagcgctgaaatggaagaatctcagaacattgatcgca cctccaagaaaaagatcgtcaaaccgaatgcattgattgattacaacaagcacatgaagggcgttgatcgtgct gaccagtacctgtcttattactctatcctgcgccgtactgtgaagtggactaaacgtctcgctatgtacatgattaa ttgtgcgctgttcaattcttacgctgtgtataaaagcgtgcgtcagcgcaaaatgggctttaaaatgttcctgaag cagacggctattcactggctgaccgacgatattccggaagatatggacattgtcccggatctccagccggtac cgagcaccagcggtatgcgtgctaaacctccgactagtgatccgccttgccgtctgtctatggatatgcgtaag cataccctgcaggcaattgtgggctctggcaaaaagaaaaatatcctgcgtcgttgccgcgtatgctctgtaca caaactgcgttctgagactcgttatatgtgtaaattttgcaatattccactccacaagggtgcgtgcttcgagaagt accatacgctgaagaactat | 19 |
| Transposase mRNA | auuuccagaccaucucccugaaaaagggugaaacuaaguucauucgcaaaaacgacauccuccugca agucuggcagucuaaaaagccuguauaucugaucucaucuauucacagcgcugaaauggaagaauc ucagaacauugaucgcaccuccaagaaaaagaucgucaaaccgaaugcauugauugauuacaacaag cacaugaagggcguugaucgugcugaccaguaccugucuuauuacucuauccugcgccguacugu gaaguggacuaaacgucucgcuauguacaugauuaauugugcgcuguucaauucuuacgcugugu auaaaagcgugcgucagcgcaaaaugggcuuuaaaauguuccugaagcagacggcuauucacuggc[?] ugaccgacgauauuccggaagauauggacauugucccggaucuccagccgguaccgagcaccagcg guaugcgugcuaaaccuccgacuagugauccgccuugccgucugucuauggauaugcguaagcau acccugcaggcaauugugggcucuggcaaaaagaaaaauauccugcgucguugccgcguaugcucu guacacaaacugcguucugagacucguuauauguguaaauuuugcaauauuccacuccacaagggu gcgugcuucgagaaguaccauacgcugaagaacuau | 20 |

### [Industrial Applicability]

The present invention relates to a transposon vector, a transposon system including the same, a transposon kit, a cell into which the transposon vector is inserted, and uses thereof, which were completed by confirming that an exogenous gene was effectively delivered to the chromosome of a target cell to produce genetically modified cells at high yield. Particularly, the transposon according to the present invention may effectively deliver a TCR- or CAR-encoding gene to immune cells, and through this, it was confirmed that the TCR- or CAR-expressing cells showed high reactivity to an antigen. Therefore, it is expected that various TCR-T cells and CAR-T cells can be produced using the transposon system according to the present invention. Particularly, conventional CAR-T cells require high costs for CAR production and delivery to target cells, but by using the transposon of the present invention, high-yield CAR-T cells can be obtained at low cost, the price of a CAR-T cell therapeutic is lowered, which reduces the production cost of CAR-T cell therapeutics. In addition, it was confirmed that the transposon of the present invention can effectively deliver an antibody gene such as a tumor virus-targeted neutralizing antibody gene to HEK293 cells used in the mass production of antibodies, various antibodies can be easily produced in large quantities using the transposon of the present invention. Particularly, since the transposon according to the present invention is a gene delivery vehicle and is not limited to the type of gene that can be delivered, it is expected to be actively used in the development of genome-modified cell lines expressing various genes depending on the purpose, in addition to an antibody gene.

## Claims

1. A transposon vector, comprising:
a 5' ITR having a sequence of at least 71 consecutive nucleotides in the nucleotide sequence represented by SEQ ID NO: 1; and a 3' ITR having a sequence of at least 66 consecutive nucleotides in the nucleotide sequence represented by SEQ ID NO: 2.

2. The transposon vector of claim 1, wherein the 5' ITR is selected from the following:
a 5' ITR having the nucleotide sequence represented by SEQ ID NO: 1;
a 5' ITR having the nucleotide sequence represented by SEQ ID NO: 5; or
a 5' ITR having the nucleotide sequence represented by SEQ ID NO: 6, and
the 3' ITR is selected from the following:
a 3' ITR having the nucleotide sequence represented by SEQ ID NO: 2;
a 3' ITR having the nucleotide sequence represented by SEQ ID NO: 9;
a 3' ITR having the nucleotide sequence represented by SEQ ID NO: 10; or
a 3' ITR having the nucleotide sequence represented by SEQ ID NO: 11.

3. The transposon vector of claim 1 or 2, wherein the 5' ITR includes one or more of the nucleotide sequences represented by SEQ ID NO: 7, 5'-ACACTTGG-3', and SEQ ID NO: 8.

4. The transposon vector of claim 1 or 2, wherein the 3' ITR includes one or more of the nucleotide sequences represented by SEQ ID NO: 13 and SEQ ID NO: 14.

5. The transposon vector of claim 1 or 2, wherein the nucleotide sequence of the 5' ITR is included upstream of a position where target DNA is inserted into the transposon vector in the 5' to 3' direction, or
the nucleotide sequence of the 3' ITR is included downstream of a position where the target DNA is inserted into the transposon vector in the 5' to 3' direction.

6. The transposon vector of claim 1, wherein the transposon vector includes antisense DNA having a reverse complement sequence of the nucleotide sequence of the 3' ITR in the 5' to 3' direction downstream of the position where target DNA is inserted into the transposon vector.

7. The transposon vector of claim 6, wherein the reverse complement sequence of the 3' ITR includes the nucleotide sequence represented by any one of SEQ ID NOs: 15 to 17.

8. The transposon vector of claim 1, wherein the transposon vector includes one or more target DNA sequences downstream of the 5' ITR and upstream of the 3' ITR.

9. The transposon vector of claim 8, wherein the target DNA sequence is any one or more selected from the group consisting of a therapeutic polypeptide coding sequence, an siRNA coding sequence, an miRNA coding sequence, a reporter protein coding sequence, an antigen-specific receptor coding sequence, a recombinant antibody coding sequence or a fragment thereof, a neutralizing antibody coding sequence or a fragment thereof, an immune checkpoint inhibitor coding sequence, a cytokine receptor coding sequence, a CAR coding sequence or a fragment thereof, and a T-cell receptor (TCR) coding sequence or a fragment thereof.

10. The transposon vector of claim 1, wherein the transposon vector includes a promoter, one or more target DNAs, and a poly(A) signal, in which the 5' ITR, the promoter, the target DNA, the poly(A) signal, and the 3' ITR are sequentially operably linked.

11. The transposon vector of claim 1, wherein the transposon vector is a circular plasmid, linearized double stranded DNA (dsDNA), hairpin dsDNA, or minicircle dsDNA.

12. The transposon vector of claim 1, wherein the transposon vector has a size of 1,000 to 20,000 bp.

13. A transposon system for delivering target DNA, comprising:
a) the transposon vector of claim 1 into which target DNA is inserted; and
b) a nucleic acid molecule including a sequence encoding a transposase protein or transposase.

14. The transposon system of claim 13, wherein the transposase protein includes the amino acid sequence represented by SEQ ID NO: 18.

15. A transposon kit for delivering target DNA, comprising:
the transposon system for delivering target DNA of claim 13, and instructions.

16. A cell into which a) the transposon vector of claim 1 into which target DNA is inserted; and
b) a nucleic acid molecule including a sequence encoding a transposase protein or transposase are introduced.

17. The cell of claim 16, wherein the target DNA is cleaved from the transposon vector by the transposase, and the cleaved target DNA is integrated into the genome of the cell.

18. The cell of claim 16, wherein the cell is selected from the group consisting of a T cell, an NK cell, a B cell, a dendritic cell, a macrophage, and a mast cell.

19. The cell of claim 16, wherein the cell is co-cultured with feeder cells after introducing the transposon vector.

20. The cell of claim 19, wherein the feeder cell is a cell irradiated with radiation.

21. The cell of claim 16, wherein the cell expresses the target DNA for 7 days or more after introducing the transposon vector.

22. A method of inserting a target DNA sequence into the genome of a cell, comprising:
introducing a) the transposon vector of claim 1 into which target DNA is inserted; and b) a nucleic acid molecule including a sequence encoding a transposase protein or transposase into a cell.

23. The method of claim 22, wherein the introduction is performed by electroporation.

24. The method of claim 22, further comprising:
co-culturing the transposon vector-inserted cells with feeder cells after the introduction.

25. The method of claim 24, wherein the co-culturing with the feeder cells is performed immediately after the introduction.

26. The method of claim 22, wherein the transposon vector is a circular plasmid, linearized double stranded DNA (dsDNA), hairpin dsDNA, or minicircle dsDNA.

27. A pharmaceutical composition for preventing or treating cancer, comprising:
immune cells into which a) the transposon vector of claim 1 into which target DNA is inserted; and b) a nucleic acid molecule including a sequence encoding a transposase protein or transposase are introduced as an active ingredient,
wherein the target DNA is one or more selected from the group consisting of a tumor antigen-specific CAR coding sequence or a fragment thereof, an oncovirus-specific neutralizing antibody coding sequence or a fragment thereof, an immune checkpoint inhibitor coding sequence, and a tumor antigen-specific T-cell receptor (TCR) coding sequence or a fragment thereof.

28. The pharmaceutical composition of claim 27, wherein the tumor antigen is one or more selected from the group consisting of CD19, NY-ESO-1, EGFR, TAG72, interleukin 13 receptor alpha-2 subunit (IL13Rα2), CD52, CD33, CD20, TSLPR, CD22, CD30, GD3, CD171, neural cell adhesion molecule (NCAM), folate binding protein (FBP), Lewis-Y antigen (Le(Y)), prostate stem cell antigen (PSCA), prostate-specific membrane antigen (PSMA), carcinoembryonic antigen (CEA), human epidermal growth factor receptor 2 (HER2), mesothelin, hyaluronate receptor variant 6 (CD44v6), B7-H3, glypican-3, receptor tyrosine kinase like orphan receptor 1 (ROR1), survivin, folate receptor (FOLR1), Wilm's tumor antigen (WT1), vascular endothelial growth factor 2 (VEGFR2), an oncovirus antigen, TP53, KRAS, and a neoantigen.

29. A kit for preventing or treating cancer, comprising:
a transposon system that comprises a) the transposon vector of claim 1 into which target DNA is inserted; and b) a nucleic acid molecule including a sequence encoding a transposase protein or transposase,
wherein the target DNA is a tumor antigen-specific CAR coding sequence or a fragment thereof, or a tumor antigen-specific T-cell receptor (TCR) coding sequence or a fragment thereof.

30. A method of preventing or treating cancer, comprising:
administering immune cells into which a) the transposon vector of claim 1 into which target DNA is inserted, and b) a nucleic acid molecule including a sequence encoding a transposase protein or transposase are introduced to an individual in need thereof,
wherein the target DNA is one or more selected from the group consisting of a tumor antigen-specific CAR coding sequence or a fragment thereof, an oncovirus-specific neutralizing antibody coding sequence or a fragment thereof, an immune checkpoint inhibitor coding sequence, and a tumor antigen-specific T-cell receptor (TCR) coding sequence or a fragment thereof.

31. A use of immune cells into which a) the transposon vector of claim 1 into which target DNA is inserted; and b) a nucleic acid molecule including a sequence encoding a transposase protein or transposase are introduced for preventing or treating cancer,
wherein the target DNA is one or more selected from the group consisting of a tumor antigen-specific CAR coding sequence or a fragment thereof, an oncovirus-specific neutralizing antibody coding sequence or a fragment thereof, an immune checkpoint inhibitor coding sequence, and a tumor antigen-specific T-cell receptor (TCR) coding sequence or a fragment thereof.

32. A use of immune cells into which a) the transposon vector of claim 1 into which target DNA is inserted; and b) a nucleic acid molecule including a sequence encoding a transposase protein or transposase are introduced for preparing a drug for treating cancer,
wherein the target DNA is one or more selected from the group consisting of a tumor antigen-specific CAR coding sequence or a fragment thereof, an oncovirus-specific neutralizing antibody coding sequence or a fragment thereof, an immune checkpoint inhibitor coding sequence, and a tumor antigen-specific T-cell receptor (TCR) coding sequence or a fragment thereof.
